(19) Europäisches Patentamt
European Patent Office
Office européen des brevets

(11) **EP 4 291 250 B1**

(12) **EUROPEAN PATENT SPECIFICATION**

(45) Date of publication and mention of the grant of the patent:
**15.01.2025 Bulletin 2025/03**

(21) Application number: **22705803.9**

(22) Date of filing: **15.02.2022**

(51) International Patent Classification (IPC):
***A61K 51/04*** *(2006.01)* ***A61P 35/00*** *(2006.01)*
***A61K 103/30*** *(2006.01)*

(52) Cooperative Patent Classification (CPC):
**A61K 51/0497; A61K 51/0402; A61P 35/00**

(86) International application number:
**PCT/EP2022/053699**

(87) International publication number:
**WO 2022/171901 (18.08.2022 Gazette 2022/33)**

(54) **DUAL MODE RADIOTRACER AND THERAPEUTICS**

DUALMODUS-RADIOTRACER UND -THERAPEUTIKA

AGENTS À DEUX MODES RADIOTRACEURS ET THÉRAPEUTIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.02.2021 EP 21157154**

(43) Date of publication of application:
**20.12.2023 Bulletin 2023/51**

(73) Proprietor: **Technische Universität München**
**80333 München (DE)**

(72) Inventors:
- **WESTER, Hans-Jürgen**
  **85301 Schweitenkirchen (DE)**
- **FISCHER, Sebastian**
  **85221 Dachau (DE)**
- **WURZER, Alexander**
  **81673 München (DE)**
- **KUNERT, Jan-Philip**
  **80797 München (DE)**

(74) Representative: **Ravizza, Claudio**
**Bracco Imaging SpA**
**Intellectual Property Department**
**Via Egidio Folli, 50**
**20134 Milano (IT)**

(56) References cited:
**WO-A1-2019/020831 WO-A1-2020/157177**
**WO-A1-2020/157184**

- **ALEXANDER WURZER ET AL: "Radiohybrid ligands: a novel tracer concept exemplified by 18 F- or 68 Ga-labeled rhPSMA-inhibitors", THE JOURNAL OF NUCLEAR MEDICINE, 20 December 2019 (2019-12-20), US, XP055686996, ISSN: 0161-5505, DOI: 10.2967/jnumed.119.234922**
- **ALEXANDER WURZER ET AL: "Preclinical comparison of four [F, Ga]rhPSMA-7 isomers: influence of the stereoconfiguration on pharmacokinetics", EJNMMI RESEARCH, BIOMED CENTRAL LTD, LONDON, UK, vol. 10, no. 1, 7 December 2020 (2020-12-07), pages 1 - 10, XP021285239, DOI: 10.1186/S13550-020-00740-Z**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 291 250 B1

## Description

The present invention relates to compounds that bind to prostate-specific membrane antigen (PSMA) comprising a PSMA binding moiety, a linker group comprising a silicon-fluoride acceptor (SIFA) moiety and a chelator moiety, optionally containing a chelated nonradioactive or radioactive cation, wherein the SIFA moiety comprises a covalent bond between a silicon and a fluorine atom which can be $^{18}F$.

## BACKGROUND

*Prostate cancer*

Prostate Cancer (PCa) remained over the last decades the most common malignant disease in men with high incidence for poor survival rates. Due to its overexpression in prostate cancer (Silver et al., Clinical Cancer Research 3, 81-85 (1997)), prostate-specific membrane antigen (PSMA) or glutamate carboxypeptidase II (GCP II) proved its eligibility as excellent target for the development of highly sensitive radiolabelled agents for endoradiotherapy and imaging of PCa (Afshar-Oromieh et al., European journal of nuclear medicine and molecular imaging 42, 197-209 (2015); Benešová et al., Journal of Nuclear Medicine 56, 914-920 (2015); Robu et al., Journal of Nuclear Medicine, jnumed. 116.178939 (2016); Weineisen et al.; Journal of Nuclear Medicine 55, 1083-1083 (2014); Rowe et al., Prostate cancer and prostatic diseases (2016); Maurer et al., Nature Reviews Urology (2016)). Prostate-specific membrane antigen is an extracellular hydrolase whose catalytic center comprises two zinc(II) ions with a bridging hydroxido ligand. It is highly upregulated in metastatic and hormone-refractory prostate carcinomas, but its physiologic expression has also been reported in kidneys, salivary glands, small intestine, brain and, to a low extent, also in healthy prostate tissue. In the intestine, PSMA facilitates absorption of folate by conversion of pteroylpoly-$\gamma$-glutamate to pteroylglutamate (folate). In the brain, it hydrolyses N-acetyl-Laspartyl-L-glutamate (NAAG) to N-acetyl-L-aspartate and glutamate.

*Prostate-specific membrane antigen (PSMA)*

Prostate-specific membrane antigen (PSMA) is a type II transmembrane glycoprotein that is highly overexpressed on prostate cancer epithelial cells. Despite its name, PSMA is also expressed, to varying degrees, in the neovasculature of a wide variety of nonprostate cancers. Among the most common nonprostate cancers to demonstrate PSMA expression include breast, lung, colorectal, and renal cell carcinoma.

The general necessary structures of PSMA targeting molecules comprise a binding unit that encompasses a zinc-binding group (such as urea (Zhou et al., Nature Reviews Drug Discovery 4, 1015-1026 (2005)), phosphinate or phosphoramidate) connected to a P1' glutamate moiety, which warrants high affinity and specificity to PSMA and is typically further connected to an effector functionality (Machulkin et al., Journal of drug targeting, 1-15 (2016)). The effector part is more flexible and to some extent tolerant towards structural modifications. The entrance tunnel accommodates two other prominent structural features, which are important for ligand binding. The first one is an arginine patch, a positively charged area at the wall of the entrance funnel and the mechanistic explanation for the preference of negatively charged functionalities at the P1 position of PSMA. This appears to be the reason for the preferable incorporation of negative charged residues within the ligand-scaffold. An in-depth analysis about the effect of positive charges on PSMA ligands has been, to our knowledge, so far not conducted. Upon binding, the concerted repositioning of the arginine side chains can lead to the opening of an S1 hydrophobic accessory pocket, the second important structure that has been shown to accommodate an iodo-benzyl group of several urea based inhibitors, thus contributing to their high affinity for PSMA (Barinka et al., Journal of medicinal chemistry 51, 7737-7743 (2008)).

Zhang et al. discovered a remote binding site of PSMA, which can be employed for bidentate binding mode (Zhang et al., Journal of the American Chemical Society 132, 12711-12716 (2010)). The so called arene-binding site is a simple structural motif shaped by the side chains of Arg463, Arg511 and Trp541, and is part of the GCPII entrance lid. The engagement of the arene binding site by a distal inhibitor moiety can result in a substantial increase in the inhibitor affinity for PSMA due to avidity effects. PSMA I&T was developed with the intention to interact this way with PSMA, albeit no crystal structure analysis of binding mode is available. A necessary feature according to Zhang et al. is a linker unit (Suberic acid in the case of PSMA I&T) which facilitates an open conformation of the entrance lid of GCPII and thereby enabling the accessibility of the arene-binding site. It was further shown that the structural composition of the linker has a significant impact on the tumor-targeting and biologic activity as well as on imaging contrast and pharmacokinetics (Liu et al., Bioorganic & medicinal chemistry letters 21, 7013-7016 (2011)), properties which are crucial for both high imaging quality and efficient targeted endoradiotherapy.

Two categories of PSMA targeting inhibitors are currently used in clinical settings. On the one side there are tracers with chelating units for radionuclide complexation such as PSMA I&T or related compounds (Kiess et al., The quarterly journal of nuclear medicine and molecular imaging 59, 241 (2015)). On the other side there are small molecules,

comprising a targeting unit and effector molecules.

The most often used agents for selective PSMA imaging are PSMA HBED-CC (Eder et al., Bioconjugate chemistry 23, 688-697 (2012)), PSMA-617 (Benešová et al., Journal of Nuclear Medicine 56, 914-920 (2015)) and PSMA I&T (Weineisen et al.; Journal of Nuclear Medicine 55, 1083-1083 (2014)), which are predominantly labelled with $^{68}Ga$ (88.9% $\beta^+$, $E_{\beta+, max}$ = 1.89 MeV, $t_{½}$ = 68 min). Among these $^{68}Ga$-PSMA-HBED-CC (also known as $^{68}Ga$-PSMA-11), is so far considered as the golden standard for PET imaging of PCa.

*$^{18}F$ labelling*

Recently, several groups have focused on the development of novel $^{18}F$-labelled urea-based inhibitors for PCa diagnosis. In contrast to the radiometal $^{68}Ga$, which can be obtained from commercially distributed $^{68}Ge/^{68}Ga$ radionuclide generators ($^{68}Ge$; $t_{½}$ = 270.8 d), the radioisotope $^{18}F$-fluoride (96.7% $\beta^+$, $E_{\beta+, max}$ = 634 keV) requires an on-site cyclotron for its production. Despite this limitation, $^{18}F$ offers due to its longer half-live ($t_{½}$ = 109.8 min) and its lower positron energy, significant advantages in terms of routine-handling and image quality. Additionally, there is the possibility for largescale production in a cyclotron, which would be beneficial for a higher patient throughput and reduction of production costs. The $^{18}F$-labelled urea-based PSMA inhibitor $^{18}F$-DCFPyl demonstrated promising results in the detection of primary and metastatic PCa (Rowe et al., Molecular Imaging and Biology, 1-9 (2016)) and superiority to $^{68}Ga$-PSMA-HBED-CC in a comparative study (Dietlein et al., Molecular Imaging and Biology 17, 575-584 (2015)). Based on the structure of PSMA-617, the $^{18}F$-labelled analogue PSMA-1007 was recently developed, which showed comparable tumor-to-organ ratios (Cardinale et al., Journal of nuclear medicine: official publication, Society of Nuclear Medicine 58, 425-431 (2017); Giesel et al., European journal of nuclear medicine and molecular imaging 43, 1929-1930 (2016)). A comparative study with $^{68}Ga$-PSMA-HBED-CC revealed similar diagnostic accuracy of both tracers and a reduced urinary clearance of $^{18}F$-PSMA-1007, enabling a better assessment of the prostate (Giesel et al., European journal of nuclear medicine and molecular imaging 44, 678-688 (2017)).

An attractive approach for introducing $^{18}F$ labels is the use of silicon fluoride acceptors (SIFA). Silicon fluoride acceptors are described, for example, in Lindner et al., Bioconjugate Chemistry 25, 738-749 (2014). In order to preserve the silicon-fluoride bond, the use of silicon fluoride acceptors introduces the necessity of sterically demanding groups around the silicone atom. This in turn renders silicon fluoride acceptors highly hydrophobic. In terms of binding to the target molecule, in particular to the target molecule which is PSMA, the hydrophobic moiety provided by the silicone fluoride acceptor may be exploited for the purpose of establishing interactions of the radio-diagnostic or -therapeutic compound with the hydrophobic pocket described in Zhang et al., Journal of the American Chemical Society 132, 12711-12716 (2010). Yet, prior to binding, the higher degree of lipophilicity introduced into the molecule poses a severe problem with respect to the development of radiopharmaceuticals with suitable in vivo biodistribution, i.e. low unspecific binding in non-target tissue.

*Failure to solve the hydrophobicity problem*

Despite many attempts, the hydrophobicity problem caused by silicon fluoride acceptors has not been satisfactorily solved in the prior art.

To explain further, Schirrmacher E. et al. (Bioconjugate Chem. 2007, 18, 2085-2089) synthesized different $^{18}F$-labelled peptides using the highly effective labelling synthon p-(di-tert-butylfluorosilyl) benzaldehyde ([$^{18}F$]SIFA-A), which is one example of a silicon fluoride acceptor. The SIFA technique resulted in an unexpectedly efficient isotopic $^{18}F$-$^{18}F$ exchange and yielded the $^{18}F$-synthon in almost quantitative yields in high specific activities between 225 and 680 GBq/$\mu$mol (6081-18 378 Ci/mmol) without applying HPLC purification. [$^{18}F$]SIFA-benzaldehyde was finally used to label the N-terminal amino-oxy (N-AO) derivatized peptides AO-Tyr3 -octreotate (AO-TATE), cyclo(fK(AO-N)RGD) and N-AO-$PEG_2$-[D-Tyr-Gln-Trp-Ala-Val-Ala-His-Thi-Nle-$NH_2$] (AO-BZH3, a bombesin derivative) in high radiochemical yields. Nevertheless, the labelled peptides are highly lipophilic (as can be taken from the HPLC retention times using the conditions described in this paper) and thus are unsuitable for further evaluation in animal models or humans.

In Wängler C. et al. (Bioconjugate Chem., 2009, 20 (2), pp 317-321), the first SIFA-based Kit-like radiofluorination of a protein (rat serum albumin, RSA) has been described. As a labelling agent, 4-(di-tert-butyl[$^{18}F$]fluorosilyl)benzenethiol (Si[$^{18}F$]FA-SH) was produced by simple isotopic exchange in 40-60% radiochemical yield (RCY) and coupled the product directly to maleimide derivatized serum albumin in an overall RCY of 12% within 20-30 min. The technically simple labelling procedure does not require any elaborated purification procedures and is a straightforward example of a successful application of Si-18F chemistry for in vivo imaging with PET. The time-activity cureves and $\mu$PET images of mice showed that most of the activity was localized in the liver, thus demonstrating that the labelling agent is too lipophilic and directs the in vivo probe to hepatobiliary excretion and extensive hepatic metabolism.

Wängler C. et al. (see Bioconjug Chem. 2010 Dec 15;21(12):2289-96) subsequently tried to overcome the major drawback of the SIFA technology, the high lipophilicity of the resulting radiopharmaceuticals, by synthesizing and

evaluating new SIFA-octreotate analogues (SIFA-Tyr3-octreotate, SIFA-Asn(AcNH-β-Glc)-Tyr3-octreotate and SIFA-Asn(AcNH-β-Glc)-PEG-Tyr3-octreotate). In these compounds, hydrophilic linkers and pharmacokinetic modifiers were introduced between the peptide and the SIFA-moiety, i.e. a carbohydrate and a PEG linker plus a carbohydrate. As a measure of lipophilicity of the conjugates, the log P(ow) was determined and found to be 0.96 for SIFA-Asn(AcNH-β-Glc)-PEG-$Tyr^3$-octreotate and 1.23 for SIFA-Asn(AcNH-β-Glc)-$Tyr^3$-octreotate. These results show that the high lipophilicity of the SIFA moiety can only be marginally compensated by applying hydrophilic moieties. A first imaging study demonstrated excessive hepatic clearance /liver uptake and thus has never been transferred into a first human study.

Bernard-Gauthier et al. (Biomed Res Int. 2014;2014:454503) reviews a great plethora of different SIFA species that have been reported in the literature ranging from small prosthetic groups and other compounds of low molecular weight to labelled peptides and most recently affibody molecules. Based on these data the problem of lipophilicity of SIFA-based prosthetric groups has not been solved sofar; i.e. a methodology that reduces the overall lipophilicity of a SIFA conjugated peptide to a log D lower than approx -2,0 has not been described.

In Lindner S. et al. (Bioconjug Chem. 2014 Apr 16;25(4):738-49) it is described that PEGylated bombesin (PESIN) derivatives as specific GRP receptor ligands and RGD (one-letter codes for arginine-glycine-aspartic acid) peptides as specific αvβ3 binders were synthesized and tagged with a silicon-fluorine-acceptor (SIFA) moiety. To compensate the high lipophilicity of the SIFA moiety various hydrophilic structure modifications were introduced leading to reduced logD values. SIFA-Asn(AcNH-β-Glc)-PESIN, SIFA-Ser(β-Lac)-PESIN, SIFA-Cya-PESIN, SIFA-LysMe3-PESIN, SIFA-γ-carboxy-d-Glu-PESIN, SIFA-Cya2-PESIN, SIFA-LysMe3-γ-carboxy-d-Glu-PESIN, SIFA-(γ-carboxy-d-Glu)2-PESIN, SIFA-RGD, SIFA-γ-carboxy-d-Glu-RGD, SIFA-(γ-carboxy-d-Glu)2-RGD, SIFA-LysMe3-γ-carboxy-d-Glu-RGD. All of these peptides - already improved and derivatized with the aim to reduce the lipophilicity - showed a logD value in the range between +2 and -1.22.

In Niedermoser S. et al. (J Nucl Med. 2015 Jul;56(7):1100-5), newly developed $^{18}$F-SIFA- and $^{18}$F-SIFAlin- (SIFA = silicon-fluoride acceptor) modified TATE derivatives were compared with the current clinical gold standard $^{68}$Ga-DOTATATE for high-quality imaging of somatostatin receptor-bearing tumors. For this purpose, $^{18}$F-SIFA-TATE and two quite complex analogues, $^{18}$F-SIFA-Glc-PEG1-TATE, $^{18}$F-SIFAlin-Glc-Asp2-PEG1-TATE were developed. None of the agents showed a logD <-1.5.

In view of the above, the technical problem underlying the present invention can be seen in providing radio-diagnostics and radio-therapeutics which contain a silicone fluoride acceptor and which are, at the same time, characterized by favourable in-vivo properties.

WO2019/020831 and WO2020/157184 disclose ligand-SIFA-chelator conjugates. WO2020/157177, Wurzer et al. (J. Nucl. Med. 2020; 61(5): 735-742), Wurzer et al. (EJNMMI Research 2020; 10(1): 1-10) also disclose ligand-SIFA-chelator conjugates.

In the present invention a proof-of-principle has been established using specific conjugates which bind with high affinity to prostate-specific antigen (PSMA) as target. Accordingly, a further technical problem underlying the present invention can be seen in providing improved radio-therapeutics and -diagnostics for the medical indication which is cancer, preferably prostate cancer.

## SUMMARY OF THE INVENTION

The invention is described in claim 1.

An aspect of the present invention relates to compounds of Formula (1):

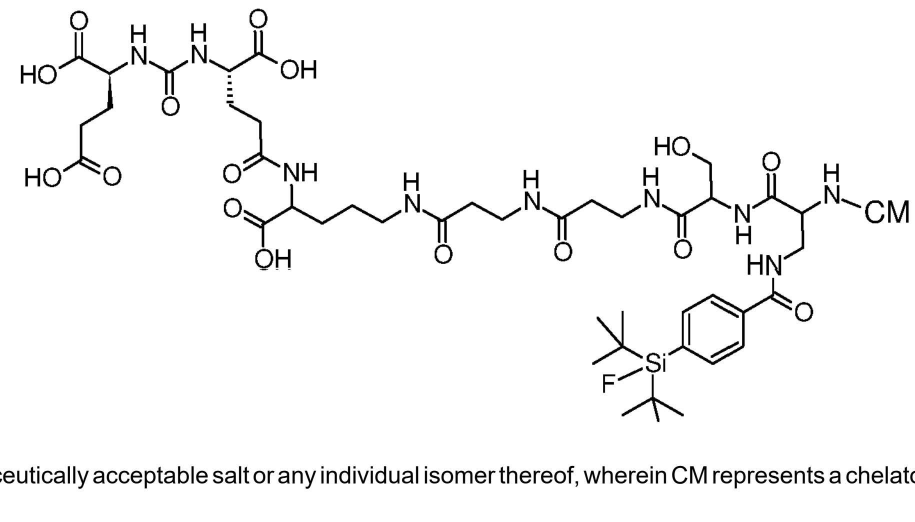

(1);

or a pharmaceutically acceptable salt or any individual isomer thereof, wherein CM represents a chelator moiety, optionally

containing a chelated nonradioactive or radioactive cation and the fluorine atom is optionally $^{18}F$.

An aspect of the present invention relates to compounds of Formula (1a):

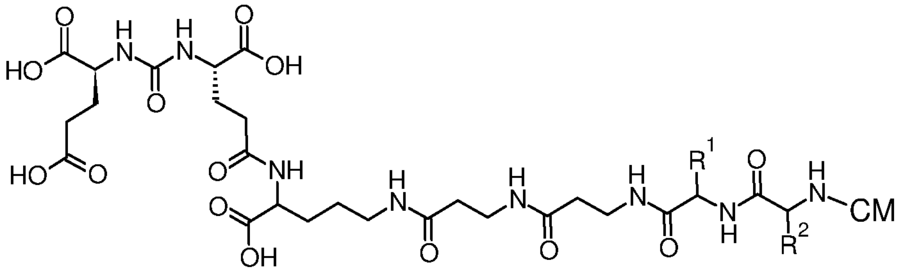

(1a)

or a pharmaceutically acceptable salt or any individual isomer thereof, wherein either

$R^1$ is $-(CH_2)_nR^3$ where n is 1, 2 or 3 and $R^3$ is selected from OH, $NH_2$ or $NHC(O)NH_2$ and $R^2$ is

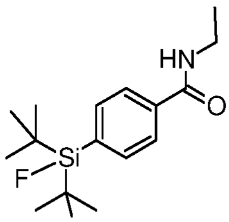

;

or

$R^2$ is $-(CH_2)_nR^3$ where n is 1, 2 or 3 and $R^3$ is selected from OH, $NH_2$ or $NHC(O)NH_2$ and $R^1$ is

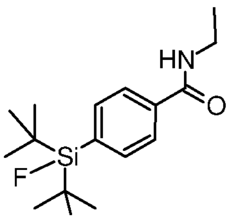

;

and

CM represents a chelator moiety, optionally containing a chelated nonradioactive or radioactive cation and the fluorine atom is optionally $^{18}F$.

An aspect of the present invention relates to compounds of Formula (1b):

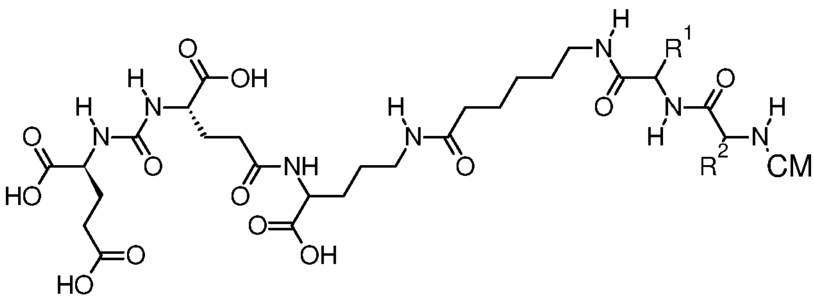

(1b)

or a pharmaceutically acceptable salt or any individual isomer thereof, wherein either

$R^1$ is $-(CH_2)_nR^3$ where n is 1, 2 or 3 and $R^3$ is selected from OH, $NH_2$ or $NHC(O)NH_2$ and $R^2$ is

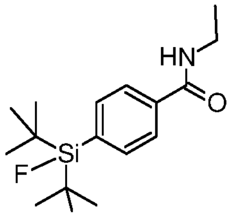

;

or

$R^2$ is $-(CH_2)_nR^3$ where n is 1, 2 or 3 and $R^3$ is selected from OH, $NH_2$ or $NHC(O)NH_2$ and $R^1$ is

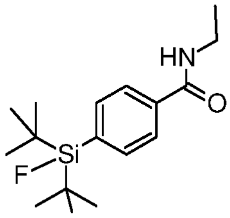

;

and

CM represents a chelator moiety, optionally containing a chelated nonradioactive or radioactive cation and the fluorine atom is optionally $^{18}F$.

An aspect of the present invention relates to compounds of Formula (1c):

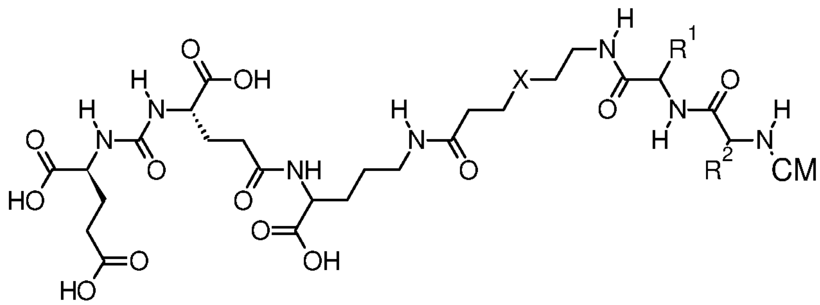

(1c)

or a pharmaceutically acceptable salt or any individual isomer thereof, wherein either

$R^1$ is $-(CH_2)_nR^3$ where n is 1, 2 or 3 and $R^3$ is selected from OH, $NH_2$ or $NHC(O)NH_2$ and $R^2$ is

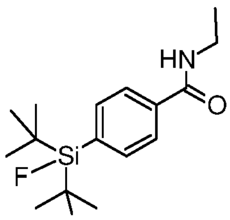

;

or

$R^2$ is $-(CH_2)_nR^3$ where n is 1, 2 or 3 and $R^3$ is selected from OH, $NH_2$ or $NHC(O)NH_2$ and $R^1$ is

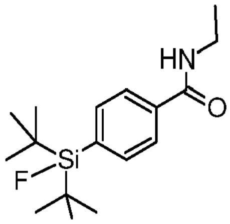

;

X is $CH_2$ or NHCO and

CM represents a chelator moiety, optionally containing a chelated nonradioactive or radioactive cation and the fluorine atom is optionally $^{18}F$.

Also provided is a pharmaceutical or diagnostic composition comprising or consisting of one or more compounds of Formula (1). The compounds of the invention may be for use as a cancer diagnostic or imaging agent.

The compounds or compositions of the invention may be for use in the treatment of cancer. The compounds or compositions of the invention may be for use in the diagnosis, imaging or prevention of neoangiogenesis/angiogenesis. The compounds or compositions of the invention may be for use as a cancer diagnostic or imaging agent or for use in the treatment of cancer wherein the cancer is prostate, breast, lung, colorectal or renal cell carcinoma.

## DETAILED DESCRIPTION

An aspect of the present invention relates to compounds of Formula (1a) or (1b):

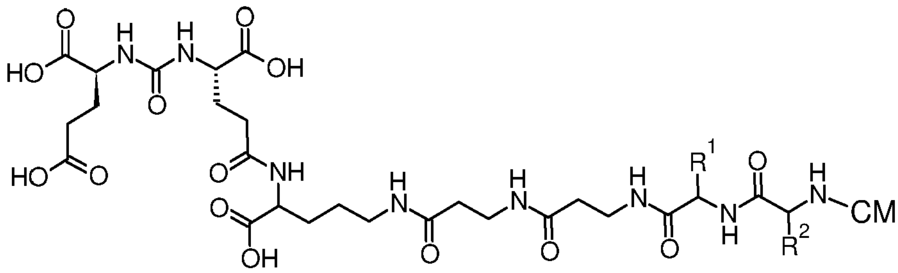

(1a),

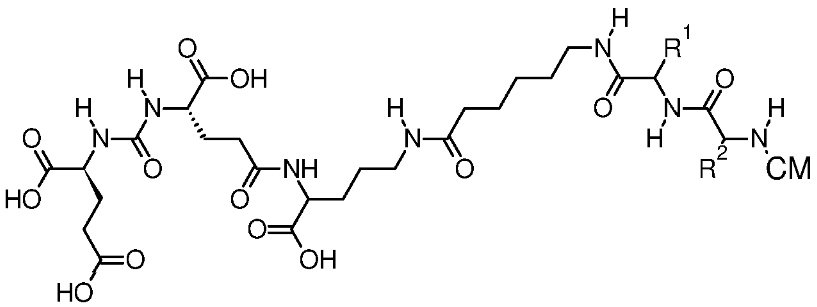

(1b)

or a pharmaceutically acceptable salt or any individual isomer thereof, wherein either

$R^1$ is $-(CH_2)_nR^3$ where n is 1, 2 or 3 and $R^3$ is selected from OH, $NH_2$ or $NHC(O)NH_2$ and $R^2$ is

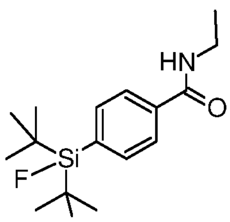

;

or

$R^2$ is $-(CH_2)_nR^3$ where n is 1, 2 or 3 and $R^3$ is selected from OH, $NH_2$ or $NHC(O)NH_2$ and $R^1$ is

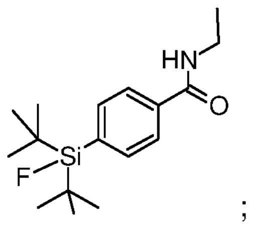

;

and
CM represents a chelator moiety, optionally containing a chelated nonradioactive or radioactive cation and the fluorine atom is optionally $^{18}F$.

An aspect of the present invention relates to compounds of Formula (1):

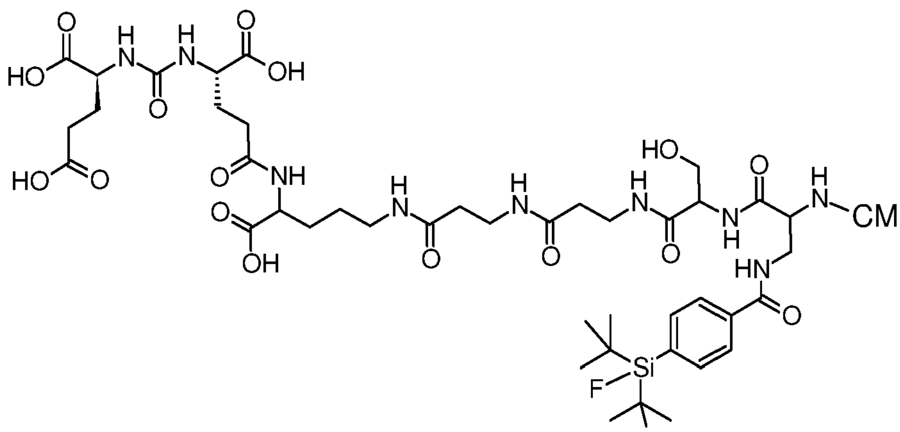

(1);

or a pharmaceutically acceptable salt or any individual isomer thereof, wherein
CM represents a chelator moiety, optionally containing a chelated nonradioactive or radioactive cation and the fluorine atom is optionally $^{18}F$.

An aspect of the present invention relates to compounds of Formula (1'):

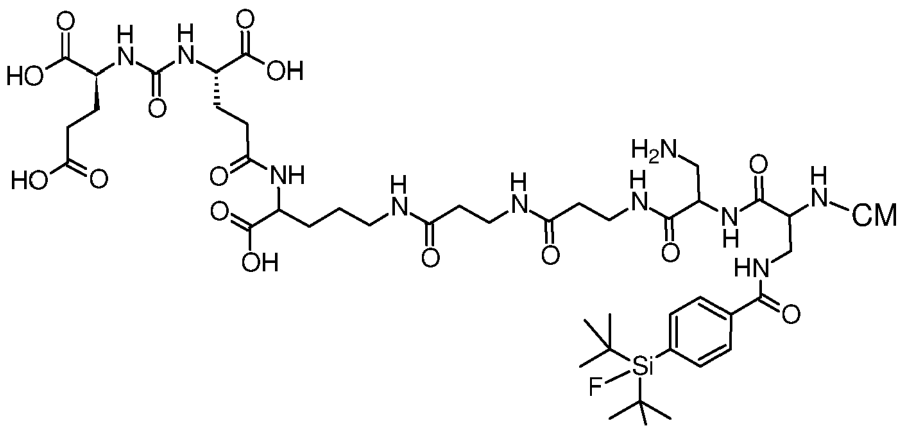

(1');

or a pharmaceutically acceptable salt or any individual isomer thereof, wherein
CM represents a chelator moiety, optionally containing a chelated nonradioactive or radioactive cation and the fluorine atom is optionally $^{18}F$.

An aspect of the present invention relates to compounds of Formula (1"):

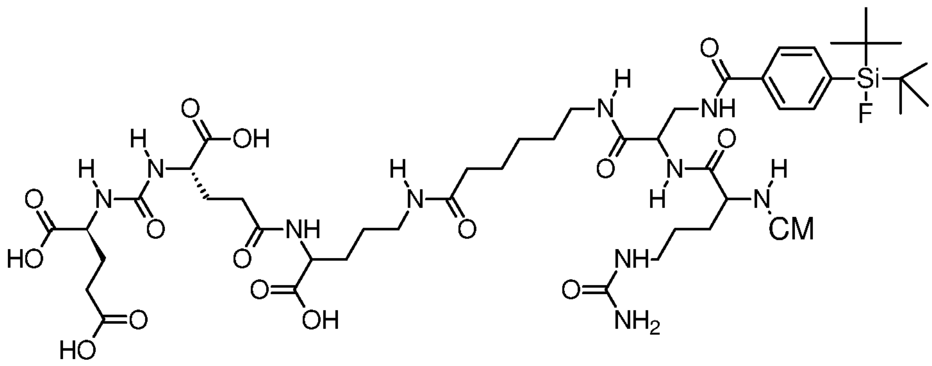

(1");

or a pharmaceutically acceptable salt or any individual isomer thereof, wherein
CM represents a chelator moiety, optionally containing a chelated nonradioactive or radioactive cation and the fluorine atom is optionally $^{18}F$.

The compounds of the invention comprise three separate moieties. The three separate moieties are a PSMA binding moiety, a linker group comprising a silicon-fluoride acceptor (SIFA) moiety and a chelator moiety (CM), optionally containing a chelated nonradioactive or radioactive cation, wherein the SIFA moiety comprises a covalent bond between a silicon and a fluorine atom which can be $^{18}F$.

For diagnostic imaging, the fluorine atom on the SIFA moiety may be $^{18}F$. The $^{18}F$ can be introduced by isotopic exchange with $^{18}F$.

The compounds of the invention require the chelator moiety (CM) to be hydrophilic. The hydrophilic chelator moiety (CM) is required to reduce the hydrophobic nature of the compounds caused by the presence of the SIFA moiety. A key aspect of the invention is the combination, within a single molecule, of a silicon fluoride acceptor and a chelator moiety or a chelate.

The cation which may be optionally chelated to the chelator moiety may be a radioactive or non-radioactive cation. It is preferably a non-radioactive metal cation. Examples of suitable cations are provided below.

The compounds of the invention may be radioactively labelled at the SIFA moiety. Also included are molecules which are not radiolabelled at all. The chelator moiety may be either a complex of a cold (non-radioactive) ion or may be devoid of any ion.

The present inventors surprisingly discovered that placement of the silicone fluoride acceptor in the neighbourhood of a hydrophilic chelator such as, but not limited to, DOTAGA or DOTA, shields or compensates efficiently the lipophilicity of the SIFA moiety to an extent which shifts the overall hydrophobicity of compound in a range which renders the compound suitable for in-vivo administration.

A further advantage of the compounds of the present invention is their surprisingly low accumulation in the kidneys of mice when compared to other PSMA targeted radiopharmaceuticals, such as PSMA I&T. Without wishing to be bound by a particular theory, it seems to be the combination of the structural element SIFA with a chelator and the choice of linker which provides for the unexpected reduction of accumulation in the kidneys.

In terms of lipophilicity/hydrophilicity, the logP value (sometimes also referred to as logD value) is an art-established measure.

The term "lipophilicity" relates to the strength of being dissolved in, or be absorbed in lipid solutions, or being adsorbed at a lipid-like surface or matrix. It denotes a preference for lipids (literal meaning) or for organic or apolar liquids or for liquids, solutions or surfaces with a small dipole moment as compared to water. The term "hydrophobicity" is used with equivalent meaning herein. The adjectives lipophilic and hydrophobic are used with corresponding meaning to the substantives described above.

The mass flux of a molecule at the interface of two immiscible or substantially immiscible solvents is governed by its lipophilicity. The more lipophilic a molecule is, the more soluble it is in the lipophilic organic phase. The partition coefficient of a molecule that is observed between water and n-octanol has been adopted as the standard measure of lipophilicity. The partition coefficient P of a species A is defined as the ratio $P = [A]_{n\text{-octanol}} / [A]_{water}$. A figure commonly reported is the logP value, which is the logarithm of the partition coefficient. In case a molecule is ionizable, a plurality of distinct microspecies (ionized and not ionized forms of the molecule) will in principle be present in both phases. The quantity describing the overall lipophilicity of an ionizable species is the distribution coefficient D, defined as the ratio D = $[\text{sum of the concentrations of all microspecies}]_{n\text{-octanol}} / [\text{sum of the concentrations of all microspecies}]_{water}$. Analogous to logP, frequently the logarithm of the distribution coefficient, logD, is reported. Often, a buffer system, such as phosphate buffered saline is used as alternative to water in the above described determination of logP.

If the lipophilic character of a substituent on a first molecule is to be assessed and/or to be determined

quantitatively, one may assess a second molecule corresponding to that substituent, wherein said second molecule is obtained, for example, by breaking the bond connecting said substituent to the remainder of the first molecule and connecting (the) free valence(s) obtained thereby to hydrogen(s).

Alternatively, the contribution of the substituent to the logP of a molecule may be determined. The contribution $\pi x_X$ of a substituent X to the logP of a molecule R-X is defined as $\pi x_X = logP_{R-X} - logP_{R-H}$, wherein R-H is the unsubstituted parent compound.

Values of P and D greater than one as well as logP, logD and $\pi x_X$ values greater than zero indicate lipophilic/hydrophobic character, whereas values of P and D smaller than one as well as logP, logD and $\pi x_X$ values smaller than zero indicate hydrophilic character of the respective molecules or substituents.

The above described parameters characterizing the lipophilicity of the lipophilic group or the entire molecule according to the invention can be determined by experimental means and/or predicted by computational methods known in the art (see for example Sangster, Octanol-water Partition Coefficients: fundamentals and physical chemistry, John Wiley & Sons, Chichester. (1997)).

The logP value of compounds of the invention may be between -5 and -1.5. It is particularly preferred that the logP value is between -3.5 and -2.0.

The compounds are preferably high affinity PSMA ligands with preferable affinity, expressed as $IC_{50}$, being below 50 nM, below 20 nM or below 5 nM.

The compounds of the invention may be compounds of Formula (2):

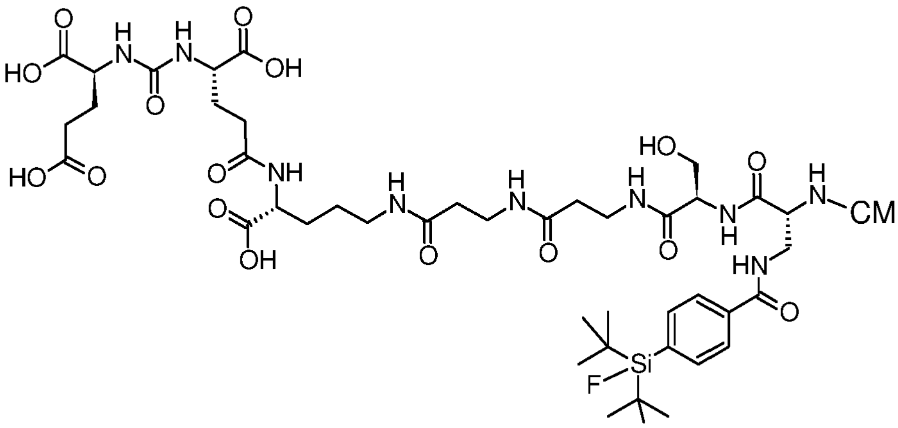

(2);

or a pharmaceutically acceptable salt or any individual isomer thereof, wherein
CM represents a chelator moiety, optionally containing a chelated nonradioactive or radioactive cation and the fluorine atom is optionally $^{18}F$.

The compounds of the invention may be compounds of Formula (3):

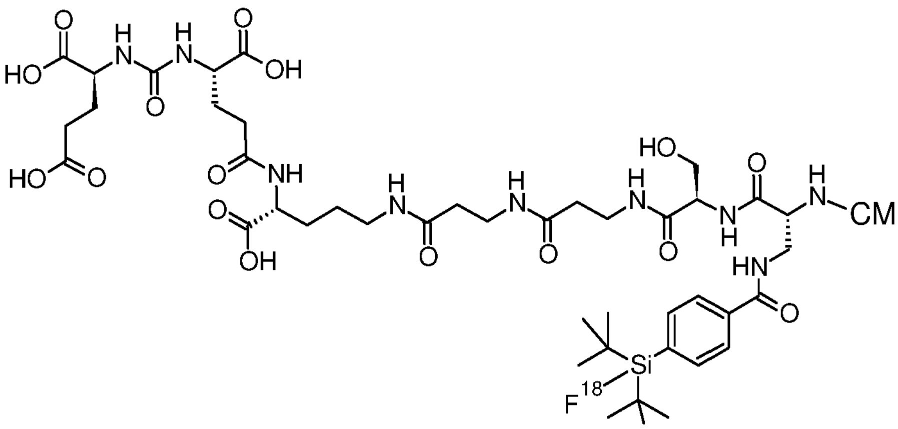

(3);

or a pharmaceutically acceptable salt or any individual isomer thereof, wherein
CM represents a chelator moiety, optionally containing a chelated nonradioactive or radioactive cation.

The compounds of the invention may be compounds of Formula (2a):

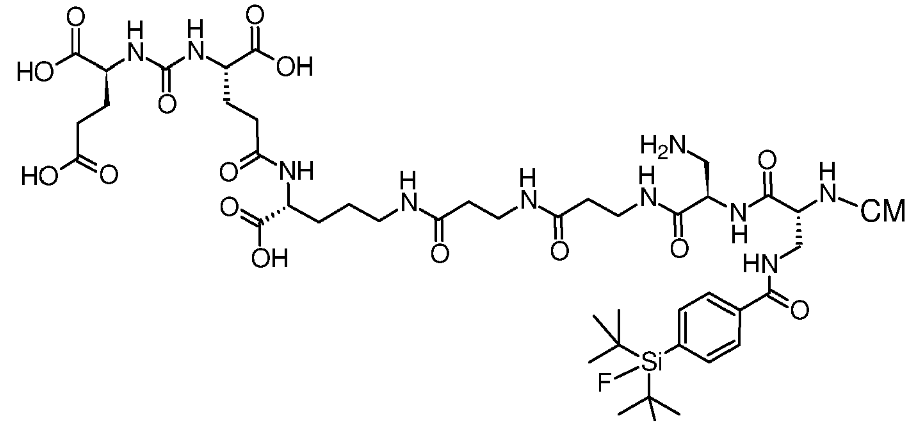

(2a);

or a pharmaceutically acceptable salt or any individual isomer thereof, wherein
CM represents a chelator moiety, optionally containing a chelated nonradioactive or radioactive cation and the fluorine atom is optionally $^{18}F$.

The compounds of the invention may be compounds of Formula (3a):

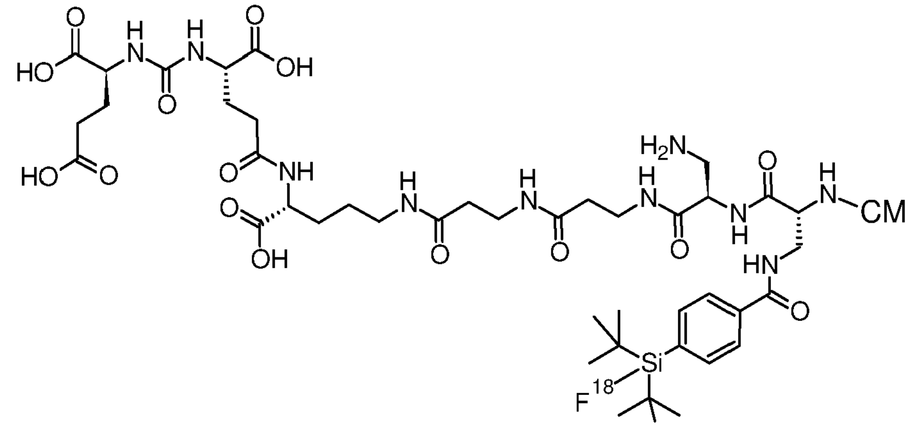

(3a);

or a pharmaceutically acceptable salt or any individual isomer thereof, wherein
CM represents a chelator moiety, optionally containing a chelated nonradioactive or radioactive cation.

An aspect of the present invention relates to compounds of Formula (2b):

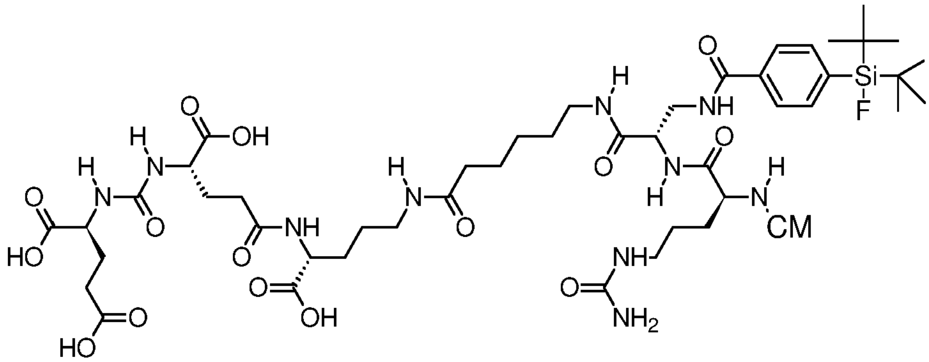

(2b);

or a pharmaceutically acceptable salt or any individual isomer thereof, wherein

CM represents a chelator moiety, optionally containing a chelated nonradioactive or radioactive cation and the fluorine atom is optionally $^{18}F$.

An aspect of the present invention relates to compounds of Formula (3b):

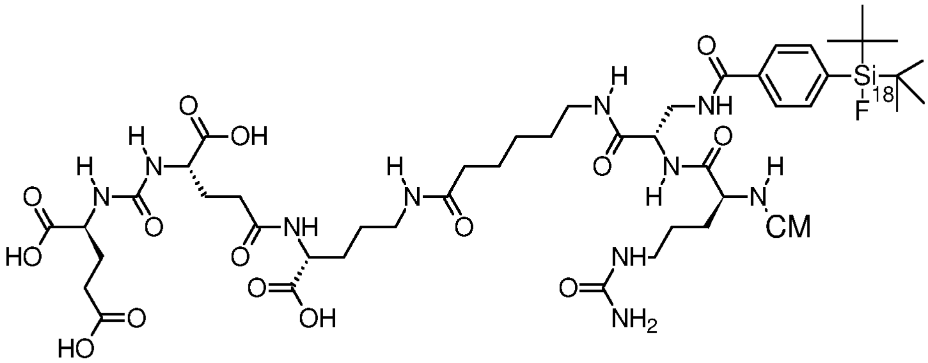

(3b);

or a pharmaceutically acceptable salt or any individual isomer thereof, wherein
CM represents a chelator moiety, optionally containing a chelated nonradioactive or radioactive cation.

In the compounds herein, a preferred chelating group (CM) comprises at least one of the following (i), (ii) or (iii):

(i) A macrocyclic ring structure with 8 to 20 ring atoms of which 2 or more, more preferably 3 or more, are selected from oxygen atoms or nitrogen atoms. Preferably, 6 or less ring atoms are selected from oxygen atoms or nitrogen atoms. Especially preferred is that 3 or 4 ring atoms are nitrogen atoms or oxygen atoms. Among the oxygen and nitrogen atoms, preference is given to the nitrogen atoms. In combination with the macrocyclic ring structure, the preferred chelating group may comprise 2 or more, such as 2 to 6, preferably 2 to 4, carboxyl groups and/or hydroxyl groups. Among the carboxyl groups and the hydroxyl groups, preference is given to the carboxyl groups.
(ii) An acyclic, open chain chelating structure with 8 to 20 main chain (back bone) atoms of which 2 or more, more preferably 3 or more are heteroatoms selected from oxygen atoms or nitrogen atoms. Preferably, 6 or less back bone atoms are selected from oxygen atoms or nitrogen atoms. Among the oxygen and nitrogen atoms, preference is given to the nitrogen atoms. More preferably, the open chain chelating structure is a structure which comprises a combination of 2 or more, more preferably 3 or more heteroatoms selected from oxygen atoms or nitrogen atoms, and 2 or more, such as 2 to 6, preferably 2 to 4, carboxyl groups and/or hydroxyl groups. Among the carboxyl groups and the hydroxyl groups, preference is given to the carboxyl groups.
(iii) A branched chelating structure containing a quaternary carbon atom. Preferably the quaternary carbon atom is substituted with 3 identical chelating groups in addition to the SIFA/ligand moiety. The substituted chelating groups can comprise an amide. The substituted chelating groups can comprise an aromatic group. The substituted chelating groups can comprise a hydroxypyridinone.

The chelator moiety (CM) may comprise at least one of:

(i) a macrocyclic ring structure with 8 to 20 ring atoms of which 2 or more are heteroatoms selected from oxygen atoms and nitrogen atoms;
(ii) an acyclic, open chain chelating structure with 8 to 20 main chain atoms of which 2 or more are heteroatoms selected from oxygen atoms and nitrogen atoms; or
(iii) a branched chelating structure containing a quaternary carbon atom.

In preferred specific examples, the chelator moiety is a residue of a chelating agent selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicyclo[6.6.2]hexadecan (DO2A) 1,4,7,10-tetracyclododecan-N,N',N",N'''-tetraacetic acid (DOTA), $\alpha$-(2-carboxyethyl)-1,4,7,10-tetraazacyclododecane-1,4,7,10-tetraacetic acid (DOTAGA), 1,4,7,10 tetraazacyclododecane N, N', N", N''' 1,4,7,10-tetra(methylene) phosphonic acid (DOTMP), N,N'-dipyridoxylethylendiamine-N,N'-diacetate-5,5'-bis(phosphat) (DPDP), diethylene triamine N,N',N" penta(methylene) phosphonic acid (DTMP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 6-hydrazinyl-N-methylpyridine-3-carboxamide (HYNIC), tetra 3-hydroxy-N-methyl-2-pyridinone chelators (4-((4-(3-(bis(2-(3-hydroxy-1-methyl-2-oxo-1,2-dihydropyridine-4-carboxamido)ethyl)amino)-2-((bis(2-(3-hydroxy-1-methyl-2-oxo-1,2-dihydropyridine-4-carboxamido)ethyl)amino)methyl)propyl)phenyl)amino)-4-oxobutanoic acid), abbreviated as Me-3,2-HOPO, 1,4,7-triazacyclononan-1-succinic acid-4,7-diacetic acid (NODASA), 1-(1-carboxy-3-carboxypropyl)-4,7-(carbooxy)-1,4,7-triazacyclononane (NODAGA), 1,4,7-triazacyclononanetriacetic acid (NOTA), 4,11-bis(carboxymethyl)-1,4,8,11-tetraazabicyclo[6.6.2]hexadecane (TE2A), 1,4,8,11-tetraazacyclododecane-1,4,8,11-tetraacetic acid (TETA), tris(hydroxypyridinone) (THP), terpyridin-bis(methyleneamintetraacetic acid (TMT), 1,4,7-triazacyclononane-1,4,7-tris[methylene(2-carboxyethyl)phosphinic acid] (TRAP), 1,4,7,10-tetraazacyclotridecan-N,N',N",N‴-tetraacetic acid (TRITA), 3-[[4,7-bis[[2-carboxyethyl(hydroxy)phosphoryl]methyl]-1,4,7-triazonan-1-yl]methyl-hydroxy-phosphoryl]propanoic acid, and triethylenetetraaminehexaacetic acid (TTHA), which residue is provided by covalently binding a carboxyl group contained in the chelating agent to the remainder of the conjugate via an ester or an amide bond.

The chelator moiety may be 1,4,7,10-tetracyclododecan-N,N',N",N‴-tetraacetic acid (DOTA) or α-(2-carboxyethyl)-1,4,7,10-tetraazacyclododecane-1,4,7,10-tetraacetic acid (DOTAGA).

Particular chelators are shown below:

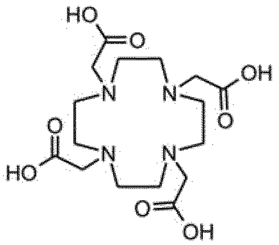

DOTA

,

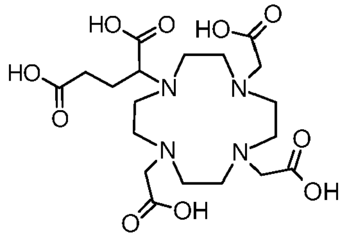

DOTAGA

,

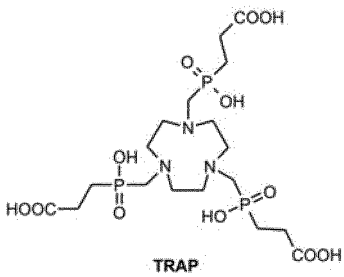

TRAP

,

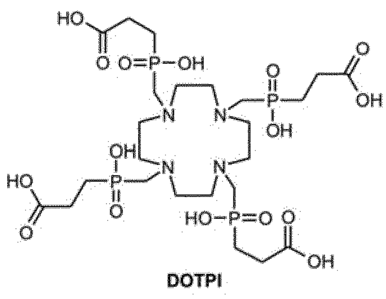

DOTPI

,

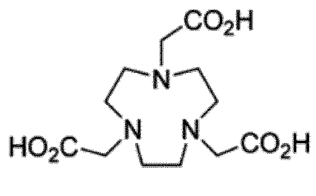

NOTA

,

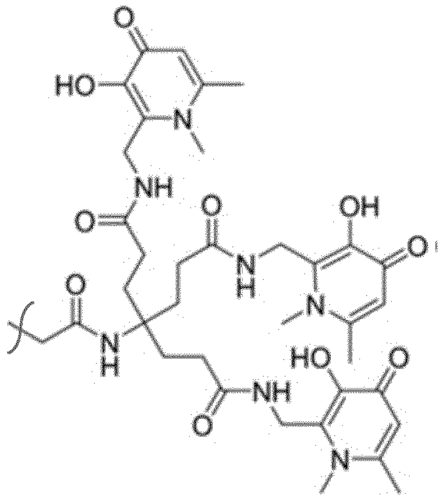

,

THP

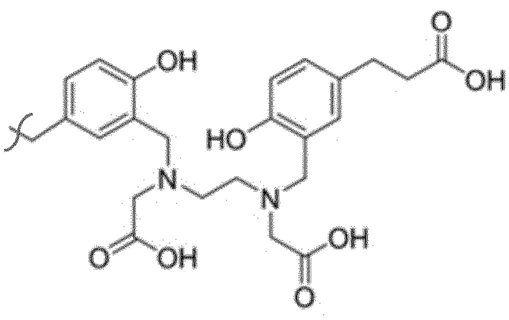

,

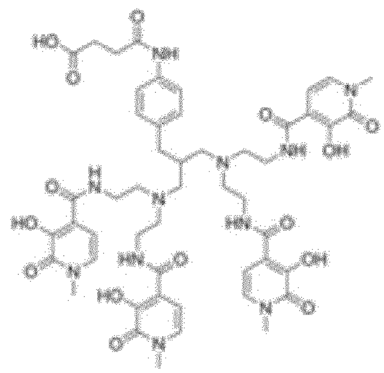

.

HBED

Me-3,2-HOPO

Among the above exemplary chelating agents, particular preference is given to a chelating agent selected from TRAP, DOTA and DOTAGA.

Metal- or cation-chelating macrocyclic and acyclic compounds are well-known in the art and available from a number of manufacturers. While the chelating moiety in accordance with the present invention is not particularly limited, it is understood that numerous moieties can be used in an off-the-shelf manner by a skilled person without further ado.

The chelating group may comprise a chelated cation which may be radioactive or non-radioactive, preferably a chelated metal cation which may be radioactive or non-radioactive. The chelating group may comprise a chelated cation which is radioactive. The chelating group may comprise a chelated cation which is non-radioactive.

Especially preferred is that CM represents a chelating agent selected from DOTA and DOTAGA bound with one of its carboxylic groups via an amide bond to the remainder of the conjugate.

In order to be used in PET imaging, the compounds require a positron emitting atom. The compounds include $^{18}$F for medical use. The compounds may be compounds wherein F is $^{18}$F and CM comprises a radioactive metal cation. The compounds may be compounds wherein F is $^{18}$F and CM comprises a nonradioactive metal cation. The compounds may be compounds wherein F is $^{18}$F and CM comprises a radioactive metal cation. Most preferred compounds of the invention are wherein F includes $^{18}$F and CM comprises a nonradioactive metal cation.

Preferred examples of cations that may be chelated by the chelating group are the non-radioactive cations of Sc, Cr, Mn, Co, Fe, Ni, Cu, Ga, Zr, Y, Tc, Ru, Rh, Pd, Ag, In, Sn, te, Pr, Pm, Tb, Sm, Gd, Tb, Ho, Dy, Er, Yb, Tm, Lu, Re, Pt, Hg, Au, Pb At, Bi, Ra, Ac, Th; more preferably the cations of Sc, Cu, Ga, Y, In, Tb, Ho, Lu, Re, Pb, Bi, Ac, Th and Er. The cation may be Ga. The cation may be Lu.

The chelator moiety may contain a chelated cation selected from the cations of $^{43}$Sc, $^{44}$Sc, $^{47}$Sc, $^{51}$Cr, $^{52m}$Mn, $^{58}$Co, $^{52}$Fe, $^{56}$Ni, $^{57}$Ni, $^{62}$Cu, $^{64}$Cu, $^{67}$Cu, $^{66}$Ga, $^{67}$Ga $^{68}$Ga, $^{89}$Zr, $^{90}$Y, $^{89}$Y, <Tc, $^{99m}$Tc, $^{97}$Ru, $^{105}$Rh, $^{109}$Pd, $^{111}$Ag, $^{110m}$In, $^{111}$In, $^{113m}$In, $^{114m}$In, $^{117m}$Sn, $^{121}$Sn, $^{127}$Te, $^{142}$Pr, $^{143}$Pr, $^{149}$Pm, $^{151}$Pm, $^{149}$Tb, $^{152}$Tb, $^{155}$Tb, $^{161}$Tb, $^{153}$Sm, $^{157}$Gd, $^{161}$Tb, $^{166}$Ho, $^{165}$Dy, $^{169}$Er, $^{169}$Yb, $^{175}$Yb, $^{172}$Tm, $^{177}$Lu, $^{186}$Re, $^{188}$Re, $^{191}$Pt, $^{197}$Hg, $^{198}$Au, $^{199}$Au, $^{212}$Pb, $^{203}$Pb, $^{211}$At., $^{212}$Bi,

$^{213}Bi$, $^{223}Ra$, $^{225}Ac$, $^{227}Th$, a cationic molecule comprising $^{18}F$ or a cation such as $^{18}F$-$[AlF]^{2+}$; more preferably the cations of $^{44}Sc$, $^{47}Sc$, $^{64}Cu$, $^{67}Cu$, $^{68}Ga$, $^{90}Y$, $^{111}In$, $^{161}Tb$, $^{166}Ho$, $^{177}Lu$, $^{188}Re$, $^{212}Pb$, $^{212}Bi$, $^{213}Bi$, $^{225}Ac$, and $^{227}Th$ or a cationic molecule comprising $^{18}F$.

The chelator moiety may contain a chelated cation selected from the cations of $^{43}Sc$, $^{44}Sc$, $^{47}Sc$, $^{64}Cu$, $^{67}Cu$, $^{67}Ga$, $^{68}Ga$, $^{90}Y$, $^{111}In$, $^{149}Tb$, $^{152}Tb$, $^{155}Tb$, $^{161}Tb$, $^{166}Ho$, $^{177}Lu$, $^{186}Re$, $^{188}Re$,$^{212}Pb$, $^{212}Bi$, $^{213}Bi$, $^{225}Ac$, and $^{227}Th$ or a cationic molecule comprising $^{18}F$. The chelator moiety may contain a chelated cation selected from the cations of Ga or Lu. The chelator moiety may contain a chelated Ga cation. The chelator moiety may contain a chelated Lu cation. The chelator moiety may contain a chelated cation selected from the cations of $^{68}Ga$ or $^{177}Lu$. The chelator moiety may contain a chelated $^{68}Ga$ cation. The chelator moiety may contain a chelated $^{177}Lu$ cation.

In the compounds herein, CM may be selected from:

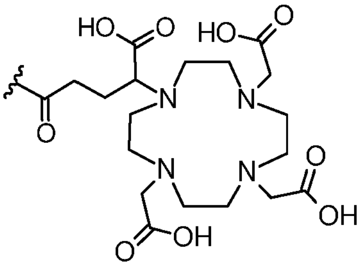 ; 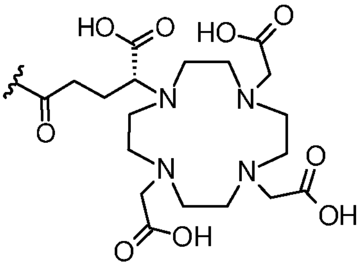 ; 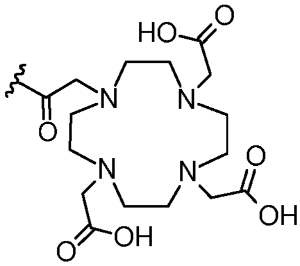 ;

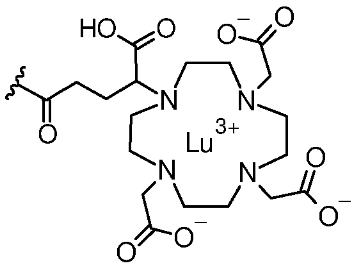 ; 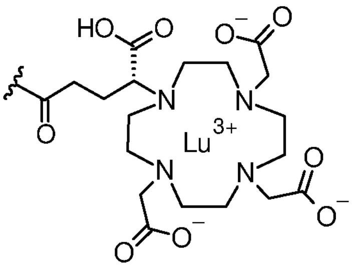 ; 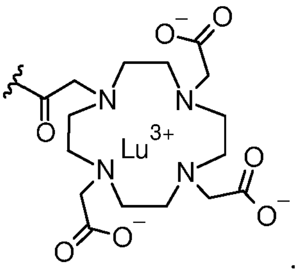 .

The compound may be:

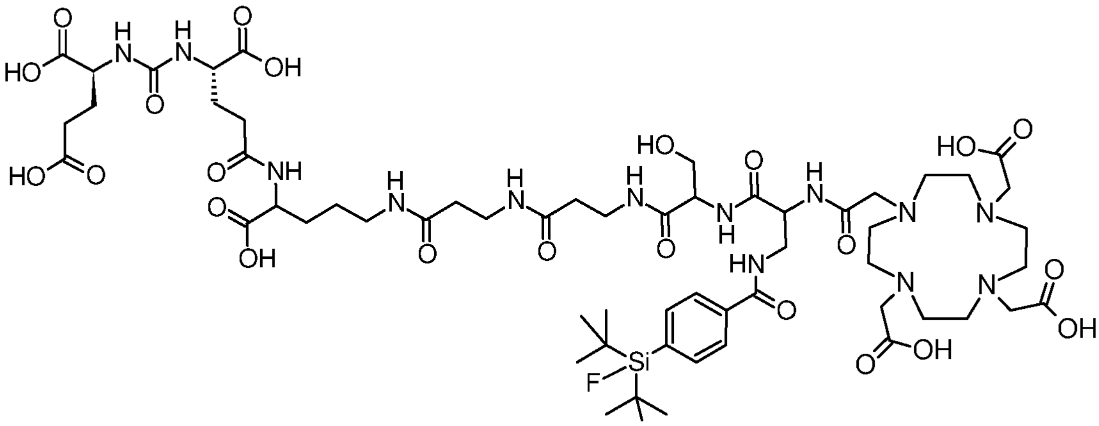 ;

or a pharmaceutically acceptable salt or any individual isomer thereof, wherein the compound optionally contains a chelated nonradioactive or radioactive cation and wherein the fluorine atom is optionally $^{18}F$.

The compound may be:

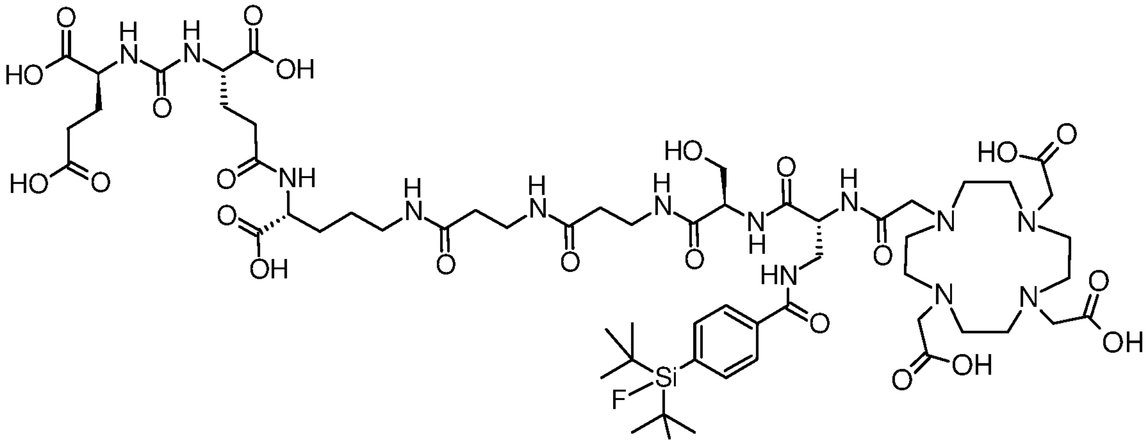

;

or a pharmaceutically acceptable salt thereof, wherein the compound optionally contains a chelated nonradioactive or radioactive cation and wherein the fluorine atom is optionally $^{18}F$.

The compound may be:

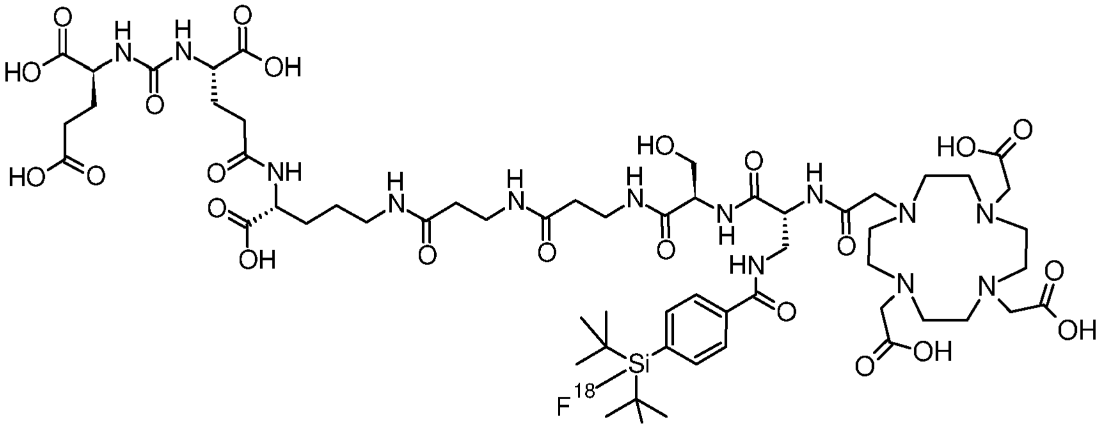

;

or a pharmaceutically acceptable salt thereof, wherein the compound optionally contains a chelated nonradioactive or radioactive cation.

The compound may be:

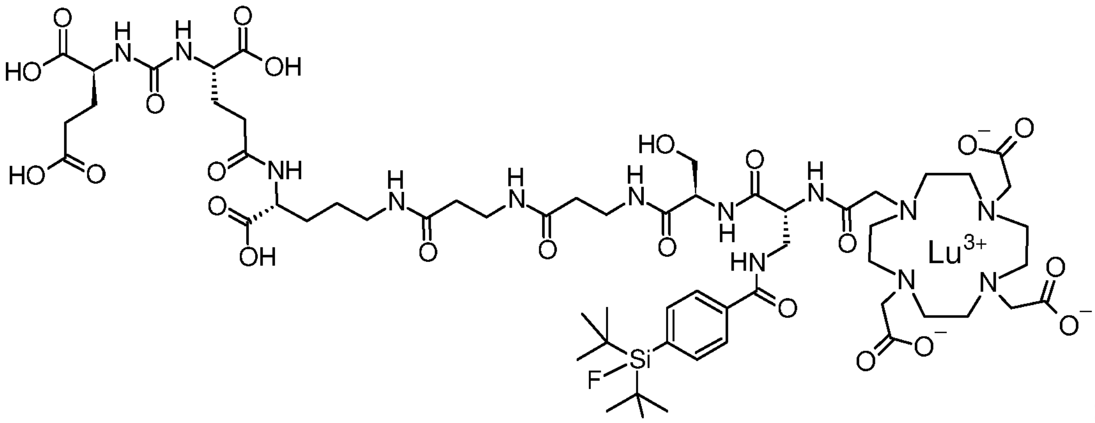

;

or a pharmaceutically acceptable salt thereof, wherein the fluorine atom is optionally $^{18}F$.

The compound may be:

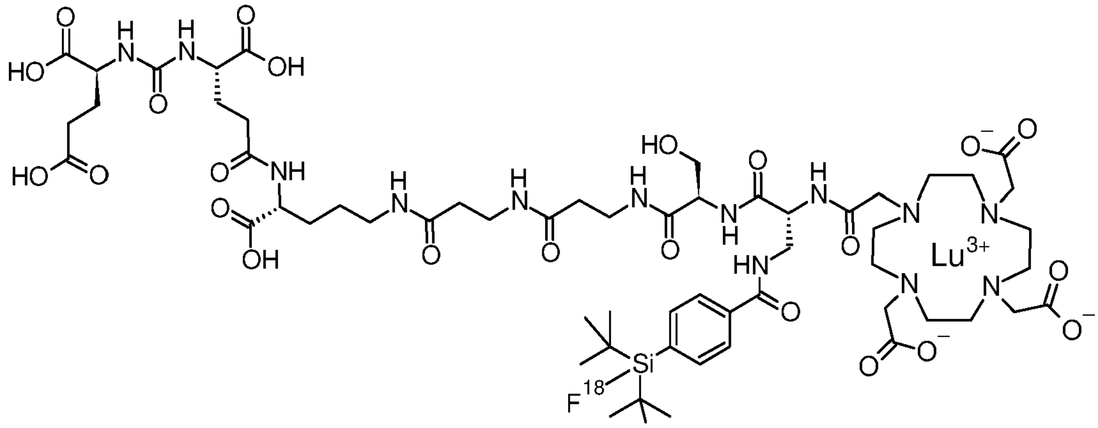

;

or a pharmaceutically acceptable salt thereof.

The compound may be:

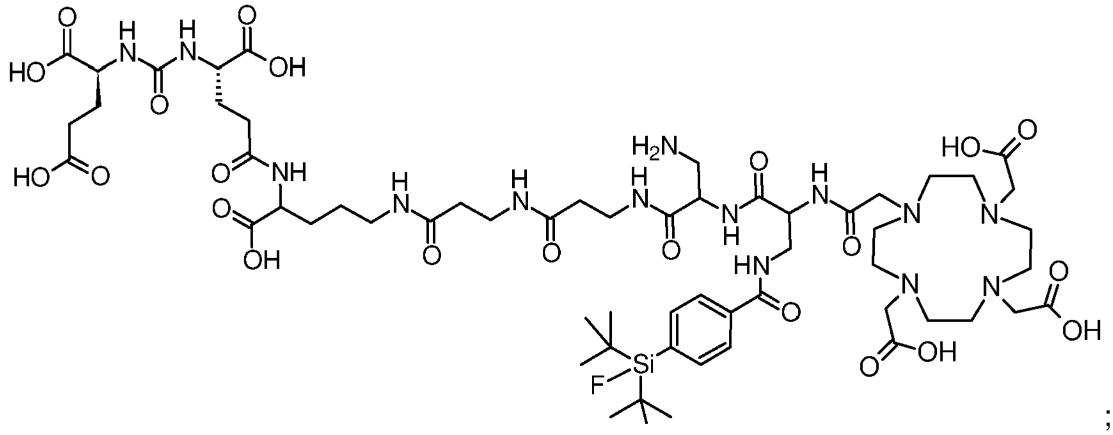

;

or a pharmaceutically acceptable salt or any individual isomer thereof, wherein the compound optionally contains a chelated nonradioactive or radioactive cation and wherein the fluorine atom is optionally $^{18}F$.

The compound may be:

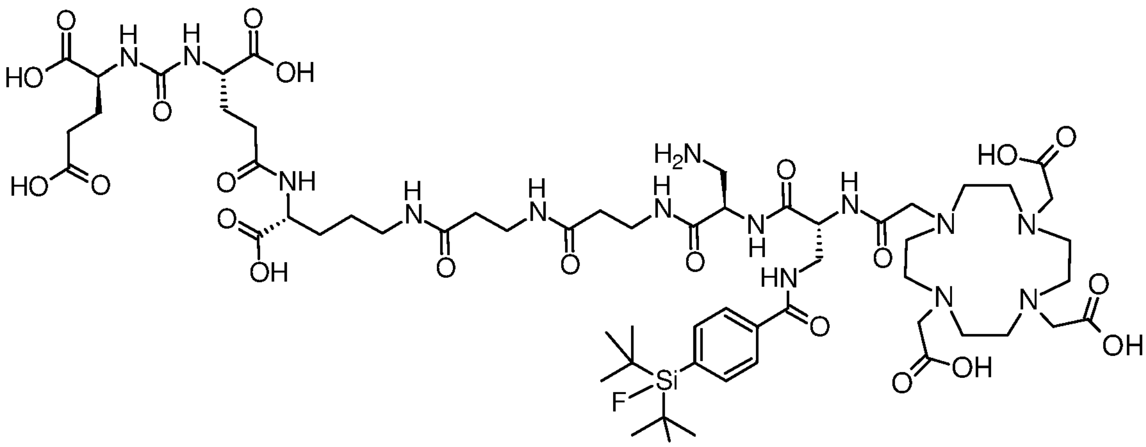

;

or a pharmaceutically acceptable salt thereof, wherein the compound optionally contains a chelated nonradioactive or radioactive cation and wherein the fluorine atom is optionally $^{18}F$.

The compound may be:

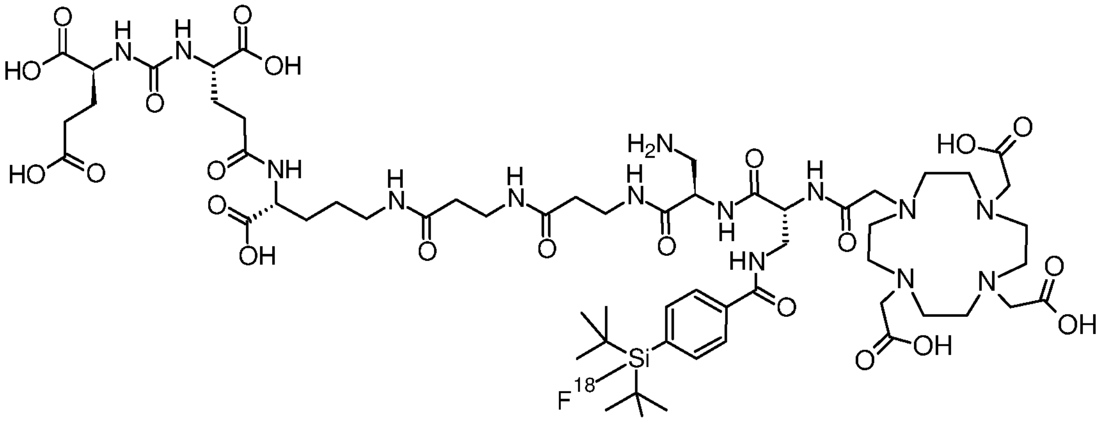

;

or a pharmaceutically acceptable salt thereof, wherein the compound optionally contains a chelated nonradioactive or radioactive cation.

The compound may be:

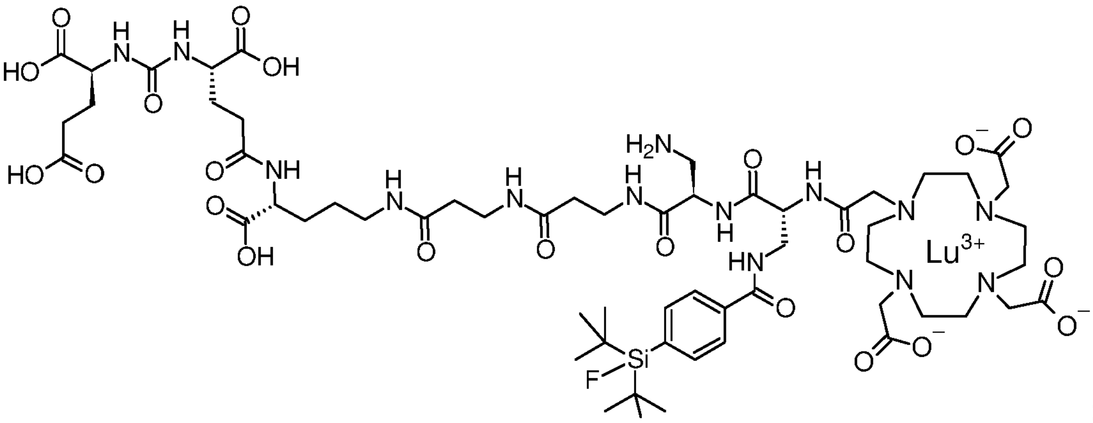

;

or a pharmaceutically acceptable salt thereof, wherein the fluorine atom is optionally $^{18}F$.

The compound may be:

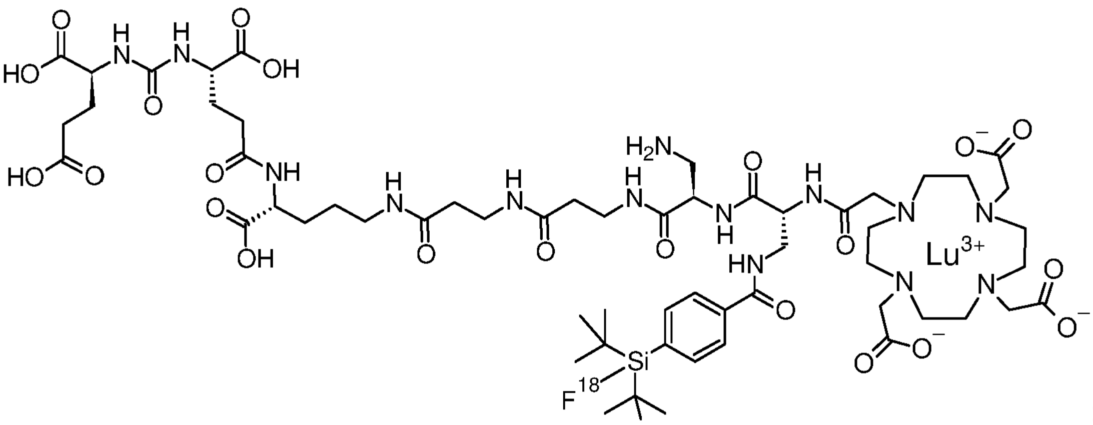

;

or a pharmaceutically acceptable salt thereof.

The compound may be:

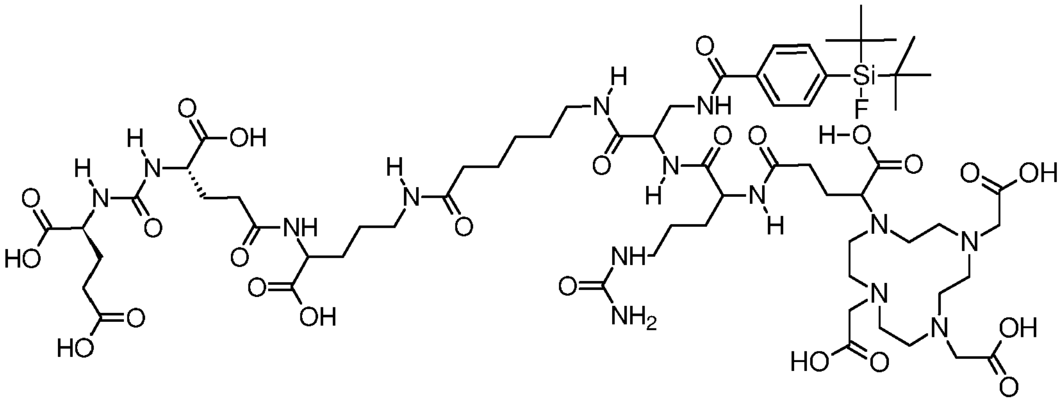

;

or a pharmaceutically acceptable salt or any individual isomer thereof, wherein the compound optionally contains a chelated nonradioactive or radioactive cation and wherein the fluorine atom is optionally $^{18}F$.

The compound may be:

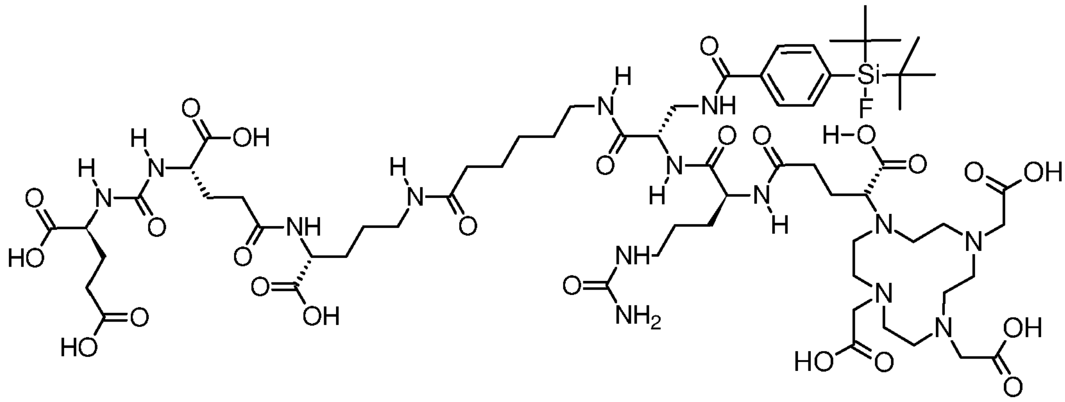

;

or a pharmaceutically acceptable salt thereof, wherein the compound optionally contains a chelated nonradioactive or radioactive cation and wherein the fluorine atom is optionally $^{18}F$.

The compound may be:

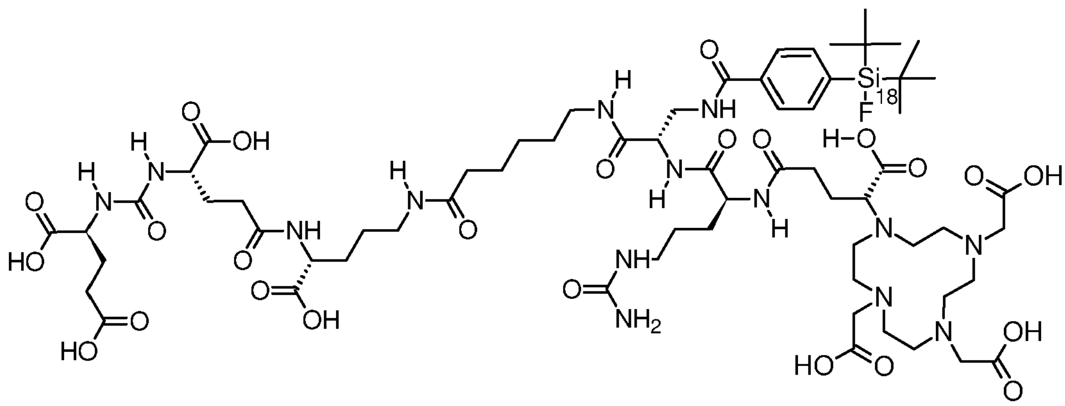

;

or a pharmaceutically acceptable salt thereof, wherein the compound optionally contains a chelated nonradioactive or radioactive cation.

The compound may be:

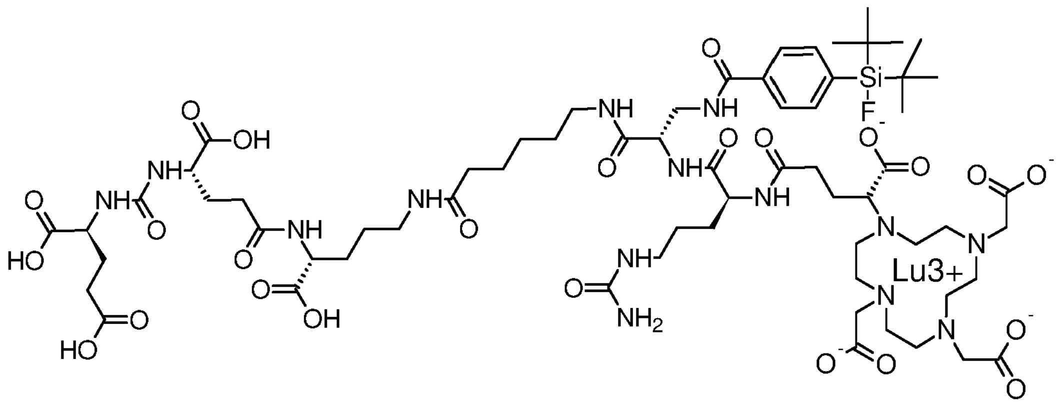

;

or a pharmaceutically acceptable salt thereof, wherein the fluorine atom is optionally $^{18}F$.

The compound may be:

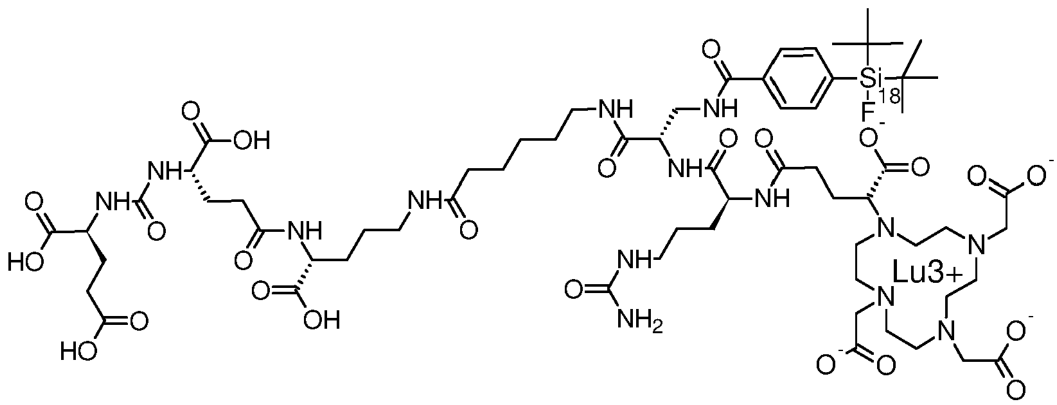

;

or a pharmaceutically acceptable salt thereof.

The compound may be:

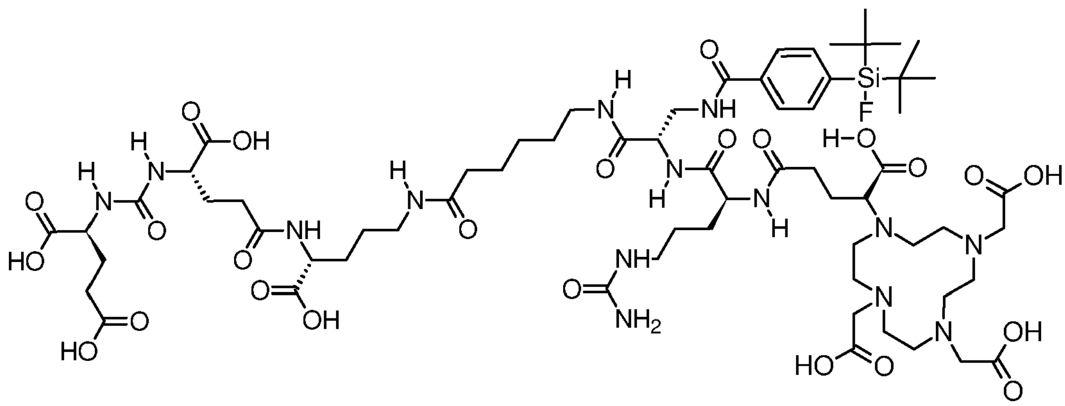

;

or a pharmaceutically acceptable salt thereof, wherein the compound optionally contains a chelated nonradioactive or radioactive cation and wherein the fluorine atom is optionally $^{18}F$.

The compound may be:

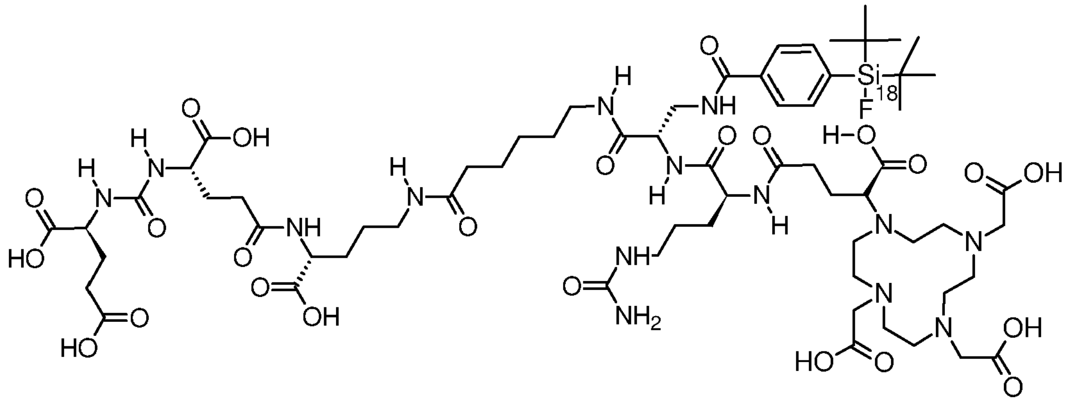

or a pharmaceutically acceptable salt thereof, wherein the compound optionally contains a chelated nonradioactive or radioactive cation.

The compound may be:

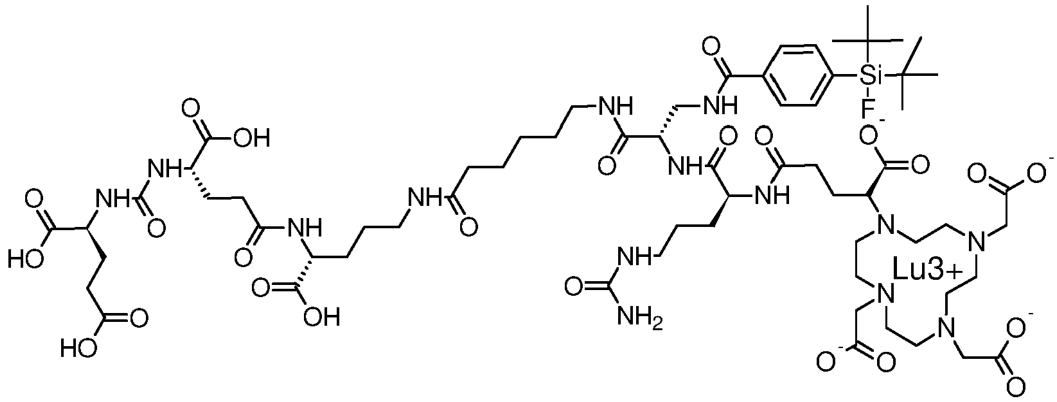

or a pharmaceutically acceptable salt thereof, wherein the fluorine atom is optionally $^{18}F$.

The compound may be:

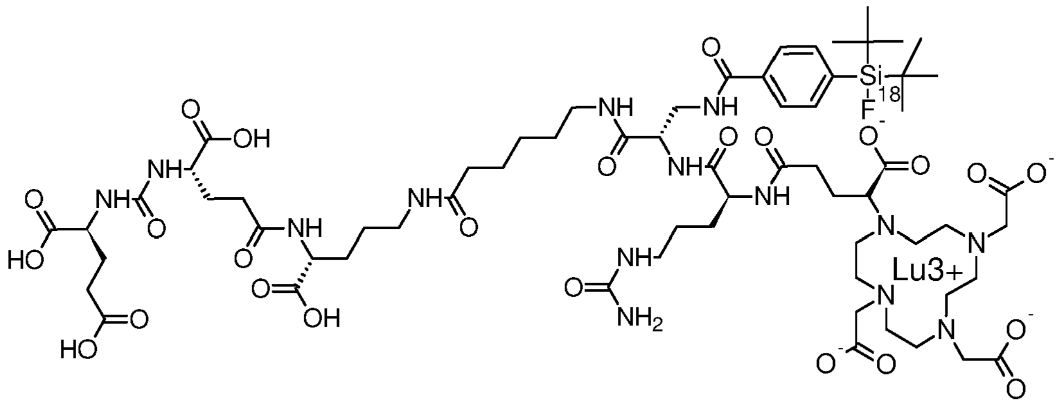

or a pharmaceutically acceptable salt thereof.

Preferred labelling schemes for these most preferred compounds are as defined herein above.

Also provided is a pharmaceutical imaging composition comprising or consisting of one or more compounds of the invention as disclosed herein above.

Also provided is a diagnostic composition comprising or consisting of one or more compounds of the invention as disclosed herein above.

The pharmaceutical composition may further comprise pharmaceutically acceptable carriers, excipients and/or diluents. Examples of suitable pharmaceutical carriers, excipients and/or diluents are well known in the art and include

phosphate buffered saline solutions, water, emulsions, such as oil/water emulsions, various types of wetting agents, sterile solutions etc. Compositions comprising such carriers can be formulated by well-known conventional methods. These pharmaceutical compositions can be administered to the subject at a suitable dose. Administration of the suitable compositions may be effected in different ways, e.g., by intravenous, intraperitoneal, subcutaneous, intramuscular, topical, intradermal, intranasal or intrabronchial administration. It is particularly preferred that said administration is carried out by injection and/or delivery, e.g., to a site in the pancreas or into a brain artery or directly into brain tissue. The compositions may also be administered directly to the target site, e.g., by biolistic delivery to an external or internal target site, like the pancreas or brain. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Pharmaceutically active matter may be present in an effective therapeutic amount, which may be between 0.1 ng and 10 mg/kg body weight per dose; however, doses below or above this exemplary range are envisioned, especially considering the aforementioned factors.

Also provided is one or more compounds of the invention as disclosed herein above for use in diagnostic medicine.

Preferred uses in medicine are in nuclear medicine such as nuclear diagnostic imaging, also named nuclear molecular imaging, and/or targeted radiotherapy of diseases associated with an overexpression, preferably of PSMA on the diseased tissue.

Also provided is a compound of the invention as defined herein above for use in a method of diagnosing and/or staging cancer, preferably prostate cancer. Prostate cancer is not the only cancer to express PSMA. Nonprostate cancers to demonstrate PSMA expression include breast, lung, colorectal, and renal cell carcinoma. Thus any compound described herein having a PSMA binding moiety can be used in the diagnosis, imaging or treatment of a cancer having PSMA expression.

Preferred indications are the detection or staging of cancer, such as, but not limited high grade gliomas, lung cancer and especially prostate cancer and metastasized prostate cancer, the detection of metastatic disease in patients with primary prostate cancer of intermediate-risk to high-risk, and the detection of metastatic sites, even at low serum PSA values in patients with biochemically recurrent prostate cancer. Another preferred indication is the imaging and visualization of neoangiogensis.

Also provided is a compound of the invention as defined herein above for use in a method of diagnosing and/or staging cancer, preferably prostate cancer.

Also provided is a pharmaceutical or diagnostic composition comprising or consisting of one or more compounds of Formula (1). The compounds of the invention may be for use as a cancer diagnostic or imaging agent. Accordingly also provided is a method of imaging and/or diagnosing cancer comprising administering a compound of Formula (1) or a composition comprising a compound of Formula (1). The compounds or compositions of the invention may be for use in the treatment of cancer. The compounds or compositions of the invention may be for use in the diagnosis, imaging or prevention of neoangiogenesis/angiogenesis. The compounds or compositions of the invention may be for use as a cancer diagnostic or imaging agent or for use in the treatment of cancer wherein the cancer is prostate, breast, lung, colorectal or renal cell carcinoma.

The term "treatment", in relation to the uses of any of the compounds described herein, including those of Formula (1) is used to describe any form of intervention where a compound is administered to a subject suffering from, or at risk of suffering from, or potentially at risk of suffering from the disease or disorder in question. Thus, the term "treatment" covers both preventative (prophylactic) treatment and treatment where measurable or detectable symptoms of the disease or disorder are being displayed.

The term "effective therapeutic amount" (for example in relation to methods of treatment of a disease or condition) refers to an amount of the compound which is effective to produce a desired therapeutic effect.

References to chemical functional groups are to be interpreted in their conventional sense (e.g. as defined in the IUPAC Gold Book), unless indicated otherwise. "optionally substituted" as applied to any group means that the said group may if desired be substituted with one or more substituents, which may be the same or different.

To the extent that any of the compounds described have chiral centres, the present invention extends to all optical isomers of such compounds, whether in the form of racemates or resolved enantiomers. The invention described herein relates to all crystal forms, solvates and hydrates of any of the disclosed compounds however so prepared. To the extent that any of the compounds disclosed herein have acid or basic centres such as carboxylates or amino groups, then all salt forms of said compounds are included herein. In the case of pharmaceutical uses, the salt should be seen as being a pharmaceutically acceptable salt.

Salts or pharmaceutically acceptable salts that may be mentioned include acid addition salts and base addition salts as well as salt forms arising due to the presence of the chelated nonradioactive or radioactive cation. Such salts may be formed by conventional means, for example by reaction of a free acid or a free base form of a compound with one or more equivalents of an appropriate acid or base, optionally in a solvent, or in a medium in which the salt is insoluble,

followed by removal of said solvent, or said medium, using standard techniques (e.g. in vacuo, by freeze-drying or by filtration). Salts may also be prepared by exchanging a counter-ion of a compound in the form of a salt with another counter-ion, for example using a suitable ion exchange resin.

Beyond the suitable chelated nonradioactive or radioactive cations described herein above, further examples of pharmaceutically acceptable salts include acid addition salts derived from mineral acids and organic acids, and salts derived from metals such as sodium, magnesium, potassium and calcium.

Examples of acid addition salts include acid addition salts formed with acetic, 2,2-dichloroacetic, adipic, alginic, aryl sulfonic acids (e.g. benzenesulfonic, naphthalene-2-sulfonic, naphthalene-1,5-disulfonic and p-toluenesulfonic), ascorbic (e.g. L-ascorbic), L-aspartic, benzoic, 4-acetamidobenzoic, butanoic, (+) camphoric, camphor-sulfonic, (+)-(1S)-camphor-10-sulfonic, capric, caproic, caprylic, cinnamic, citric, cyclamic, dodecylsulfuric, ethane-1,2-disulfonic, ethanesulfonic, 2-hydroxyethanesulfonic, formic, fumaric, galactaric, gentisic, glucoheptonic, gluconic (e.g. D-gluconic), glucuronic (e.g. D-glucuronic), glutamic (e.g. L-glutamic), $\alpha$-oxoglutaric, glycolic, hippuric, hydrobromic, hydrochloric, hydriodic, isethionic, lactic (e.g. (+)-L-lactic and ($\pm$)-DL-lactic), lactobionic, maleic, malic (e.g. (-)-L-malic), malonic, ($\pm$)-DL-mandelic, metaphosphoric, methanesulfonic, 1-hydroxy-2-naphthoic, nicotinic, nitric, oleic, orotic, oxalic, palmitic, pamoic, phosphoric, propionic, L-pyroglutamic, salicylic, 4-amino-salicylic, sebacic, stearic, succinic, sulfuric, tannic, tartaric (e.g.(+)-L-tartaric), thiocyanic, undecylenic and valeric acids.

Also encompassed are any solvates of the compounds and their salts. Preferred solvates are solvates formed by the incorporation into the solid state structure (e.g. crystal structure) of the compounds of the invention of molecules of a non-toxic pharmaceutically acceptable solvent (referred to below as the solvating solvent). Examples of such solvents may include water, alcohols (such as ethanol, isopropanol and butanol) and dimethylsulfoxide. Solvates can be prepared by recrystallising the compounds of the invention with a solvent or mixture of solvents containing the solvating solvent. Whether or not a solvate has been formed in any given instance can be determined by subjecting crystals of the compound to analysis using well known and standard techniques such as thermogravimetric analysis (TGA), differential scanning calorimetry (DSC) and X-ray crystallography.

The solvates can be stoichiometric or non-stoichiometric solvates. Particular solvates may be hydrates, and examples of hydrates include hemihydrates, monohydrates and dihydrates. For a more detailed discussion of solvates and the methods used to make and characterise them, see Bryn et al, Solid-State Chemistry of Drugs, Second Edition, published by SSCI, Inc of West Lafayette, IN, USA, 1999, ISBN 0-967-06710-3.

The compounds of the invention may contain one or more isotopic substitutions, and a reference to a particular element includes within its scope all isotopes of the element. For example, a reference to hydrogen includes within its scope 1H, 2H (D), and 3H (T). Similarly, references to carbon and oxygen include within their scope respectively 12C, 13C and 14C and 160 and 180. In an analogous manner, a reference to a particular functional group also includes within its scope isotopic variations, unless the context indicates otherwise. For example, a reference to an alkyl group such as an ethyl group or an alkoxy group such as a methoxy group also covers variations in which one or more of the hydrogen atoms in the group is in the form of a deuterium or tritium isotope, e.g. as in an ethyl group in which all five hydrogen atoms are in the deuterium isotopic form (a perdeuteroethyl group) or a methoxy group in which all three hydrogen atoms are in the deuterium isotopic form (a trideuteromethoxy group). The isotopes may be radioactive or non-radioactive.

## PREPARATION OF THE COMPOUNDS OF THE INVENTION

Some compounds of Formula (1) and derivatives or synthetic intermediates thereof can be prepared in accordance with synthetic methods known to the skilled person. In some embodiments, the invention provides a process for the preparation of a compound as defined in Formula (1). Compound 2C013 of the invention may be prepared according to the methods described below. $^{nat}Lu$ refers to naturally occurring non radioactive Lutetium which is composed of $^{175}Lu$ and $^{176}Lu$. $^{177}Lu$ refers to radioactive Lutetium 177.

### Solid Phase Peptide Synthesis

*TCP-resin loading (General Procedure 1a (GP1a))*

Loading of the tritylchloride polystyrene (TCP) resin with a Fmoc-protected amino acid (AA) was carried out by stirring a solution of the TCP-resin (1.60 mmol/g) and Fmoc-AA-OH (1.5 eq.) in anhydrous DCM with DIPEA (3.8 eq.) at room temperature for 2 h. Remaining tritylchloride was capped by the addition of methanol (2 mL/g resin) for 15 min. Subsequently the resin was filtered and washed with DCM (2 $\times$ 5 mL/g resin), DMF (2 $\times$ 5 mL/g resin) and methanol (5 mL/g resin) and dried *in vacuo.* Final loading *l* of Fmoc-AA-OH was determined by the following equation:

$$l\left[\frac{mmol}{g}\right]=\frac{(m_2-m_1)\times 1000}{(M_W-M_{HCl})\,m_2}$$

$m_2$ = mass of loaded resin [g]

$m_1$ = mass of unloaded resin [g]

$M_w$ = molecular weight of AA [g/mol]

*On-resin Amide Bond Formation (GP2)*

For conjugation of a building block to the resin-bound peptide, a mixture of TBTU with HOBt or HOAt is used for pre-activation of the carboxylic with DIPEA or 2,4,6-trimethylpyridine as a base in DMF (10 mL/g resin). After 5 min at rt, the solution was added to the swollen resin. The exact stoichiometry and reaction time for each conjugation step is given in the respective synthesis protocols. After reaction, the resin was washed with DMF (6 × 5 mL/g resin).

*On-resin Fmoc-deprotection (GP3)*

The resin-bound Fmoc-peptide was treated with 20% piperidine in DMF (*v*/*v*, 8 mL/g resin) for 5 min and subsequently for 15 min. Afterwards, the resin was washed thoroughly with DMF (8 × 5 mL/g resin).

*On-resin Dde-deprotection (GP4)*

The Dde-protected peptide was dissolved in a solution of 2% hydrazine monohydrate in DMF (*v*/*v*, 5 mL/g resin) and shaken for 20 min (GP4a). In the case of present Fmoc-groups, Dde-deprotection was performed by adding a solution of imidazole (0.92 g/g resin), hydroxylamine hydrochloride (1.26 g/g resin) in NMP (5.0 mL/g resin) and DMF (1.0 mL/g resin) for 3 h at room temperature (GP4b). After deprotection the resin was washed with DMF (8 × 5 mL/g resin).

*On-resin acetylation (GP6)*

A mixture of NMP/acetic anhydride/DIPEA (85/10/5, *v*/*v*/*v*) was added to the resin for 15 min. Afterwards, the resin was washed thoroughly with DMF (6 × 5 mL/g resin).

**2C013**

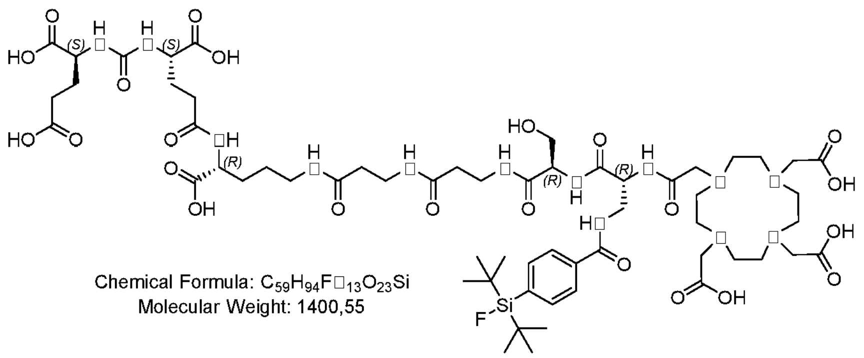

Chemical Formula: $C_{59}H_{94}F\square_{13}O_{23}Si$
Molecular Weight: 1400,55

The synthesis was carried out on 2-chlorotrityl chloride resin (1.60 mmol/g). Fmoc-D-Orn(Dde)-OH (2.75 eq.) was loaded to the resin as first building block according to GP1a, and after subsequent Fmoc cleavage (GP3), (*t*BuO)EuE(O*t*Bu)$_2$ (2.0 eq.) was conjugated with TBTU (2.0 eq.), HOAt (2.0 eq.) and DIPEA (6.0 eq.) in DMF for 4.5 h (GP2). Deprotection of Dde was carried out with a mixture of hydrazine in DMF (GP4a). Subsequently, Fmoc-β-Ala-OH (2.0 eq.) was conjugated with TBTU (2.0 eq.), HOAt (2.0 eq.) and DIPEA (6.0 eq.) in DMF for 2.5 h (GP2), followed by acetylation of unreacted amines with acetic anhydride and DIPEA in NMP (GP6) and subsequent Fmoc deprotection (GP3). Coupling of Fmoc-β-Ala-OH (GP2), acetylation (GP6) and Fmoc deprotection (GP3) was carried out a second time as described above, followed by conjugation of Fmoc-D-Ser(tBu)-OH with TBTU (2.0 eq.), HOAt (2.0 eq.) and DIPEA (6.0 eq.) in DMF for 2.5 h (GP2). After Fmoc deprotection (GP3), Fmoc-D-Dap(Dde)-OH (2.0 eq.) was conjugated with TBTU (2.0 eq.), HOAt (2.0 eq.) and 2,4,6-trimethylpyridine (6.7 eq.) in DMF for 2.5 h (GP2). Orthogonal deprotection of Dde was carried out

using hydroxylamin hydrochloride and imidazole in a mixture of DMF and NMP for 3.5 h (GP4b). Subsequently, SiFA-BA (2.0 eq.) was pre-activated in a mixture of TBTU (2.0 eq), HOAt (2.0 eq.) and DIPEA (6.0 eq.) in DMF, added to the resin and left to react for 2.5 h (GP2). The remaining Fmoc protecting group was cleaved according to GP3 and DOTA(*t*Bu)$_3$ (2.0 eq.) was conjugated with TBTU (2.0 eq.), HOAt (2.0 eq.) and DIPEA (6.0 eq.) in DMF for 2.5 h (GP2) and the resulting solution was left at RT over night to achieve quantitative deprotection of acid-labile protecting groups. After purification by semipreparative RP-HPLC, 2C013 was obtained as colourless, amorphous solid (11%). RP-HPLC (10-70% B in 15 min): $t_R$ = 10.1 min, K' = 4.73. Calculated monoisotopic mass ($C_{59}H_{94}FN_{13}O_{23}Si$): 1399.6; found: m/z = 1400.0 $[M+H]^+$, 700.3 $[M+2H]^{2+}$.

**[$^{nat}$Lu]Lu-2C013**

The corresponding 2C013 $^{nat}$Lu-complex was prepared from a 2 mM solution of 2C013 (1.0 eq.) in DMSO with a 20 mM aqueous solution of $LuCl_3$ (2.5 eq.), heated to 95 °C for 30 min. After cooling, the $^{nat}$Lu-chelate formation was confirmed using RP-HPLC and MS. RP-HPLC (10-70% B in 15 min): $t_R$ = 10.0 min, K' = 5.00. Calculated monoisotopic mass ($C_{59}H_{91}FLuN_{13}O_{23}Si$): 1571.5; found: m/z = 1572.4 $[M+H]^+$, 786.3 $[M+2H]^{2+}$.

**2C011**

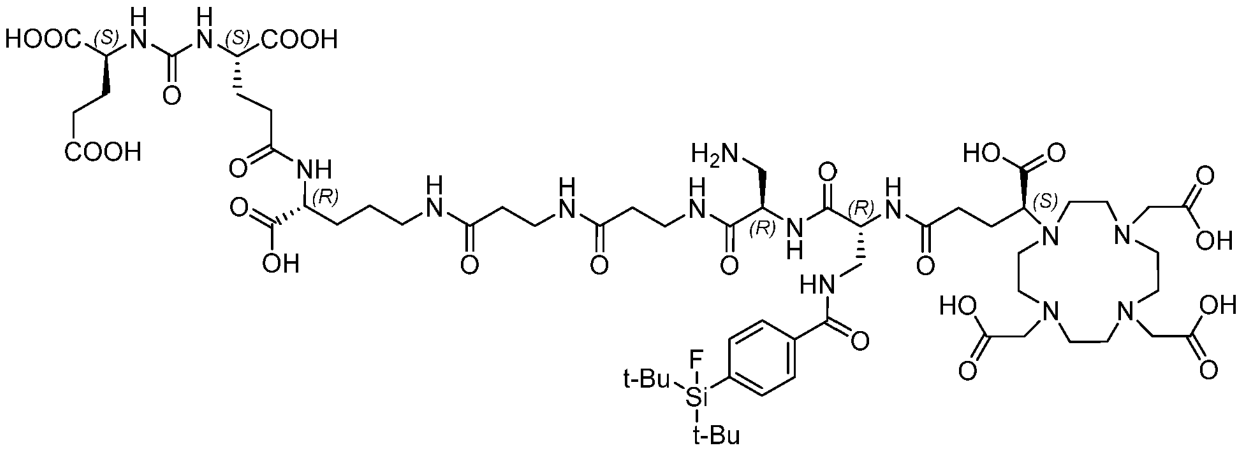

Chemical Formula: $C_{62}H_{99}FN_{14}O_{24}Si$
Molecular Weight: 1471,63

Initially analagous to 2C013, replacing the Fmoc-D-Ser(tBu)-OH with Fmoc-D-Dap(Boc)-OH (1.5 eq.) pre-activated with HOAt (1.5 eq.), TBTU (1.5 eq.) and DIPEA (4.5 eq.) in DMF for 5 min and coupled to the resin for 2 h (GP2). Subsequent installation of the radiohybrid unit D-Dap(SiFA)-(S)-DOTA-GA was achieved according to the synthesis above starting with the coupling of Fmoc-D-Dap(Dde)-OH. The cleaved, dried crude product was purified by RP-HPLC. RP-HPLC (10 to 90% B in 15 min): $t_R$ = 8.0 min. K' = 3.5. Calculated monoisotopic mass ($C_{62}H_{99}FN_{14}O_{24}Si$): 1470.7 found: m/z = 1471.9 $[M+H]^+$, 736.3 $[M+2H]^{2+}$.

**2C014**

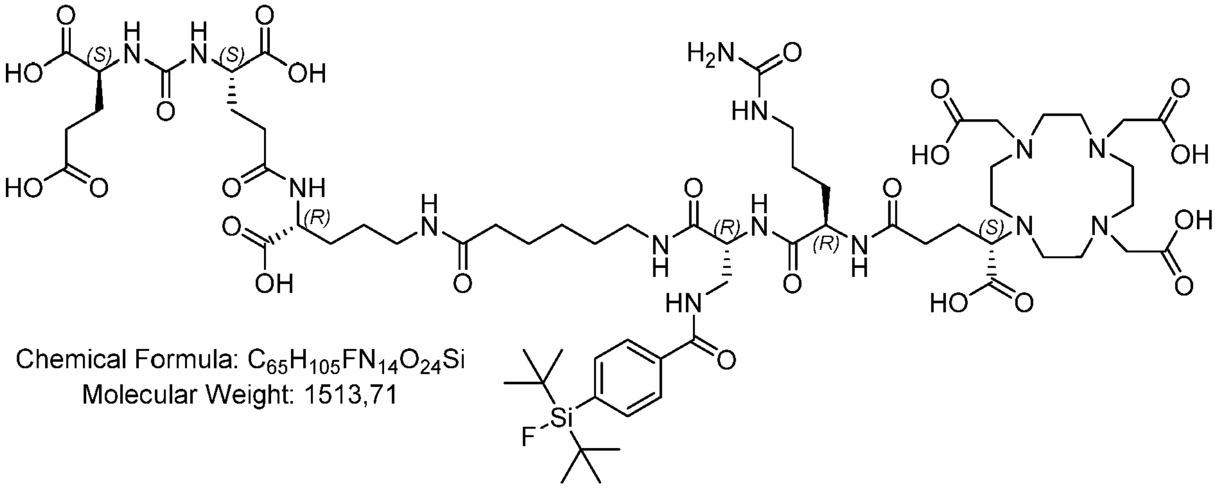

Chemical Formula: $C_{65}H_{105}FN_{14}O_{24}Si$
Molecular Weight: 1513,71

The synthesis was carried out on 2-chlorotrityl chloride resin (1.60 mmol/g). Fmoc-D-Orn(Dde)-OH (2.75 eq.) was loaded to the resin as first building block according to GP1a, and after subsequent Fmoc cleavage (GP3), (*t*BuO) EuE(O*t*Bu)$_2$ (2.0 eq.) was conjugated with TBTU (2.0 eq.), HOAt (2.0 eq.) and DIPEA (6.0 eq.) in DMF for 4.5 h (GP2). Deprotection of Dde was carried out with a mixture of hydrazine in DMF (GP4a). Subsequently, Fmoc-Ahx-OH (2.0 eq.) was conjugated with TBTU (2.0 eq.), HOAt (2.0 eq.) and DIPEA (6.0 eq.) in DMF for 2.5 h (GP2). After Fmoc deprotection (GP3), Fmoc-D-Dap(Dde)-OH (2.0 eq.) was conjugated with TBTU (2.0 eq.), HOAt (2.0 eq.) and 2,4,6-trimethylpyridine (6.7 eq.) in DMF for 2.5 h (GP2). Orthogonal deprotection of Dde was carried out using hydroxylamin hydrochloride and imidazole in a mixture of DMF and NMP for 3.5 h (GP4b). Subsequently, SiFA-BA (2.0 eq.) was conjugated with TBTU (2.0 eq.), HOAt (2.0 eq.) and DIPEA (6.0 eq.) in DMF for 2.5 h (GP2). Fmoc deprotection (GP3),followed by conjugation of Fmoc-D-Cit-OH (2.0 eq.) with TBTU (2.0 eq), HOAt (2.0 eq.) and DIPEA (6.0 eq.) in DMF for 2.5 h (GP2) followed by subsequent Fmoc cleavage (GP3), before final conjugation of (*S*)-DOTAGA(*t*Bu)$_4$. After purification by semipreparative RP-HPLC, 2C014 was obtained as colourless, amorphous solid (33%). *RP*-HPLC (10-70% B in 15 min): $t_R$ = 10.3 min, K' = 4.76. Calculated monoisotopic mass ($C_{65}H_{105}FN_{14}O_{24}Si$): 1512.7; found: m/z = 1514.4 $[M+H]^+$, 757.6 $[M+2H]^{2+}$.

## 2C015

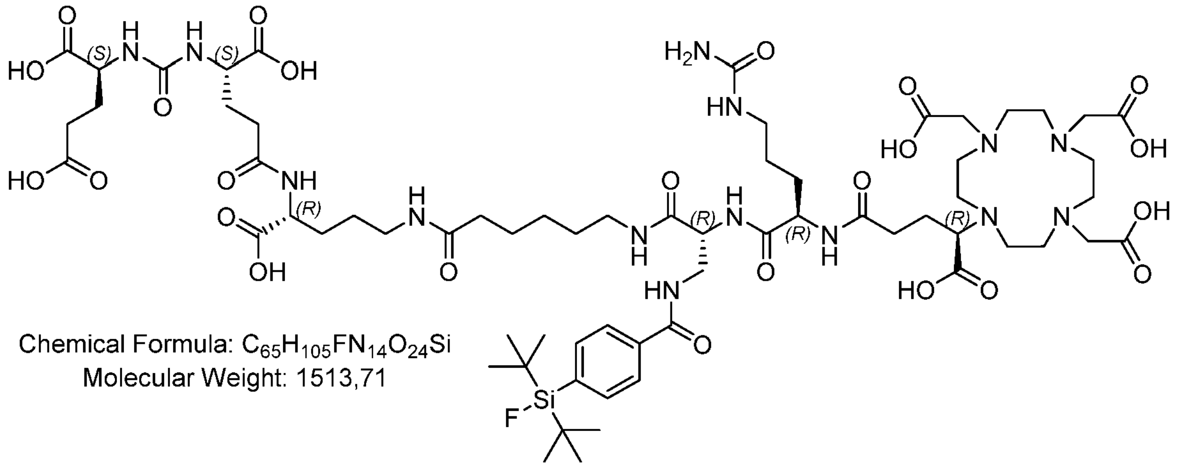

Chemical Formula: $C_{65}H_{105}FN_{14}O_{24}Si$
Molecular Weight: 1513,71

The synthesis of 2C015 was carried out in analogy to 2C014, only differing by conjugation of (*R*)-DOTAGA(*t*Bu)$_4$. After purification by semipreparative RP-HPLC, 2C015 was obtained as colourless, amorphous solid (33%). RP-HPLC (10-70% B in 15 min): $t_R$ = 10.2 min, K' = 4.81. Calculated monoisotopic mass ($C_{65}H_{105}FN_{14}O_{24}Si$): 1512.7; found: m/z = 1513.4 $[M+H]^+$, 757.6 $[M+2H]^{2+}$.

## *In Vitro* Experiments

### Cell Culture

PSMA-positive LNCAP cells (300265; Cell Lines Service, Eppelheim, Germany) were cultivated in Dulbecco modified Eagle medium/Nutrition Mixture F-12 with Glutamax (1 : 1) (DMEM-F12, Biochrom, Berlin, Germany) supplemented with fetal bovine serum (10%, FBS Zellkultur, Berlin, Germany) and kept at 37°C in a humidified $CO_2$ atmosphere (5%). A mixture of trypsin and EDTA (0.05%, 0.02%) in PBS (Biochrom) was used in order to harvest cells. Cells were counted with a Neubauer hemocytometer (Paul Marienfeld, Lauda-Königshofen, Germany).

### Affinity Determinations ($IC_{50}$)

For PSMA affinity ($IC_{50}$) determinations, the respective ligand was diluted (serial dilution $10^{-4}$ to $10^{-10}$) in Hank's balanced salt solution (HBSS, Biochrom). In the case of metal-complexed ligands, the crude reaction mixture was diluted analogously, without further purification. Cells were harvested 24 $\pm$ 2 hours prior to the experiment and seeded in 24-well plates (1.5 $\times$ $10^5$ cells in 1 mL/well). After removal of the culture medium, the cells were carefully washed with 500 $\mu$L of HBSS, supplemented with 1% bovine serum albumin (BSA, Biowest, Nuaillé, France) and left 15 min on ice for equilibration in 200 $\mu$L HBSS (1% BSA). Next, 25 $\mu$L per well of solutions, containing either HBSS (1% BSA, control) or the respective ligand in increasing concentration ($10^{-10}$ - $10^{-4}$ M in HBSS) were added with subsequent addition of 25 $\mu$L of [$^{125}$I]I-BA-KuE (2.0 nM) in HBSS (1% BSA). After incubation on ice for 60 min, the experiment was terminated by removal of the medium and consecutive rinsing with 200 $\mu$L of HBSS (1% BSA). The media of both steps were combined in one fraction and represent the amount of free radioligand. Afterwards, the cells were lysed with 250 $\mu$L of 1 M aqueous NaOH for at least 10 min. After a washing step (250 $\mu$L of 1 M NaOH), both fractions, representing the amount of bound ligand, were united. Quantification of all collected fractions was accomplished in a $\gamma$-counter. PSMA-affinity determinations were carried out at least three times per ligand.

### Internalization Studies

For internalization studies, LNCaP cells were harvested 24 $\pm$ 2 hours before the experiment and seeded in poly-L-lysine coated 24-well plates (1.25 $\times$ 105 cells in 1 mL/well, Greiner Bio-One, Kremsmünster, Austria). After removal of the culture medium, the cells were washed once with 500 $\mu$L DMEM-F12 (5% BSA) and left to equilibrate for at least 15 min at 37 °C in 200 $\mu$L DMEM-F12 (5% BSA). Each well was treated with either 25 $\mu$L of either DMEM-F12 (5% BSA, control) or 25 $\mu$L of a 100 $\mu$M PMPA (2-(Phosphonomethyl)-pentandioic acid, Tocris Bioscience, Bristol, UK) solution in PBS, for blockade. Next, 25 $\mu$L of the radioactive-labelled PSMA inhibitor (10.0 nM in DMEM-F12 (5% BSA)) was added and the cells were incubated at 37 °C for 60 min. The experiment was terminated by placing the 24-well plate on ice for 3 min and consecutive removal of the medium. Each well was carefully washed with 250 $\mu$L of ice-cold HBSS. Both fractions from the first steps, representing the amount of free radioligand, were combined. Removal of surface bound activity was accomplished by incubation of the cells with 250 $\mu$L of ice-cold PMPA (10 $\mu$M in PBS) solution for 5 min and rinsed again with another 250 $\mu$L of ice-cold PBS. The internalized activity was determined by incubation of the cells in 250 $\mu$L 1 M aqueous NaOH for at least 10 min. The obtained fractions were combined with those of the subsequent wash step with 250 $\mu$L 1 M aqueous NaOH. Each experiment (control and blockade) was performed in triplicate. Free, surface bound and internalized activity was quantified in a $\gamma$-counter. All internalization studies were accompanied by external reference studies, using ([$^{125}$I]I-BA)KuE (c = 0.2 nM), which were performed analogously. Data were corrected for non-specific binding and normalized to the specific-internalization observed for the radioiodinated reference compound.

### Octanol-Water Partition Coefficient ($logD_{7.4}$)

Approximately 1 MBq of the labelled tracer was dissolved in 1 mL of a 1:1 mixture (*v*/*v*) of PBS (pH 7.4) and n-octanol in a reaction vial (1.5 mL). After vigorous mixing of the suspension for 3 minutes at room temperature, the vial was centrifuged at 15000 g for 3 minutes (Biofuge 15, Heraus Sepatech, Osterode, Germany) and 100 $\mu$L aliquots of both layers were measured in a gamma counter. The experiment was repeated at least six times.

### Determination of Human Serum Albumin (HSA) Binding by High Performance Affinity Chromatography (HPAC)

HSA binding of the PSMA-addressing ligands was determined according to a previously published procedure via HPLC (Valko, K.; Nunhuck, S.; Bevan, C.; Abraham, M. H.; Reynolds, D. P. Journal of pharmaceutical sciences 2003, 92, 2236-2248). A Chiralpak HSA column (50 $\times$ 3 mm, 5 $\mu$m, H13H-2433, Daicel, Tokyo, Japan) was used at a constant flow

rate of 0.5 mL/min at rt. Mobile phase A was a freshly prepared 50 mM aqueous solution of $NH_4OAc$ (pH 6.9) and mobile phase B was isopropanol (HPLC grade, VWR). The applied gradient for all experiments was 100% A (0 to 3 min), followed by 80% A (3 to 40 min). Prior to the experiment, the column was calibrated using nine reference substances with a HSA binding, known from literature, in the range of 13 to 99% (Valko, K.; Nunhuck, S.; Bevan, C.; Abraham, M. H.; Reynolds, D. P. Journal of pharmaceutical sciences 2003, 92, 2236-2248; Yamazaki, K.; Kanaoka, M. Journal of Pharmaceutical sciences 2004, 93, 1480-1494). All substances, including the examined PSMA ligands, were dissolved in a 1:1 mixture *(v/v)* of isopropanol and a 50 mM aqueous solution of $NH_4OAc$ (pH 6.9) with a final concentration of 0.5 mg/mL. Non-linear regression was established with the OriginPro 2016G software (Northampton, United States) (Fig. 8).

**Biodistribution Studies**

Approximately 3-7 MBq (0.1-0.2 nmol) of the radioactive-labelled PSMA inhibitors were injected into the tail vein of LNCaP tumor-bearing male CB-17 SCID mice and sacrificed after 24 h post injection. Selected organs were removed, weighted and measured in a $\gamma$-counter. Results are all decay corrected and given in % injected dose per gram of tissue (% iD/g). Tumor-to-organ ratios are calculated from animals' individual ratios and are given as mean $\pm$ standard deviation.

**$\mu$SPECT/CT Imaging**

Static images were recorded of sacrificed mice, 24 h p.i. directly after blood collection, with an acquisition time of 45 min using the HE-GP-RM collimator and a step-wise multi-planar bed movement. For imaging studies, a MILabs VECTor4 small-animal SPECT/PET/OI/CT from MILabs (Utrecht, Netherlands) was applied. Furthermore, data were reconstructed using the MILabs-Rec software (version 10.02) and a pixel-based Similarity-Regulated Ordered Subsets Expectation Maximization (SROSEM) algorithm with a window-based scatter correction (20% below and 20% above the photopeak, respectively). Moreover, further data analysis was accomplished using the PMOD4.0 software acquired from PMOD TECHNOLOGIES LLC (Zurich, Switzerland) at defined settings (voxel size CT: 80 $\mu$m, voxel size SPECT: 0.8 mm, 1.6 mm (FWHM) Gaussian blurring post processing filter, with calibration factor in kBq/mL and decay correction and no attenuation correction).

**Table 1:** In vitro data of [$^{nat/177}$Lu]Lu-2C013 and the known references [$^{nat/177}$Lu]Lu-PSMA-617 and [$^{nat/177}$Lu]Lu-rhPSMA-7.3.

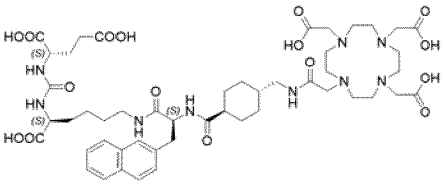

PSMA-617

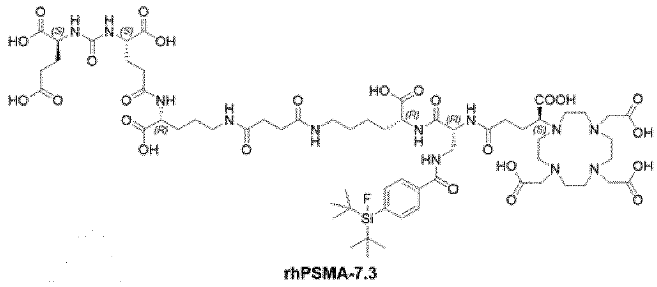

rhPSMA-7.3

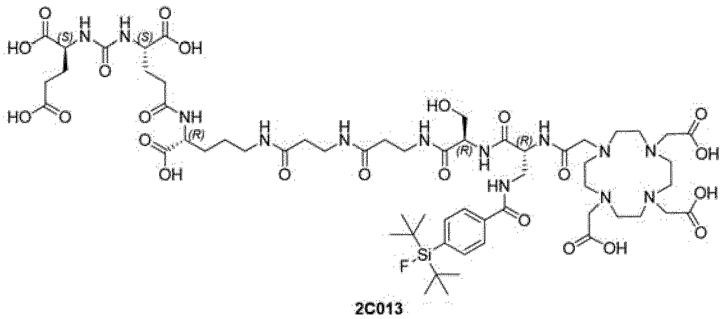

2C013

| | 2C013 | PSMA-617 | rhPSMA-7.3 |
|---|---|---|---|
| $logD_{7.4}$ | -3.30 $\pm$ 0.06 | -4.10 $\pm$ | -4.12 $\pm$ 0.11 |
| IC50 [nM] | 4.44 $\pm$ 1.61 | 3.3 $\pm$ 0.2 | 3.29 $\pm$ 1.00 |
| Internalization [%IBA-KuE] | 141 $\pm$ 8 | 203 $\pm$ 10 | 184 $\pm$ 12 |
| HSA (HPAC) [%] | 88.6 | 84.9 | 98.6 |

(continued)

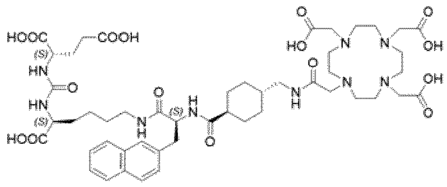

PSMA-617

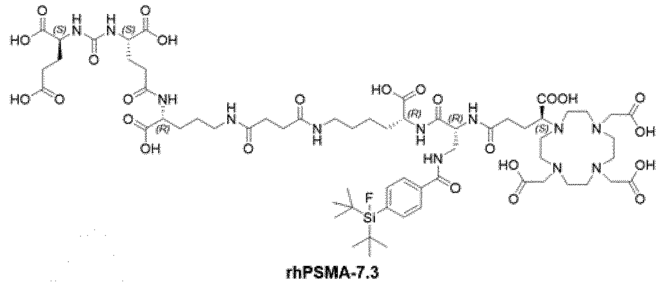

rhPSMA-7.3

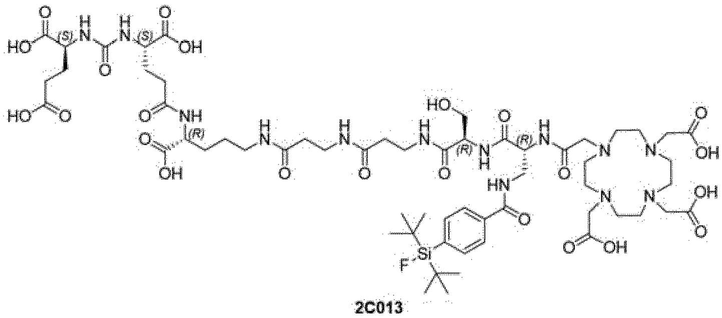

2C013

| | 2C013 | PSMA-617 | rhPSMA-7.3 |
|---|---|---|---|
| $MW_{app}$ [kDa] | 17.6 | 13.7 | 30.4 |
| Kidneys (24h) [%iD/g] | 0.95 ± 0.42 | 9.8 ± 2.7 | 1.4 ± 0.4 |
| Tumor (24h) [%iD/g] | 7.59 ±1.23 | 11.6 ± 2.2 | 7.5 ± 0.9 |
| T/K-ratio | 9.04 ± 3.09 | 1.24 ± 0.29 | 5.17 |

**Table 2:** Uptake data of [$^{nat/177}$Lu]Lu-2C013 and the known references [$^{nat/177}$Lu]Lu-PSMA-617 and [$^{nat/177}$Lu]Lu-rhPSMA-7.3, expressed as % injected dose per gram of tissue (%iD/g).

| | **[$^{177}$Lu]Lu-2C013 24 h p.i., n = 4** | | **[$^{177}$Lu]Lu-PSMA-617 24 h p.i., n = 4** | | **[$^{177}$Lu]Lu-rhPSMA-7.3 24 h p.i., n = 4** | |
|---|---|---|---|---|---|---|
| **uptake in % iD/g** | **mean** | **SD** | **mean** | **SD** | **mean** | **SD** |
| blood | 0.0013 | 0.0007 | 0.0056 | 0.0026 | 0.0032 | 0.0007 |
| heart | 0.0190 | 0.0036 | 0.0119 | 0.0043 | 0.0306 | 0.0093 |
| lung | 0.0438 | 0.0138 | 0.0393 | 0.0130 | 0.0491 | 0.0114 |
| liver | 0.2519 | 0.0643 | 0.1167 | 0.0567 | 0.2165 | 0.0402 |
| spleen | 0.3891 | 0.1184 | 0.0812 | 0.0063 | 0.7333 | 0.2328 |
| pancreas | 0.0165 | 0.0045 | 0.0106 | 0.0040 | 0.0247 | 0.0049 |
| stomach | 0.0846 | 0.0433 | 0.0201 | 0.0028 | 0.0592 | 0.0281 |
| intestine | 0.3354 | 0.2068 | 0.1185 | 0.0822 | 0.1325 | 0.0814 |
| kidney | 0.9488 | 0.4204 | 1.4421 | 0.4243 | 9.8160 | 2.7388 |
| adrenals | 0.0618 | 0.0170 | 0.1910 | 0.0711 | 0.5614 | 0.2215 |
| muscle | 0.0040 | 0.0011 | 0.0092 | 0.0018 | 0.0091 | 0.0031 |
| bone | 0.0370 | 0.0042 | 0.0267 | 0.0174 | 0.0475 | 0.0097 |
| tumor | 7.5897 | 1.2282 | 7.4568 | 0.8969 | 11.6274 | 2.2372 |
| parotid gl. | 0.0554 | 0.0103 | n.d. | n.d. | 0.1314 | 0.0424 |

(continued)

| | [$^{177}$Lu]Lu-2C013 24 h p.i., n = 4 | | [$^{177}$Lu]Lu-PSMA-617 24 h p.i., n = 4 | | [$^{177}$Lu]Lu-rhPSMA-7.3 24 h p.i., n = 4 | |
|---|---|---|---|---|---|---|
| **uptake in % iD/g** | **mean** | **SD** | **mean** | **SD** | **mean** | **SD** |
| submand. gl. | 0.0360 | 0.0038 | n.d. | n.d. | 0.0564 | 0.0103 |
| *n.d. = not determined* | | | | | | |

**Table 3:** Uptake data of [$^{nat/177}$Lu]Lu-2C013 and the known references [$^{nat/177}$Lu]Lu-PSMA-617 and [$^{nat/177}$Lu]Lu-rhPSMA-7.3, expressed as tumor-to-organ ratio.

| | [$^{177}$Lu]Lu-2C013 24 h p.i., n = 4 | | [$^{177}$Lu]Lu-PSMA-617 24 h p.i., n = 4 | | [$^{177}$Lu]Lu-rhPSMA-7.3 24 h p.i., n = 4 | |
|---|---|---|---|---|---|---|
| **tumor-to-organ ratio** | **mean** | **SD** | **mean** | **SD** | **mean** | **SD** |
| blood | 6891.48 | 2282.34 | 1423.84 | 454.65 | 3842.82 | 1145.20 |
| heart | 403.61 | 51.74 | 704.01 | 270.07 | 394.12 | 81.65 |
| lung | 189.80 | 67.60 | 210.40 | 74.00 | 239.49 | 24.05 |
| liver | 31.82 | 9.74 | 91.22 | 73.46 | 53.68 | 1.68 |
| spleen | 21.03 | 6.84 | 93.28 | 22.08 | 16.67 | 3.23 |
| pancreas | 492.10 | 159.57 | 798.52 | 292.83 | 487.03 | 132.64 |
| stomach | 145.92 | 139.74 | 375.80 | 65.16 | 261.74 | 176.88 |
| intestine | 60.29 | 82.18 | 90.33 | 49.69 | 129.10 | 86.48 |
| kidney | 9.04 | 3.09 | 5.85 | 2.99 | 1.24 | 0.29 |
| adrenals | 131.27 | 41.87 | 43.54 | 15.21 | 22.96 | 6.92 |
| muscle | 2075.70 | 817.71 | 855.38 | 278.54 | 1453.52 | 600.97 |
| bone | 208.29 | 52.48 | 471.67 | 378.45 | 263.53 | 110.54 |
| parotid gl. | 140.11 | 27.40 | n.d. | n.d. | 93.35 | 21.24 |
| submand. gl. | 211.04 | 26.91 | n.d. | n.d. | 206.71 | 26.61 |
| *n.d. = not determined* | | | | | | |

**Table 4:** Repeated dataset of *ex vivo* biodistribution studies of the compounds [$^{177}$Lu]rhPSMA-7.3 (n = 5), [$^{177}$Lu]2C013 (n = 5) and [$^{177}$Lu]PSMA-I&T (n = 5) at 24 h p.i. in male tumor-bearing CB17-SCID mice. Data expressed as percentage of the injected dose per gram (%ID/g), mean ± standard deviation.

| Activity accumulation [%ID/g] (complete dataset) | [177Lu]rhPSMA-7.3 24 h p.i., n = 5 | | [177Lu]2C013 24 h p.i., n = 5 | | [177Lu]PSMA-I&T 24 h p.i., n = 5 | |
|---|---|---|---|---|---|---|
| | mean | SD | mean | SD | mean | SD |
| blood | 0.0046 | 0.002 | 0.0016 | 0.0008 | 0.0061 | 0.0027 |
| heart | 0.0333 | 0.003 | 0.023 | 0.0059 | 0.0208 | 0.0068 |
| lung | 0.0768 | 0.0179 | 0.0548 | 0.0089 | 0.1138 | 0.0228 |
| liver | 0.2352 | 0.0382 | 0.3202 | 0.0786 | 0.0553 | 0.0263 |
| spleen | 0.5817 | 0.1783 | 0.4246 | 0.1029 | 1.2047 | 1.0492 |
| pancreas | 0.0241 | 0.0042 | 0.0173 | 0.0042 | 0.0295 | 0.0256 |
| stomach | 0.0434 | 0.0177 | 0.0349 | 0.0048 | 0.0326 | 0.0125 |
| intestine | 0.1001 | 0.0653 | 0.0615 | 0.06 | 0.0507 | 0.029 |
| kidney | 11.3738 | 1.4171 | 1.0833 | 0.3075 | 15.8527 | 11.9512 |

(continued)

| Activity accumulation [%ID/g] (complete dataset) | [177Lu]rhPSMA-7.3 24 h p.i., n = 5 | | [177Lu]2C013 24 h p.i., n = 5 | | [177Lu]PSMA-I&T 24 h p.i., n = 5 | |
|---|---|---|---|---|---|---|
| | mean | SD | mean | SD | mean | SD |
| adrenals | 0.4233 | 0.0855 | 0.1327 | 0.0453 | 1.359 | 0.4626 |
| muscle | 0.0094 | 0.0032 | 0.0056 | 0.0021 | 0.0051 | 0.0033 |
| bone | 0.0307 | 0.0067 | 0.1331 | 0.0209 | 0.0209 | 0.0059 |
| tumor | 18.174 | 4.4592 | 10.1512 | 5.2775 | 7.578 | 2.6785 |
| parotid gl. | 0.1068 | 0.0284 | 0.063 | 0.0192 | 0.1346 | 0.0814 |
| submand. gl. | 0.0644 | 0.0134 | 0.038 | 0.0082 | 0.0581 | 0.0277 |

**Table 5:**

| Tumor-to-organ ratio | [177Lu]rhPSMA-7.3 24 h p.i., n = 5 | | [177Lu]2C013 24 h p.i., n = 5 | | [177Lu]PSMA-I&T 24 h p.i., n = 5 | |
|---|---|---|---|---|---|---|
| | mean | SD | mean | SD | mean | SD |
| blood | 4255.56 | 1151.85 | 6950.64 | 3908.59 | 1288.54 | 136.3 |
| heart | 542.49 | 100.85 | 475.05 | 239.41 | 366.06 | 61.92 |
| lung | 239.97 | 46.92 | 184.32 | 85.8 | 65.08 | 10.97 |
| liver | 78.11 | 18.61 | 31.51 | 17.07 | 155.09 | 63.81 |
| spleen | 34.32 | 14.95 | 25.43 | 12.81 | 9.06 | 4.56 |
| pancreas | 754.34 | 110.2 | 587.5 | 299.5 | 336.33 | 150.72 |
| stomach | 453.7 | 147.52 | 286.18 | 142.76 | 236.69 | 41.44 |
| intestine | 254.02 | 173.89 | 263.86 | 213.07 | 191.31 | 116.16 |
| kidney | 1.64 | 0.59 | 9.48 | 4.65 | 0.61 | 0.23 |
| adrenals | 44.34 | 13.03 | 78.58 | 45.14 | 5.62 | 1.33 |
| muscle | 2123.58 | 888.96 | 1867.35 | 1045.82 | 1964.92 | 1251.14 |
| bone | 625.98 | 243.08 | 73.24 | 34.44 | 358.26 | 47.36 |
| parotid gl. | 176.66 | 42.76 | 172.33 | 91.92 | 63.21 | 14.84 |
| submand. gl. | 290.08 | 79.53 | 270.92 | 140.91 | 136.76 | 19.38 |

**Table 6: Summary of the most promising Tx-PSMA Inhibitors**

| **Code** | **In vitro** | | | | | **In vivo biodistribution (24h p.i.)** | | |
|---|---|---|---|---|---|---|---|---|
| | **$logD_{7.4}$** | **$IC_{50}$ [nM]** | **Internalization [% IBA]** | **HSA [%]** | **$MW_{app}$ [kDa]** | **blood [% iA/g]** | **kidney [%iA/g]** | **tumor [% iA/g]** |
| 2C011 | -2.88 ± 0.06 | 6.08 ± 0.16 | 163.2 ± 6.91 | 89.8 | 18.9 | 0.0042 ± 0.0019 | 2.1 ± 0.49 | 11.86 ± 0.75 |
| 2C013 | -3.30 ± 0.06 | 4.44 ± 1.61 | 141 ± 8 | 88.6 | 17.6 | 0.0013 ± 0.0007 | 0.95 ± 0.42 | 7.59 ± 1.23 |
| 2C014 | -3.37 ± 0.10 | 3.95 ± 1.36 | 235 ± 20 | 94.6 | 19.2 | 0.0014 ± 0.0006 | 2.77 ± 0.84 | 16.54 ± 3.37 |
| 2C015 | -3.40 ± 0.10 | 3.99 ± 0.74 | 226 ± 10 | 95.0 | 18.9 | 0.0007 ± 0.0005 | 2.23 ± 1.29 | 8.98 ± 2.32 |

**Table 7: Biodistribution uptake data of [nat/177Lu]Lu 2C011, [nat/177Lu]Lu 2C013, [nat/177Lu]Lu 2C014 and [nat/177Lu]Lu 2C015, expressed as % injected dose per gram of tissue (%iD/g)**

| | [$^{177}$Lu]2C011 | | [$^{177}$Lu]2C013 | | [$^{177}$Lu]2C014 | | [$^{177}$Lu]2C015 | |
|---|---|---|---|---|---|---|---|---|
| | 24 h p.i., n = 4. | | 24 h p.i., n = 4 | | 24 h p.i., n = 3 | | 24 h p.i., n = 4 | |
| uptake in % iD/g | mean | SD | mean | SD | mean | SD | mean | SD |
| blood | 0.0042 | 0.0019 | 0.0013 | 0.0007 | 0.0014 | 0.0006 | 0.0007 | 0.0005 |
| heart | 0.0254 | 0.0061 | 0.019 | 0.0036 | 0.0245 | 0.002 | 0.0237 | 0.0049 |
| lung | 0.0469 | 0.0094 | 0.0438 | 0.0138 | 0.0654 | 0.0221 | 0.0468 | 0.0116 |
| liver | 0.2848 | 0.0567 | 0.2519 | 0.0643 | 0.2376 | 0.0194 | 0.164 | 0.0258 |
| spleen | 0.3218 | 0.0557 | 0.3891 | 0.1184 | 0.4296 | 0.0621 | 0.2671 | 0.0709 |
| pancreas | 0.0264 | 0.007 | 0.0165 | 0.0045 | 0.0192 | 0.0047 | 0.0212 | 0.0034 |
| stomach | 0.0829 | 0.0379 | 0.0846 | 0.0433 | 0.0367 | 0.0099 | 0.036 | 0.0081 |
| intestine | 0.2229 | 0.0932 | 0.3354 | 0.2068 | 0.1006 | 0.018 | 0.1103 | 0.0893 |
| kidney | 2.0983 | 0.4949 | 0.9488 | 0.4204 | 2.7708 | 0.8403 | 2.2339 | 1.2866 |
| adrenals | 0.1488 | 0.0477 | 0.0618 | 0.017 | 0.1473 | 0.0467 | 0.1013 | 0.0279 |
| muscle | 0.0067 | 0.0033 | 0.004 | 0.0011 | 0.0107 | 0.0086 | 0.0062 | 0.0015 |
| bone | 0.0363 | 0.0061 | 0.037 | 0.0042 | 0.0327 | 0.0126 | 0.0223 | 0.0046 |
| tumor | 11.8594 | 0.7477 | 7.5897 | 1.2282 | 16.5415 | 3.3671 | 8.9821 | 2.3231 |
| parotid gl. | 0.0857 | 0.0181 | 0.0554 | 0.0103 | 0.09 | 0.0182 | 0.0674 | 0.0088 |
| submand. gl. | 0.052 | 0.0128 | 0.036 | 0.0038 | 0.0555 | 0.0066 | 0.0389 | 0.0049 |

## BRIEF DESCRIPTION OF THE FIGURES

**Figure 1:** μSPECT/CT scans of [$^{177}$Lu]Lu-2C013 (tumor: 57 mg; 9.8 %iD/g) and [$^{177}$Lu]Lu-rhPSMA-7.3 (tumor: 22 mg; 7.9 %iD/g).

**Figure 2:** Graphical representation of the biodistribution data of Table 2.

Error! Reference source not found.: Results of repeated biodistribution studies with (n = 5 for each compound) and standard deviation SD. Data as graphical representation of Table 4.

**Figures 4a and 4b:** Tumor-to-organ ratios of the compounds [$^{177}$Lu]rhPSMA-7.3 (n = 5), [$^{177}$Lu]2C013 (n = 5) and [$^{177}$Lu]PSMA-I&T (n = 5) at 24 h p.i. in male tumor-bearing CB17-SCID mice. Ratios are calculated individually for each mouse and expressed as mean ± standard deviation. The same data is plotted on two scales on the Y axis.

**Figure 5:** Static μSPECT/CT images (maximum intensity projections) 24 h p.i. of (A) [$^{177}$Lu]rhPSMA-7.3, (C) [$^{177}$Lu]2C013 and (E) [$^{177}$Lu]PSMA-I&T into LNCaP tumor-bearing mice (directly after blood collection) with an acquisition time of 45 min on a VECTor4 small-animal SPECT/PET/OI/CT from MILabs (Utrecht, Netherlands). Tumor weight and tracer uptake in the tumor and the kidneys (in percent of the injected dose/gram, [%ID/g]) were determined from subsequent biodistribution studies. The compound of claim 1 (image C) shows lower kidney uptake than the previously known compounds.

**Figure 6:** Ex vivo biodistribution of [177Lu]2C011 (n = 4) and the reference compounds [177Lu]rhPSMA-7.3 (n = 4), [177Lu]PSMA-617 (n = 4) and [177Lu]PSMA-I&T (n = 4) at 24 h p.i. in male tumor-bearing CB17-SCID mice. Data expressed as percentage of the injected dose per gram (%ID/g), mean ± standard deviation.

**Figure 7:** Ex vivo biodistribution of [$^{177}$Lu]2C015 (n = 4) compared with the biosdistribution of the reference compounds [$^{177}$Lu]rhPSMA-10.1 (n = 5), [$^{177}$Lu]rhPSMA-7.3 (n = 4), [$^{177}$Lu]PSMA-617 (n = 4) and [$^{177}$Lu]PSMA-

I&T (n = 4) at 24 h p.i. in male tumor-bearing CB17-SCID mice. Data expressed as percentage of the injected dose per gram (%ID/g), mean ± standard deviation.

**Figure 8:** Exemplary sigmoidal plot, showing the correlation between human serum albumin (HSA) binding of selected reference substances and retention time ($t_R$). The values of HSA binding were obtained from literature (lit. HSA [%]) (Valko, K.; Nunhuck, S.; Bevan, C.; Abraham, M. H.; Reynolds, D. P. Journal of pharmaceutical sciences 2003, 92, 2236-2248; Yamazaki, K.; Kanaoka, M. Journal of Pharmaceutical sciences 2004, 93, 1480-1494). Log $t_R$: logarithmic value of experimentally determined retention time. Log K HSA: logarithmic value of HSA binding values.

## Claims

**1.** A compound of Formula (1c):

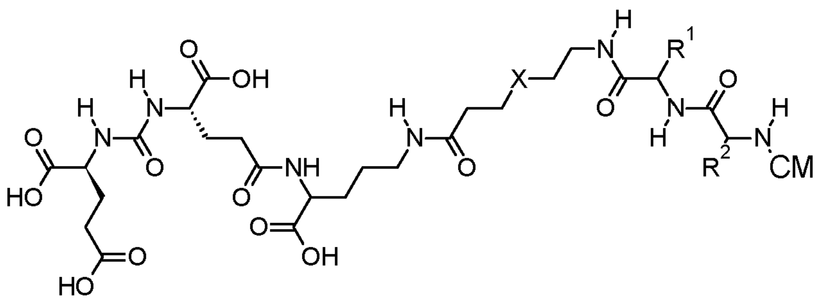

(1c)

or a pharmaceutically acceptable salt or any individual isomer thereof, wherein either
$R^1$ is $-(CH_2)_nR^3$ where n is 1, 2 or 3 and $R^3$ is selected from OH, $NH_2$ or $NHC(O)NH_2$ and $R^2$ is

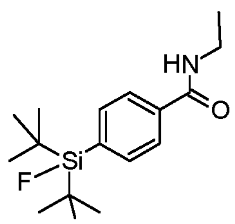

;

or
$R^2$ is $-(CH_2)_nR^3$ where n is 1, 2 or 3 and $R^3$ is selected from OH, $NH_2$ or $NHC(O)NH_2$ and $R^1$ is

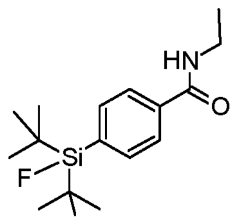

;

X is $CH_2$ or NHCO and
CM represents a chelator moiety, optionally containing a chelated nonradioactive or radioactive cation and the fluorine atom is optionally $^{18}F$.

**2.** The compound according to claim 1 of Formula (1a):

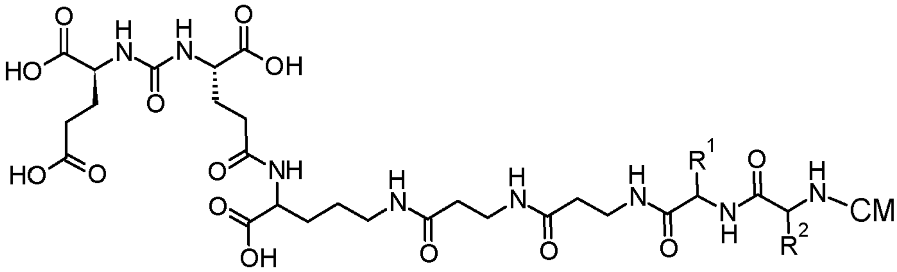

(1a),

or a pharmaceutically acceptable salt or any individual isomer thereof, wherein either $R^1$ is $-(CH_2)_nR^3$ where n is 1, 2 or 3 and $R^3$ is selected from OH, $NH_2$ or $NHC(O)NH_2$ and $R^2$ is

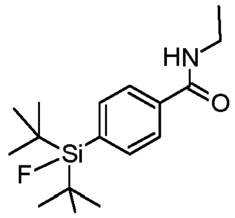

;

or

$R^2$ is $-(CH_2)_nR^3$ where n is 1, 2 or 3 and $R^3$ is selected from OH, $NH_2$ or $NHC(O)NH_2$ and $R^1$ is

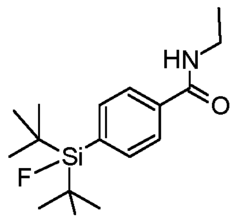

;

and

CM represents a chelator moiety, optionally containing a chelated nonradioactive or radioactive cation and the fluorine atom is optionally $^{18}F$.

**3.** The compound according to claim 1 of Formula (1b):

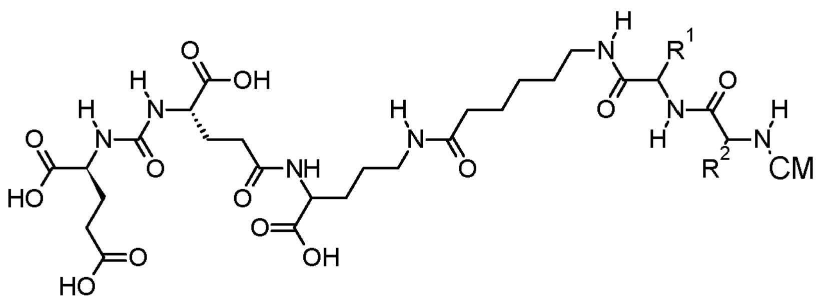

(1b)

or a pharmaceutically acceptable salt or any individual isomer thereof, wherein either $R^1$ is $-(CH_2)_nR^3$ where n is 1, 2 or 3 and $R^3$ is selected from OH, $NH_2$ or $NHC(O)NH_2$ and $R^2$ is

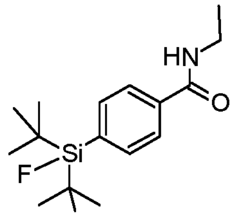

;

or

$R^2$ is $-(CH_2)_nR^3$ where n is 1, 2 or 3 and $R^3$ is selected from OH, $NH_2$ or $NHC(O)NH_2$ and $R^1$ is

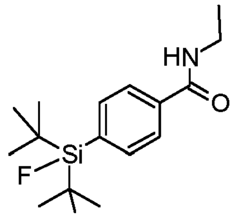

;

and

CM represents a chelator moiety, optionally containing a chelated nonradioactive or radioactive cation and the fluorine atom is optionally $^{18}F$.

**4.** The compound according to claim 1 of Formula (1):

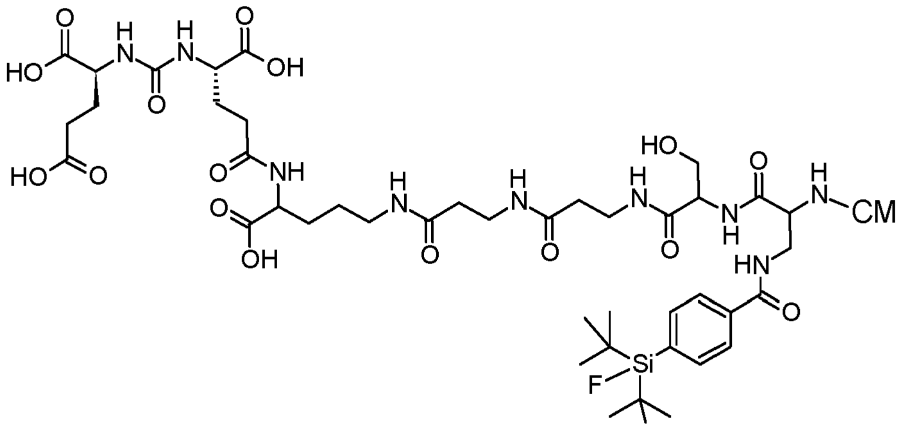

(1);

or a pharmaceutically acceptable salt or any individual isomer thereof, wherein

CM represents a chelator moiety, optionally containing a chelated nonradioactive or radioactive cation and the fluorine atom is optionally $^{18}F$.

**5.** The compound according to claim 4 which is a compound of Formula (2):

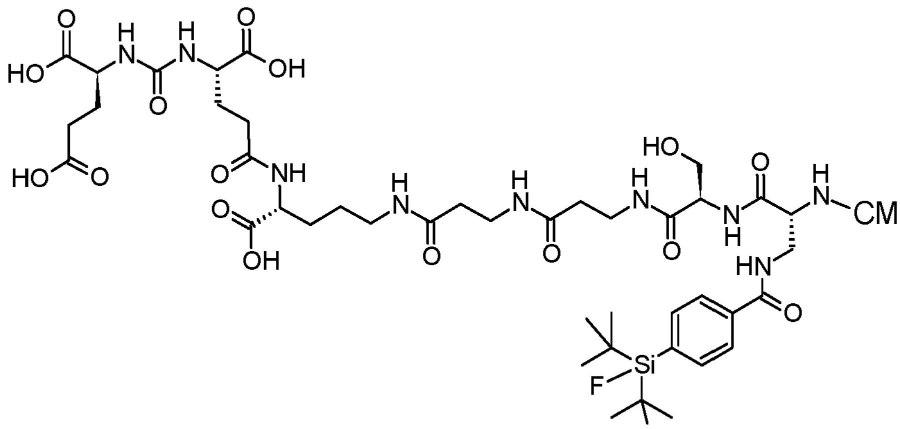

(2);

or a pharmaceutically acceptable salt or any individual isomer thereof, wherein
CM represents a chelator moiety, optionally containing a chelated nonradioactive or radioactive cation and the fluorine atom is optionally $^{18}F$.

6. The compound according to claim 5 which is a compound of Formula (3):

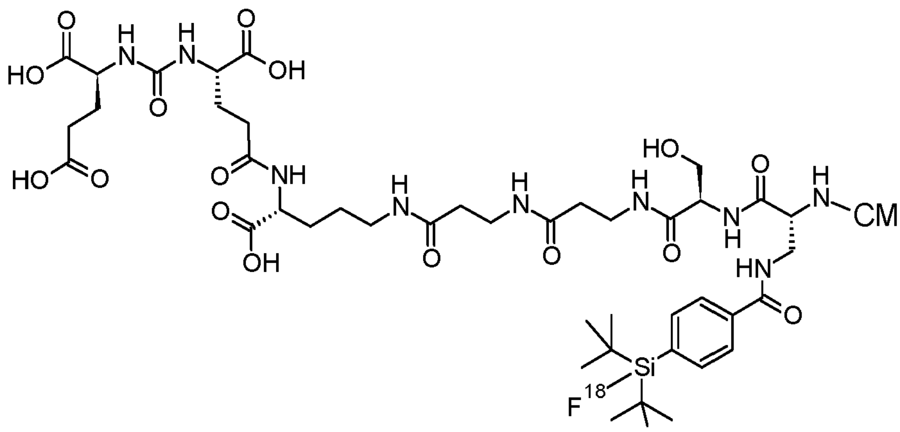

(3);

or a pharmaceutically acceptable salt or any individual isomer thereof, wherein
CM represents a chelator moiety, optionally containing a chelated nonradioactive or radioactive cation.

7. The compound according to claim 2 which is a compound of Formula (1'):

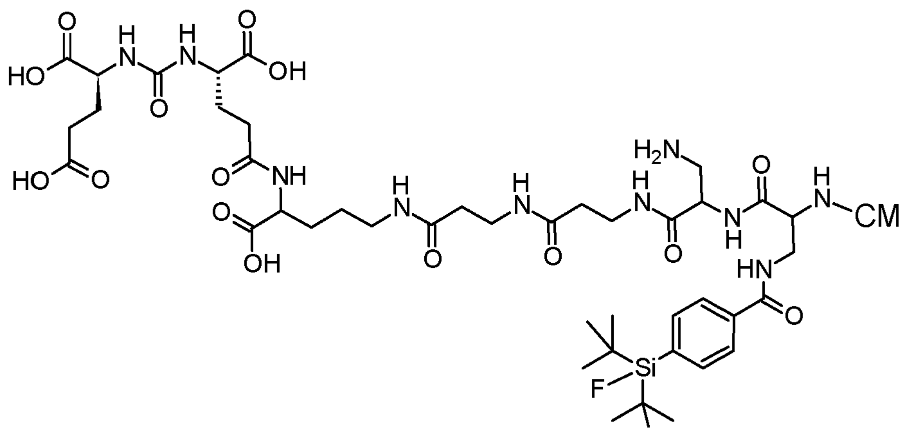

(1');

or a pharmaceutically acceptable salt or any individual isomer thereof, wherein

CM represents a chelator moiety, optionally containing a chelated nonradioactive or radioactive cation and the fluorine atom is optionally $^{18}F$.

**8.** The compound according to claim 7 which is a compound of Formula (2a):

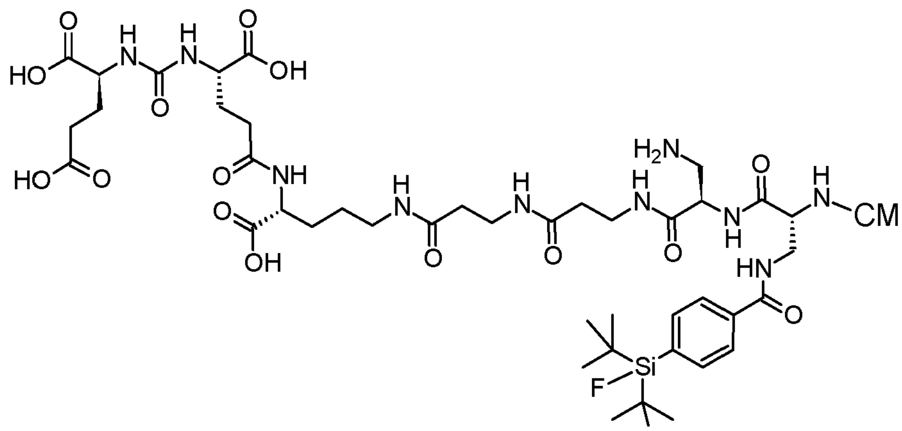

(2a);

or a pharmaceutically acceptable salt or any individual isomer thereof, wherein
CM represents a chelator moiety, optionally containing a chelated nonradioactive or radioactive cation and the fluorine atom is optionally $^{18}F$.

**9.** The compound according to claim 8 which is a compound of Formula (3a):

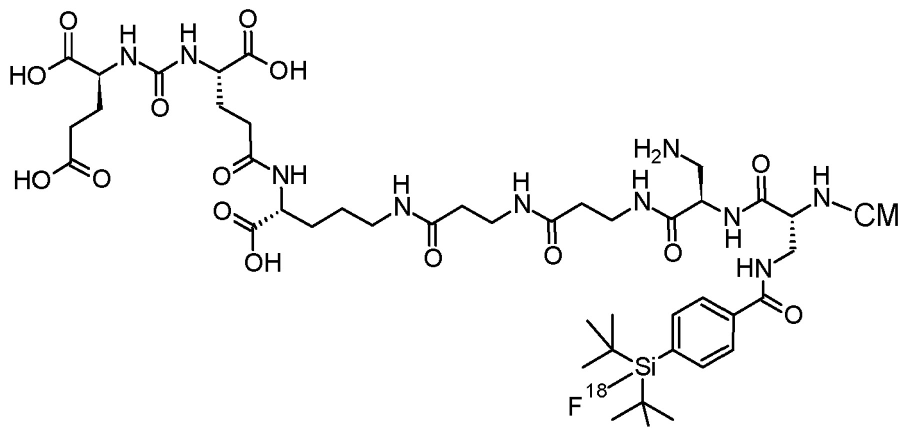

(3a);

or a pharmaceutically acceptable salt or any individual isomer thereof, wherein
CM represents a chelator moiety, optionally containing a chelated nonradioactive or radioactive cation.

**10.** The compound according to claim 3 which is a compound of Formula (1"):

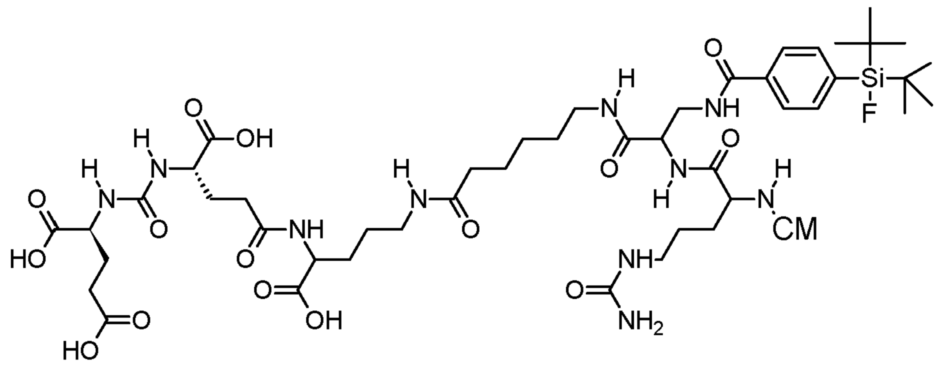

(1");

**11.** The compound according to claim 10 which is a compound of Formula (2b):

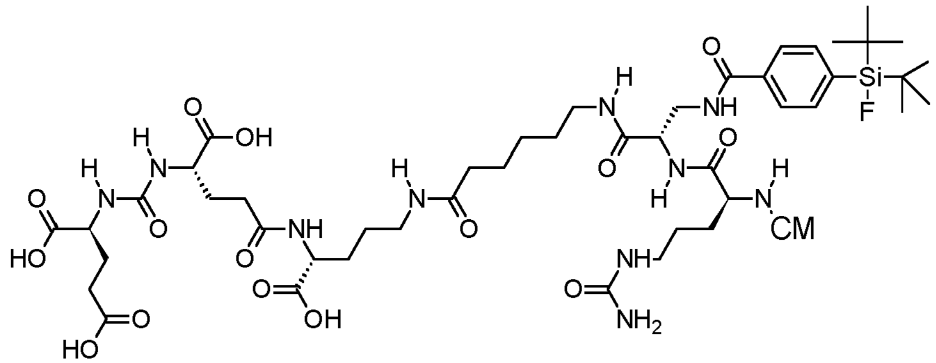

(2b);

or a pharmaceutically acceptable salt or any individual isomer thereof, wherein
CM represents a chelator moiety, optionally containing a chelated nonradioactive or radioactive cation and the fluorine atom is optionally $^{18}F$.

**12.** The compound according to claim 11 which is a compound of Formula (3b):

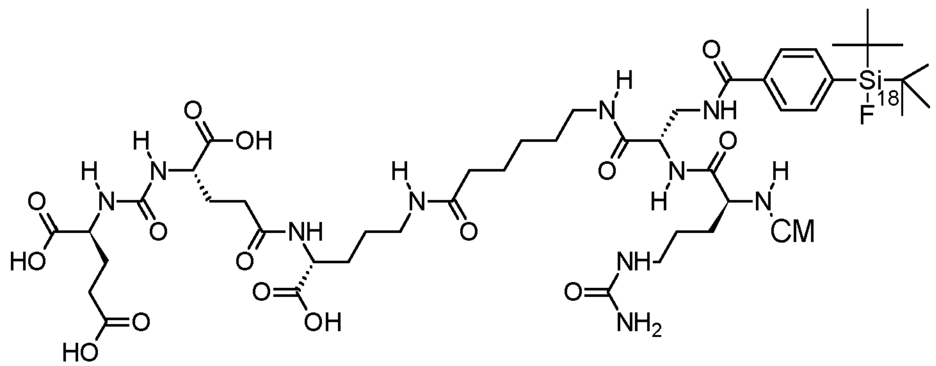

(3b);

or a pharmaceutically acceptable salt or any individual isomer thereof, wherein
CM represents a chelator moiety, optionally containing a chelated nonradioactive or radioactive cation.

**13.** The compound according to any one of claims 1 to 12, wherein the chelator moiety comprises at least one of:

(i) a macrocyclic ring structure with 8 to 20 ring atoms of which 2 or more are heteroatoms selected from oxygen atoms and nitrogen atoms;
(ii) an acyclic, open chain chelating structure with 8 to 20 main chain atoms of which 2 or more are heteroatoms selected from oxygen atoms and nitrogen atoms; or
(iii) a branched chelating structure containing a quaternary carbon atom.

**14.** The compound according to any one of claims 1 to 12, wherein the chelator moiety is selected from bis(carboxymethyl)-1,4,8,11-tetraazabicyclo[6.6.2]hexadecane (CBTE2a), cyclohexyl-1,2-diaminetetraacetic acid (CDTA), 4-(1,4,8,11-tetraazacyclotetradec-1-yl)-methylbenzoic acid (CPTA), N'-[5-[acetyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxymethyl)-1,4,8,11-tetraazabicyclo[6.6.2]hexadecan (DO2A) 1,4,7,10-tetracyclododecan-N,N',N",N'"-tetraacetic acid (DOTA), α-(2-carboxyethyl)-1,4,7,10-tetraazacyclododecane-1,4,7,10-tetraacetic acid (DOTAGA), 1,4,7,10 tetraazacyclododecane N, N', N", N'" 1,4,7,10-tetra(methylene) phosphonic acid (DOTMP), N,N'-dipyridoxylethylendiamine-N,N'-diacetate-5,5'-bis(phosphat) (DPDP), diethylene triamine N,N',N" penta(methylene) phosphonic acid (DTMP), diethylenetriaminepentaacetic acid (DTPA), ethylenediamine-N,N'-tetraacetic acid (EDTA), ethyleneglykol-O,O-bis(2-aminoethyl)-N,N,N',N'-tetraacetic acid (EGTA), N,N-bis(hydroxybenzyl)-ethylenediamine-N,N'-diacetic acid (HBED), hydroxyethyldiaminetriacetic acid (HEDTA), 1-(p-nitrobenzyl)-1,4,7,10-tetraazacyclodecan-4,7,10-triacetate (HP-DOA3), 6-hydrazinyl-N-methylpyridine-3-carboxamide (HYNIC), tetra 3-hydroxy-N-methyl-2-pyridinone chelators (4-((4-(3-(bis(2-(3-hydroxy-1-methyl-2-oxo-1,2-dihydropyridine-4-carboxamido)ethyl)amino)-2-((bis(2-(3-hydroxy-1-methyl-2-oxo-1,2-dihydropyridine-4-carboxamido)ethyl)amino)methyl)propyl)phenyl)amino)-4-oxobutanoic acid), abbreviated as Me-3,2-HOPO, 1,4,7-triazacyclononan-1-succinic acid-4,7-diacetic acid (NODASA), 1-(1-carboxy-3-carboxypropyl)-4,7-(carbooxy)-1,4,7-triazacyclononane (NODAGA), 1,4,7-triazacyclononanetriacetic acid (NOTA), 4,11-bis(carboxymethyl)-1,4,8,11-tetraazabicyclo[6.6.2]hexadecane (TE2A), 1,4,8,11-tetraazacyclododecane-1,4,8,11-tetraacetic acid (TETA), tris(hydroxypyridinone) (THP), terpyridin-bis(methyleneamintetraacetic acid (TMT), 1,4,7-triazacyclononane-1,4,7-trisimethylene(2-carboxyethyl)phosphinic acid] (TRAP), 1,4,7,10-tetraazacyclotridecan-N,N',N",N'"-tetraacetic acid (TRITA), 3-[[4,7-bis[[2-carboxyethyl(hydroxy)phosphoryl]methyl]-1,4,7-triazonan-1-yl]methyl-hydroxy-phosphoryl]propanoic acid, and triethylenetetraaminehexaacetic acid (TTHA).

**15.** The compound according to claim 14, wherein the chelator moiety is 1,4,7,10-tetracyclododecan-N,N',N",N'"-tetraacetic acid (DOTA) or α-(2-carboxyethyl)-1,4,7,10-tetraazacyclododecane-1,4,7,10-tetraacetic acid (DOTAGA).

**16.** The compound according to claim 1, which is selected from:

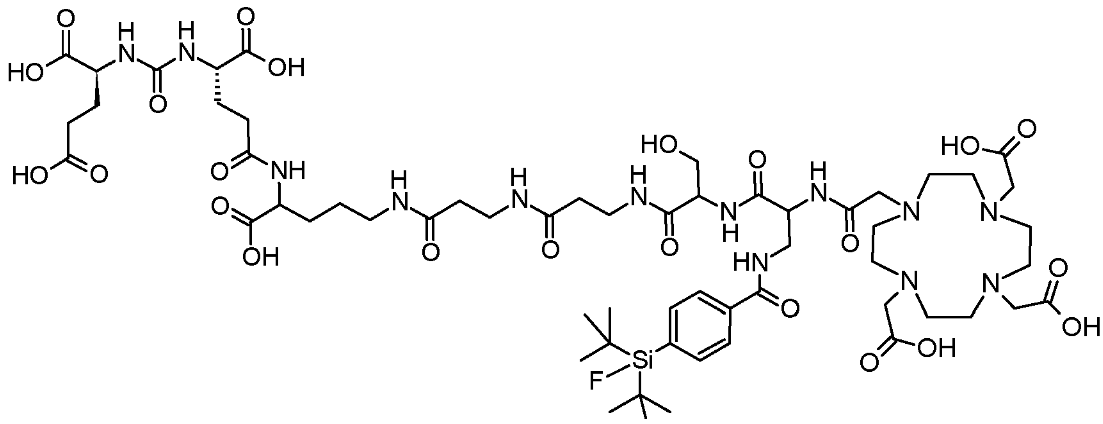

;

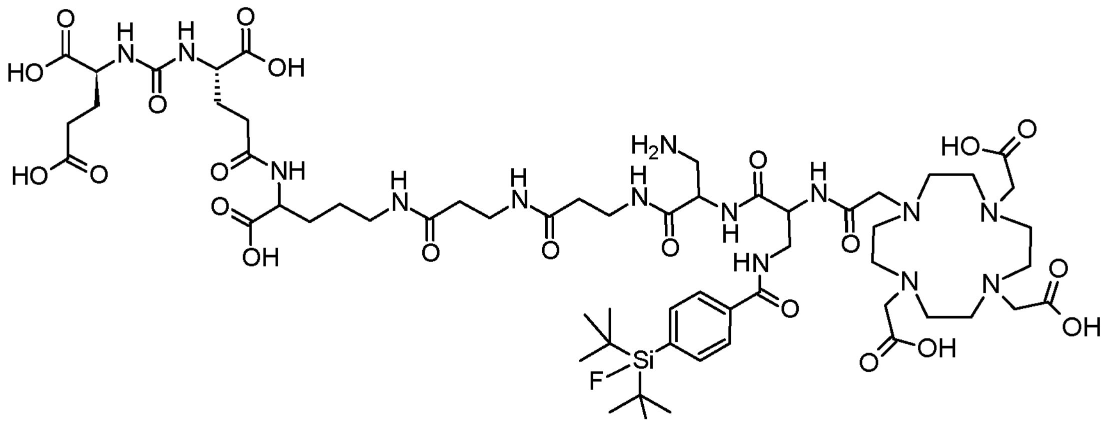

;

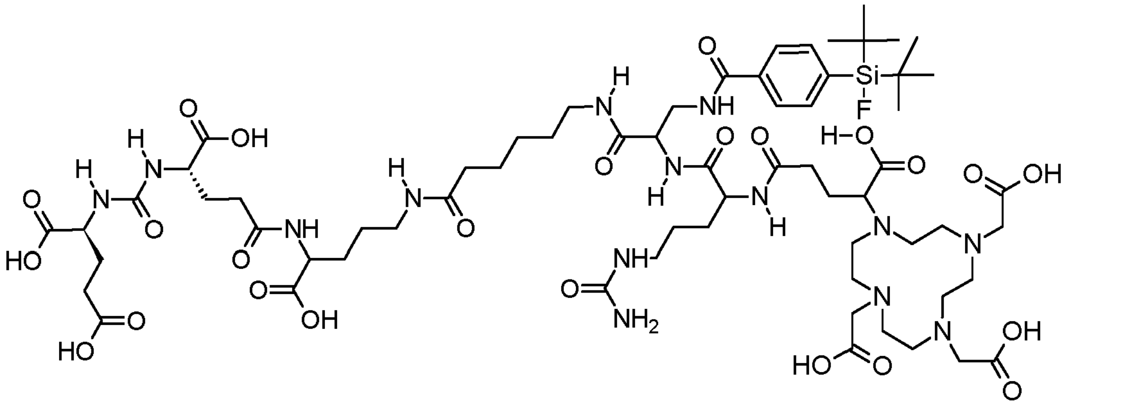

or a pharmaceutically acceptable salt or any individual isomer thereof, wherein the compound optionally contains a chelated nonradioactive or radioactive cation and wherein the fluorine atom is optionally $^{18}F$.

**17.** The compound according to claim 1 which is selected from:

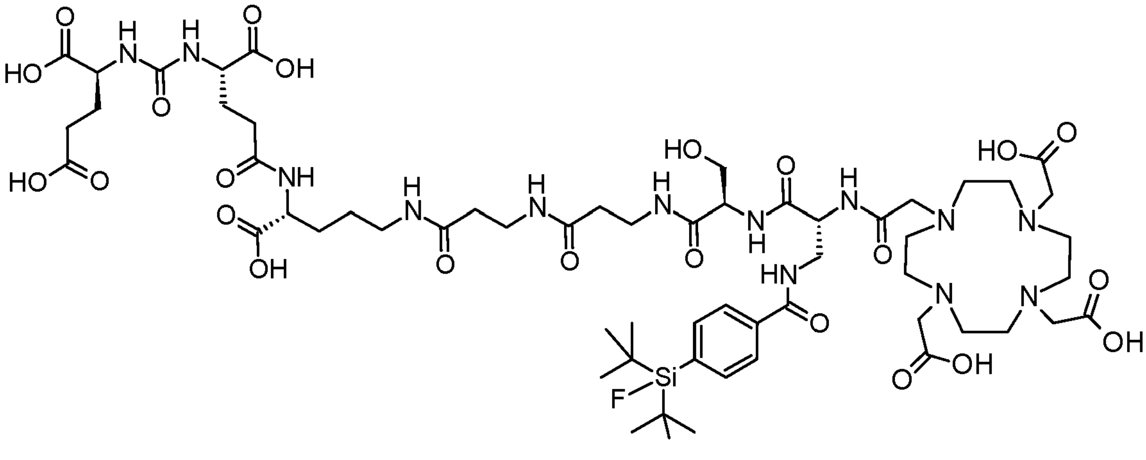

;

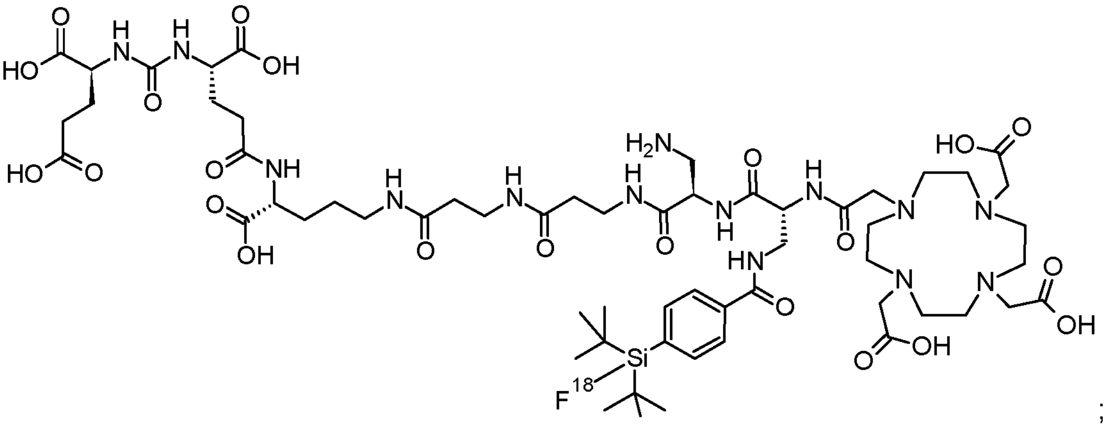

;

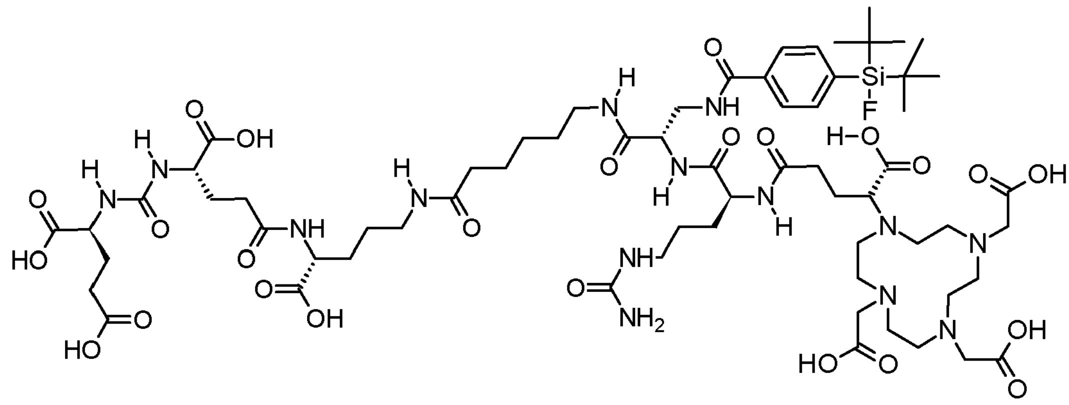

;

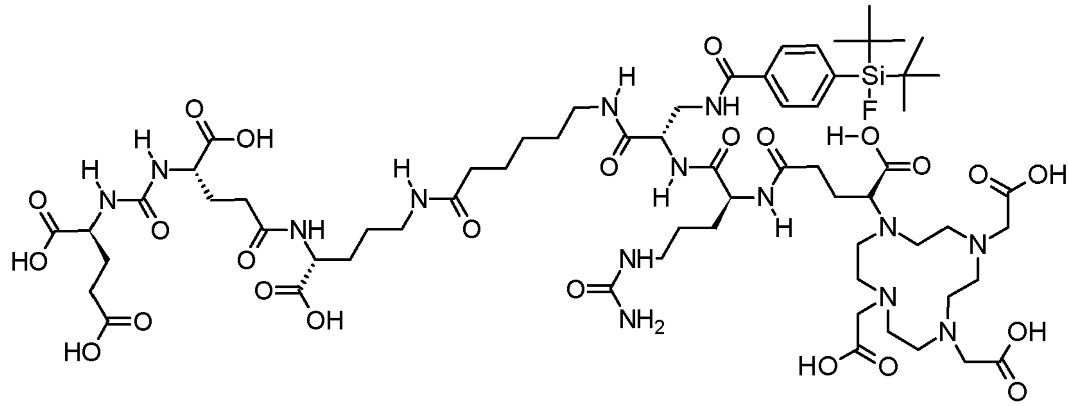

.

or a pharmaceutically acceptable salt thereof, wherein the compound optionally contains a chelated nonradioactive or radioactive cation and wherein the fluorine atom is optionally $^{18}F$.

**18.** The compound according to any one of claims 1 to 17, wherein the chelator moiety contains a chelated cation selected from the cations of $^{43}Sc$, $^{44}Sc$, $^{47}Sc$, $^{64}Cu$, $^{67}Cu$, $^{67}Ga$, $^{68}Ga$, $^{90}Y$, $^{111}In$, $^{149}Tb$, $^{152}Tb$, $^{155}Tb$, $^{161}Tb$, $^{166}Ho$, $^{177}Lu$, $^{186}Re$, $^{188}Re$, $^{212}Pb$, $^{212}Bi$, $^{213}Bi$, $^{225}Ac$, and $^{227}Th$ or a cationic molecule comprising $^{18}F$.

**19.** The compound according to claim 1 which is:

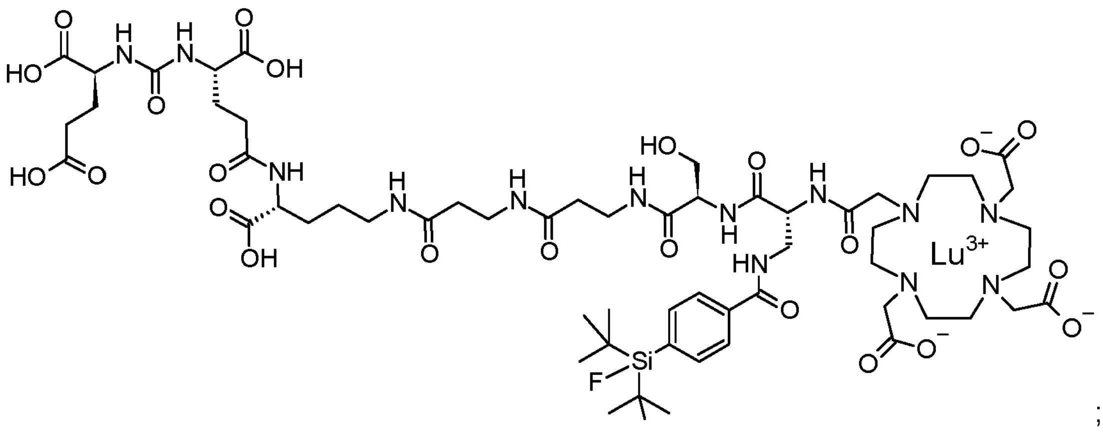

;

or a pharmaceutically acceptable salt thereof, wherein the fluorine atom is optionally $^{18}F$.

**20.** A pharmaceutically acceptable salt of a compound according to any one of claims 1 to 19.

**21.** A pharmaceutical or diagnostic composition comprising or consisting of one or more compounds according to any one

of claims 1 to 20.

**22.** A compound as claimed in any one of claims 1 to 20 or a composition as claimed in claim 21 for use as a cancer therapeutic, cancer diagnostic or imaging agent.

**23.** A compound as claimed in any one of claims 1 to 20 or a composition as claimed in claim 21 for use as a cancer diagnostic or imaging agent or for use in the treatment of cancer wherein the cancer is prostate, breast, lung, colorectal or renal cell carcinoma.

**Patentansprüche**

**1.** Verbindung der Formel (1c):

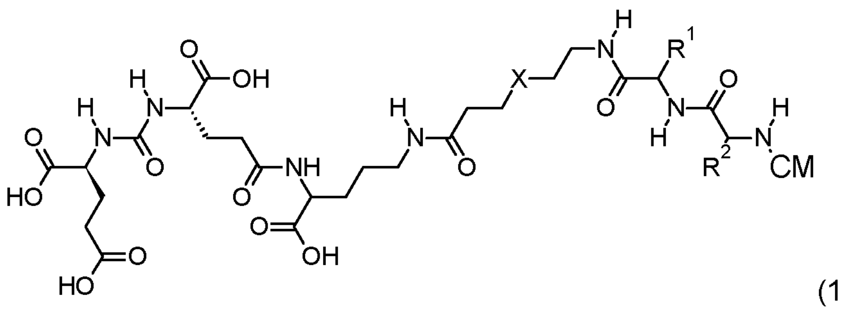

(1c)

oder ein pharmazeutisch unbedenkliches Salz oder ein beliebiges individuelles Isomer davon, wobei entweder $R^1$ für $-(CH_2)_nR^3$ steht, wobei n für 1, 2 oder 3 steht, und $R^3$ aus OH, $NH_2$ oder $NHC(O)NH_2$ ausgewählt ist und $R^2$ für

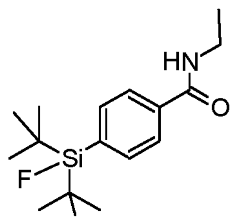

;

steht oder
$R^2$ für $-(CH_2)_nR^3$ steht, wobei n für 1, 2 oder 3 steht, und $R^3$ aus OH, $NH_2$ oder $NHC(O)NH_2$ ausgewählt ist und $R^1$ für

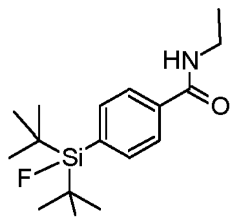

;

X für $CH_2$ oder NHCO steht und
CM für eine Chelatbildnergruppierung, die gegebenenfalls ein chelatisiertes nichtradioaktives oder radioaktives Kation enthält, steht und das Fluoratom gegebenenfalls $^{18}F$ ist.

**2.** Verbindung nach Anspruch 1 der Formel (1a):

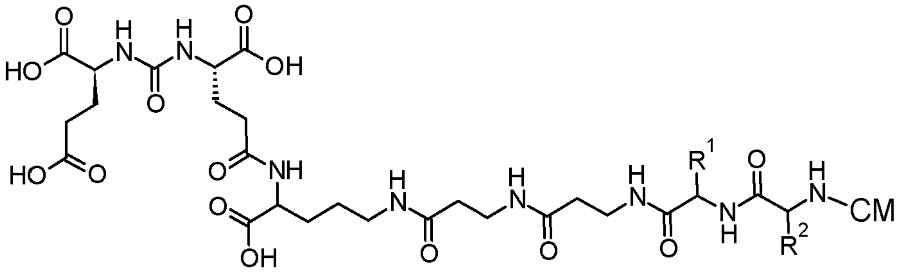

(1a),

oder ein pharmazeutisch unbedenkliches Salz oder ein beliebiges individuelles Isomer davon, wobei entweder $R^1$ für $-(CH_2)_nR^3$ steht, wobei n für 1, 2 oder 3 steht, und $R^3$ aus OH, $NH_2$ oder $NHC(O)NH_2$ ausgewählt ist und $R^2$ für

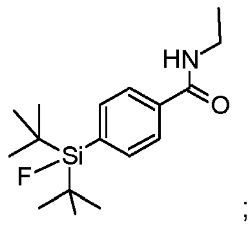

;

steht oder

$R^2$ für $-(CH_2)_nR^3$ steht, wobei n für 1, 2 oder 3 steht, und $R^3$ aus OH, $NH_2$ oder $NHC(O)NH_2$ ausgewählt ist und $R^1$ für

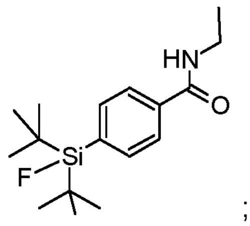

;

steht und

CM für eine Chelatbildnergruppierung, die gegebenenfalls ein chelatisiertes nichtradioaktives oder radioaktives Kation enthält, steht und das Fluoratom gegebenenfalls $^{18}F$ ist.

**3.** Verbindung nach Anspruch 1 der Formel (1b):

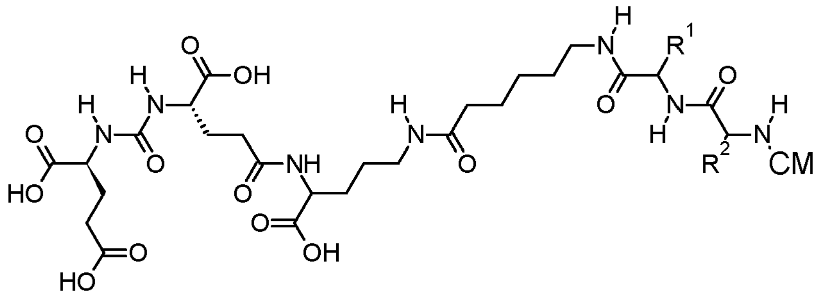

(1b)

oder ein pharmazeutisch unbedenkliches Salz oder ein beliebiges individuelles Isomer davon, wobei entweder $R^1$ für $-(CH_2)_nR^3$ steht, wobei n für 1, 2 oder 3 steht, und $R^3$ aus OH, $NH_2$ oder $NHC(O)NH_2$ ausgewählt ist und $R^2$ für

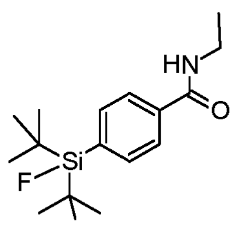

;

steht oder

$R^2$ für $-(CH_2)_nR^3$ steht, wobei n für 1, 2 oder 3 steht, und $R^3$ aus OH, $NH_2$ oder $NHC(O)NH_2$ ausgewählt ist und $R^1$ für

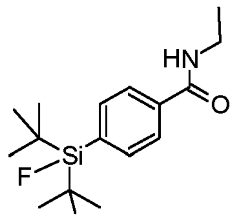

;

steht und

CM für eine Chelatbildnergruppierung, die gegebenenfalls ein chelatisiertes nichtradioaktives oder radioaktives Kation enthält, steht und das Fluoratom gegebenenfalls $^{18}F$ ist.

**4.** Verbindung nach Anspruch 1 der Formel (1):

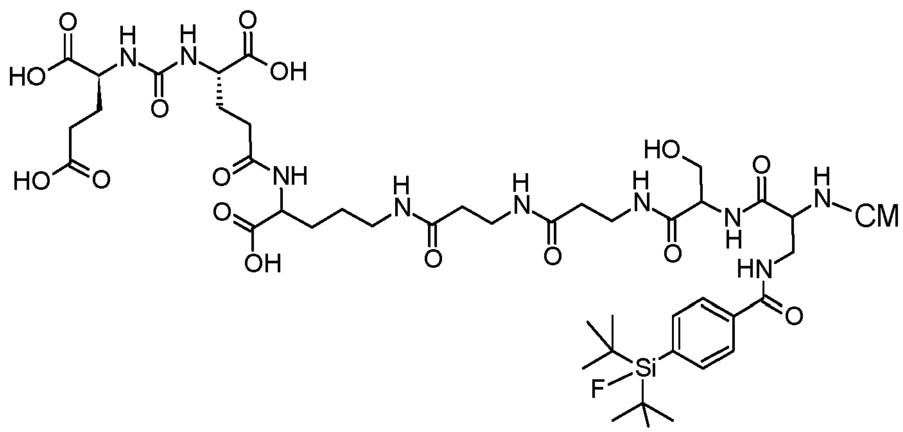

(1);

oder ein pharmazeutisch unbedenkliches Salz oder ein beliebiges individuelles Isomer davon, wobei CM für eine Chelatbildnergruppierung, die gegebenenfalls ein chelatisiertes nichtradioaktives oder radioaktives Kation enthält, steht und das Fluoratom gegebenenfalls $^{18}F$ ist.

**5.** Verbindung nach Anspruch 4, bei der es sich um eine Verbindung der Formel (2) handelt:

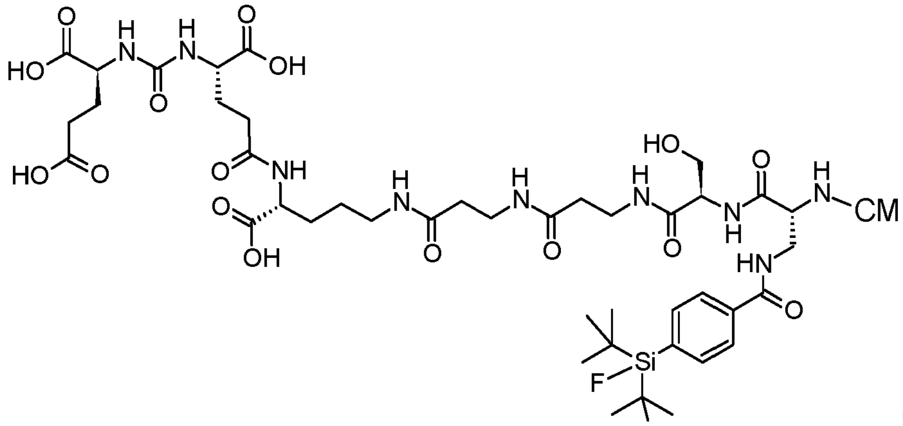

(2);

oder ein pharmazeutisch unbedenkliches Salz oder ein beliebiges individuelles Isomer davon, wobei CM für eine Chelatbildnergruppierung, die gegebenenfalls ein chelatisiertes nichtradioaktives oder radioaktives Kation enthält, steht und das Fluoratom gegebenenfalls $^{18}F$ ist.

**6.** Verbindung nach Anspruch 5, bei der es sich um eine Verbindung der Formel (3) handelt:

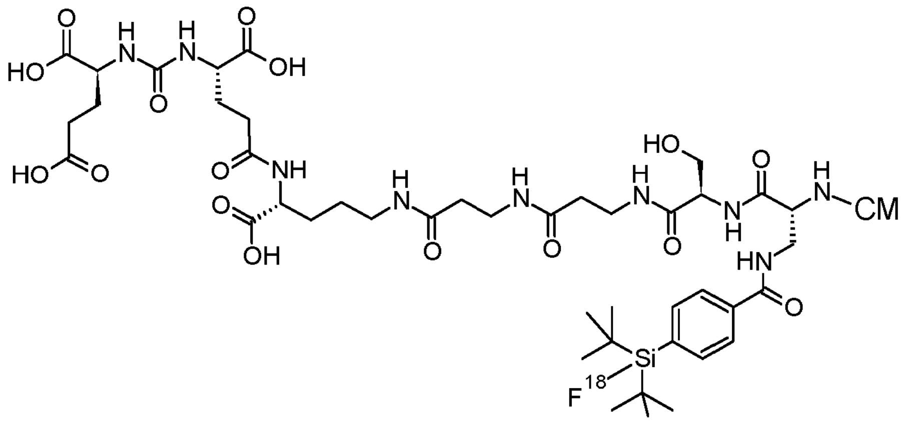

(3);

oder ein pharmazeutisch unbedenkliches Salz oder ein beliebiges individuelles Isomer davon, wobei CM für eine Chelatbildnergruppierung, die gegebenenfalls ein chelatisiertes nichtradioaktives oder radioaktives Kation enthält, steht.

**7.** Verbindung nach Anspruch 2, bei der es sich um eine Verbindung der Formel (1') handelt:

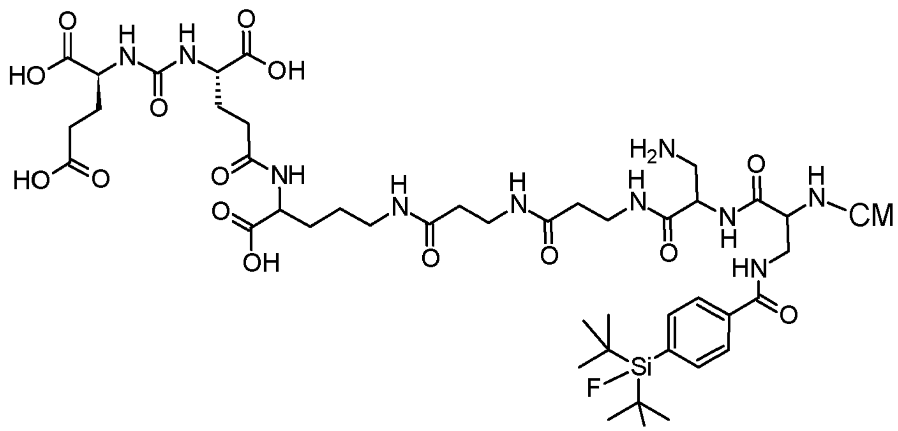

(1');

oder ein pharmazeutisch unbedenkliches Salz oder ein beliebiges individuelles Isomer davon, wobei

CM für eine Chelatbildnergruppierung, die gegebenenfalls ein chelatisiertes nichtradioaktives oder radioaktives Kation enthält, steht und das Fluoratom gegebenenfalls $^{18}F$ ist.

**8.** Verbindung nach Anspruch 7, bei der es sich um eine Verbindung der Formel (2a) handelt:

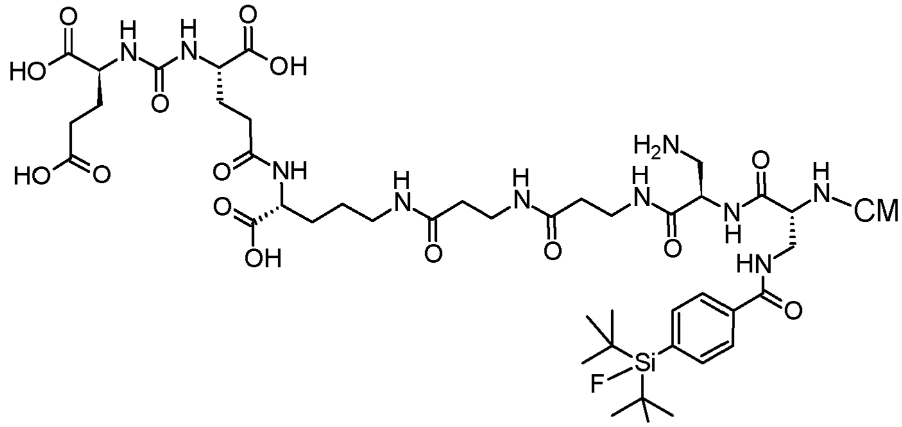

(2a);

oder ein pharmazeutisch unbedenkliches Salz oder ein beliebiges individuelles Isomer davon, wobei CM für eine Chelatbildnergruppierung, die gegebenenfalls ein chelatisiertes nichtradioaktives oder radioaktives Kation enthält, steht und das Fluoratom gegebenenfalls $^{18}F$ ist.

**9.** Verbindung nach Anspruch 8, bei der es sich um eine Verbindung der Formel (3a) handelt:

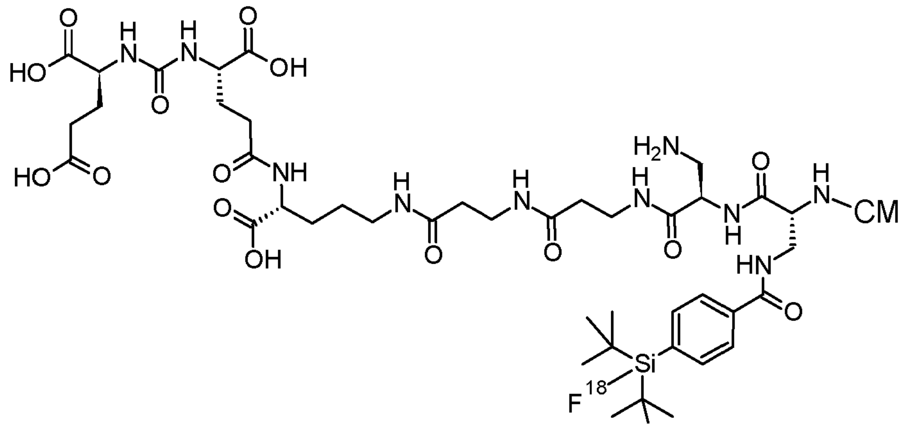

(3a);

oder ein pharmazeutisch unbedenkliches Salz oder ein beliebiges individuelles Isomer davon, wobei CM für eine Chelatbildnergruppierung, die gegebenenfalls ein chelatisiertes nichtradioaktives oder radioaktives Kation enthält, steht.

**10.** Verbindung nach Anspruch 3, bei der es sich um eine Verbindung der Formel (1") handelt:

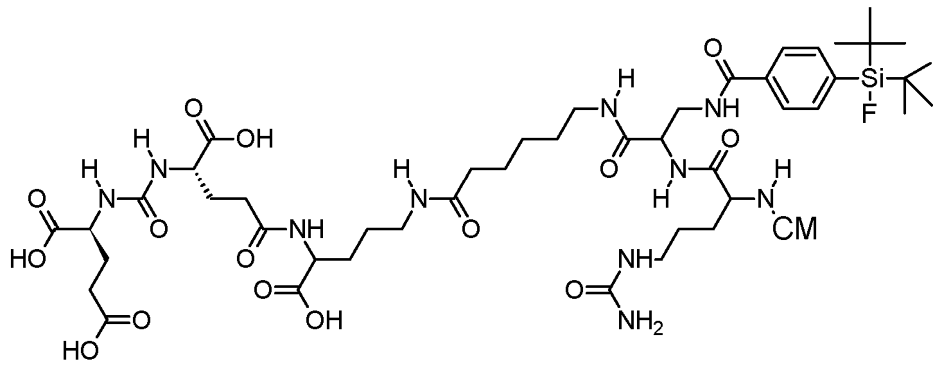

(1");

11. Verbindung nach Anspruch 10, bei der es sich um eine Verbindung der Formel (2b) handelt:

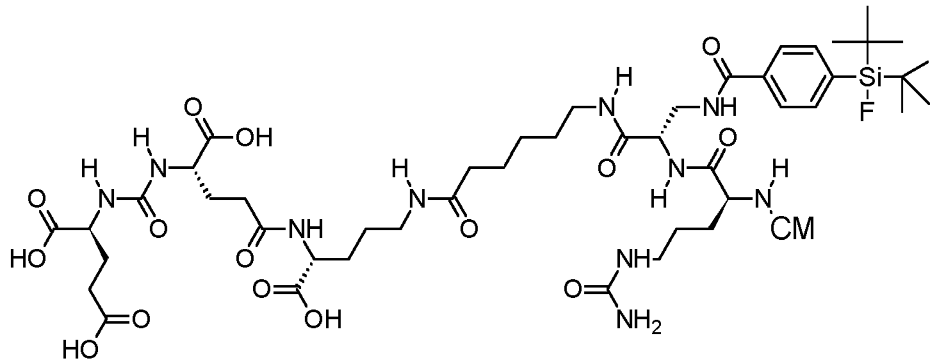

(2b);

oder ein pharmazeutisch unbedenkliches Salz oder ein beliebiges individuelles Isomer davon, wobei CM für eine Chelatbildnergruppierung, die gegebenenfalls ein chelatisiertes nichtradioaktives oder radioaktives Kation enthält, steht und das Fluoratom gegebenenfalls $^{18}F$ ist.

12. Verbindung nach Anspruch 11, bei der es sich um eine Verbindung der Formel (3b) handelt:

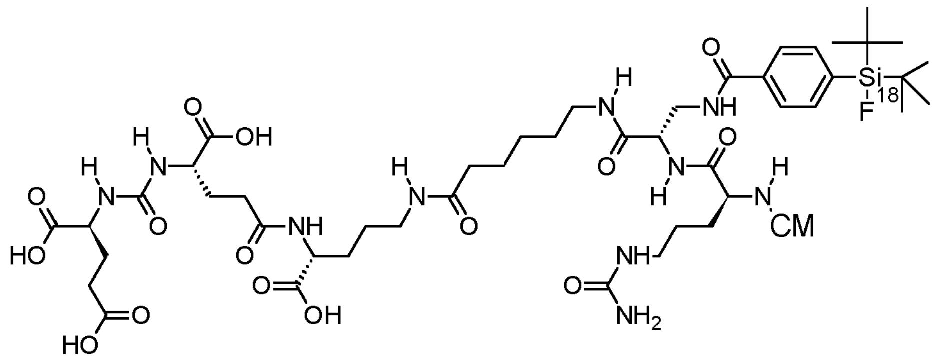

(3b);

oder ein pharmazeutisch unbedenkliches Salz oder ein beliebiges individuelles Isomer davon, wobei CM für eine Chelatbildnergruppierung, die gegebenenfalls ein chelatisiertes nichtradioaktives oder radioaktives Kation enthält, steht.

13. Verbindung nach einem der Ansprüche 1 bis 12, wobei die Chelatbildnergruppierung mindestens eines von Folgenden umfasst:

(i) eine makrocyclische Ringstruktur mit 8 bis 20 Ringatomen, von denen 2 oder mehr aus Sauerstoffatomen und Stickstoffatomen ausgewählte Heteroatome sind;
(ii) eine acyclische, offenkettige Chelatisierungsstruktur mit 8 bis 20 Hauptkettenatomen, von denen 2 oder mehr

aus Sauerstoffatomen und Stickstoffatomen ausgewählte Heteroatome sind; oder
(iii) eine verzweigte Chelatisierungsstruktur, die ein quartäres Kohlenstoffatom enthält.

**14.** Verbindung nach einem der Ansprüche 1 bis 12, wobei die Chelatbildnergruppierung aus Bis(carboxymethyl)-1,4,8,11-tetraazabicyclo[6.6.2]hexadecan (CBTE2a), Cyclohexyl-1,2-diamintetraessigsäure (CDTA), 4-(1,4,8,11-Tetraazacyclotetradec-1-yl)-methylbenzoesäure (CPTA), N'-[5-[Acetyl(hydroxy)amino]pentyl]-N-[5-[[4-[5-aminopentyl(hydroxy)amino]-4-oxobutanoyl]amino]pentyl]-N-hydroxybutandiamid (DFO), 4,11-Bis(carboxymethyl)-1,4,8,11-tetraazabicyclo[6.6.2]hexadecan (DO2A), 1,4,7,10-Tetracyclododecan-N,N',N",N"'-tetraessigsäure (DOTA), α-(2-Carboxyethyl)-1,4,7,10-tetraazacyclododecan-1,4,7,10-tetraessigsäure (DOTAGA), 1,4,7,10-Tetraazacyclododecan-N,N',N",N"'-1,4,7,10-tetra(methylen)phosphonsäure (DOTMP), N,N'-Dipyridoxylethylendiamin-N,N'-diacetat-5,5'-bis(phosphat) (DPDP), Diethylentriamin-N,N',N"-penta(methylen)phosphonsäure (DTMP), Diethylentriaminpentaessigsäure (DTPA), Ethylendiamin-N,N'-tetraessigsäure (EDTA), Ethylenglykol-O,O-bis(2-aminoethyl)-N,N,N',N'-tetraessigsäure (EGTA), N,N-bis(hydroxybenzyl)ethylendiamin-N,N'-diessigsäure (HBED), Hydroxyethyldiamintriessigsäure (HEDTA), 1-(p-Nitrobenzyl)-1,4,7,10-tetraazacyclodecan-4,7,10-triacetat (HP-DOA3), 6-Hydrazinyl-N-methylpyridin-3-carboxamid (HYNIC), Tetra-3-hydroxy-N-methyl-2-pyridinon-Chelatbildnern, (4-((4-(3-(Bis(2-(3-hydroxy-1-methyl-2-oxo-1,2-dihydropyridin-4-carboxamido)ethyl)amino)-2-((bis(2-(3-hydroxy-1-methyl-2-oxo-1,2-dihydropyridin-4-carboxamido)ethyl)amino)methyl)propyl)phenyl) amino)-4-oxobutansäure), abgekürzt als Me-3,2-HOPO, 1,4,7-Triazacyclononan-1-bernsteinsäure-4,7-diessigsäure (NODASA), 1-(1-Carboxy-3-carboxypropyl)-4,7-(carbooxy)-1,4,7-triazacyclononan (NODAGA), 1,4,7-Triazacyclononantriessigsäure (NOTA), 4,11-Bis(carboxymethyl)-1,4,8,11-tetraazabicyclo[6.6.2]hexadecan (TE2A), 1,4,8,11-Tetraazacyclododecan-1,4,8,11-tetraessigsäure (TETA), Tris(hydroxypyridinon) (THP), Terpyridin-bis(methylenamin)tetraessigsäure (TMT), 1,4,7-Triazacyclononan-1,4,7-tris[methylen(2-carboxyethyl)phosphinsäure] (TRAP), 1,4,7,10-Tetraazacyclotridecan-N,N',N",N"'-tetraessigsäure (TRITA), 3-[[4,7-Bis[[2-carboxyethyl(hydroxy)phosphoryl]methyl]-1,4,7-triazonan-1-yl]methylhydroxyphosphoryl]propansäure und Triethylentetraaminhexaessigsäure (TTHA) ausgewählt ist.

**15.** Verbindung nach Anspruch 14, wobei es sich bei der Chelatbildnergruppierung um 1,4,7,10-Tetracyclododecan-N,N',N",N"'-tetraessigsäure (DOTA) oder α-(2-Carboxyethyl)-1,4,7,10-tetraazacyclododecan-1,4,7,10-tetraessigsäure (DOTAGA) handelt.

**16.** Verbindung nach Anspruch 1, die ausgewählt ist aus:

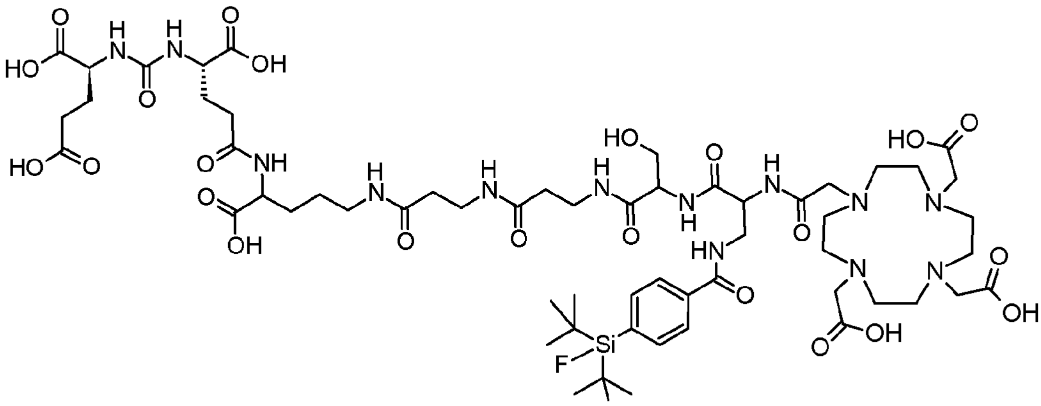

;

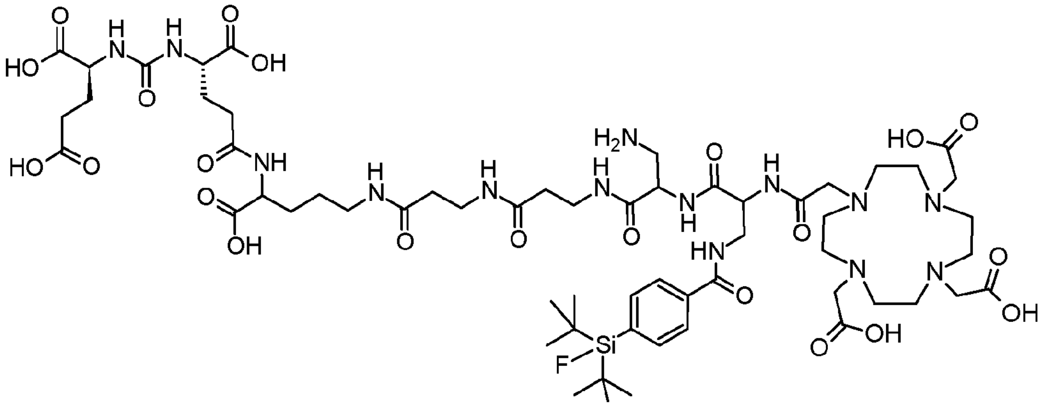

;

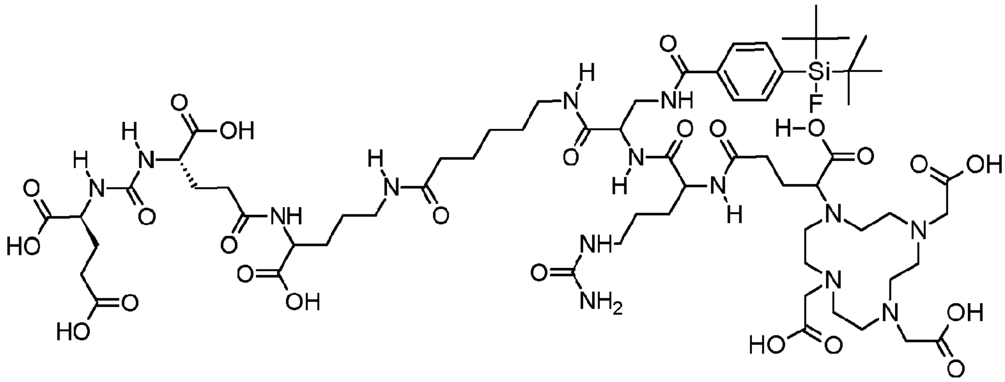

oder ein pharmazeutisch unbedenkliches Salz oder ein beliebiges individuelles Isomer davon, wobei die Verbindung gegebenenfalls ein chelatisiertes nichtradioaktives oder radioaktives Kation enthält und das Fluoratom gegebenenfalls $^{18}F$ ist.

**17.** Verbindung nach Anspruch 1, die ausgewählt ist aus:

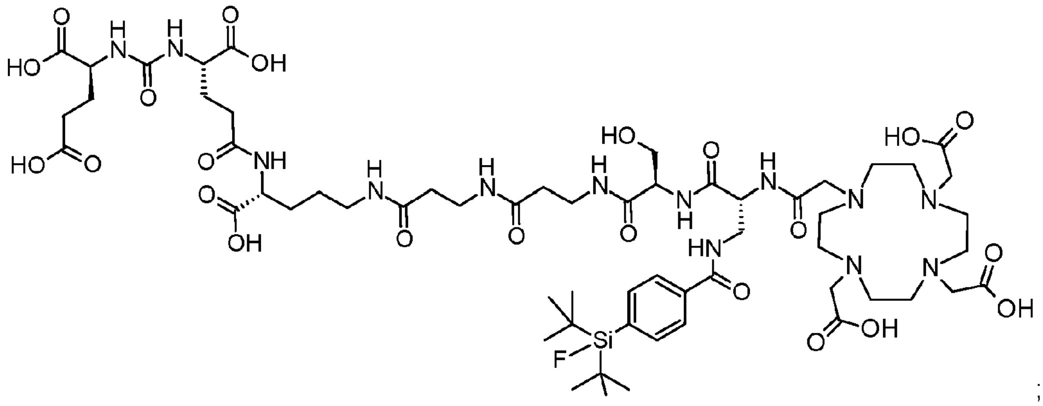

;

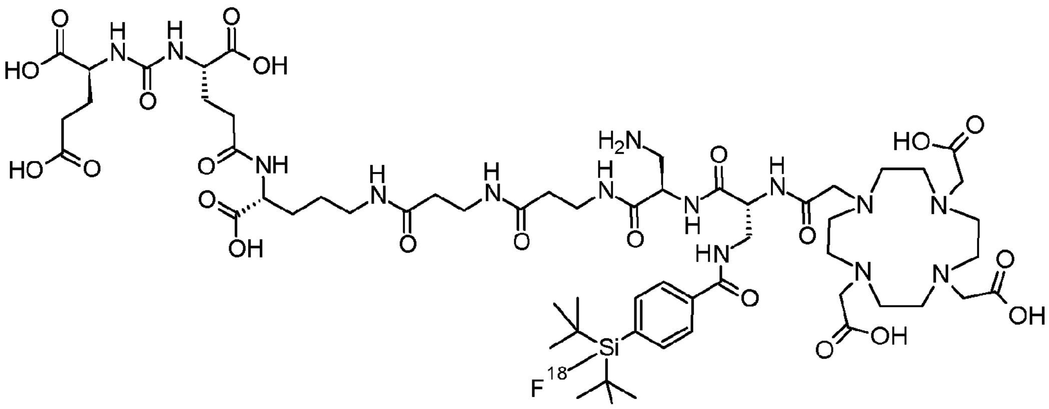

;

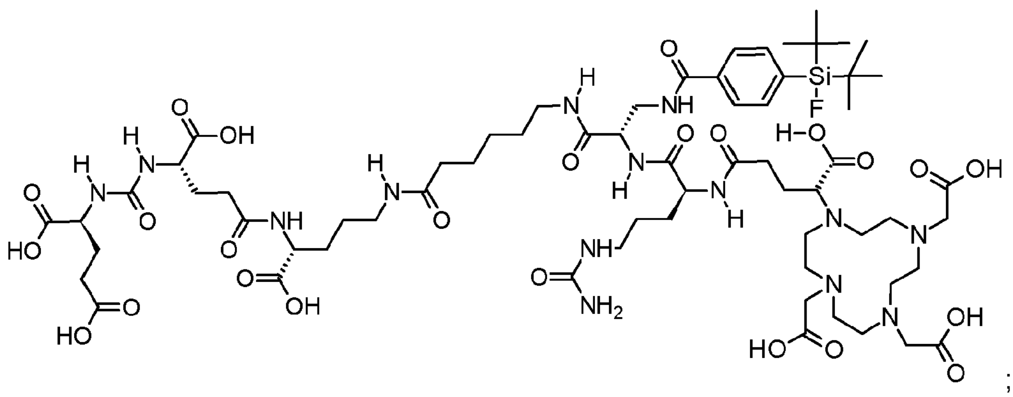

;

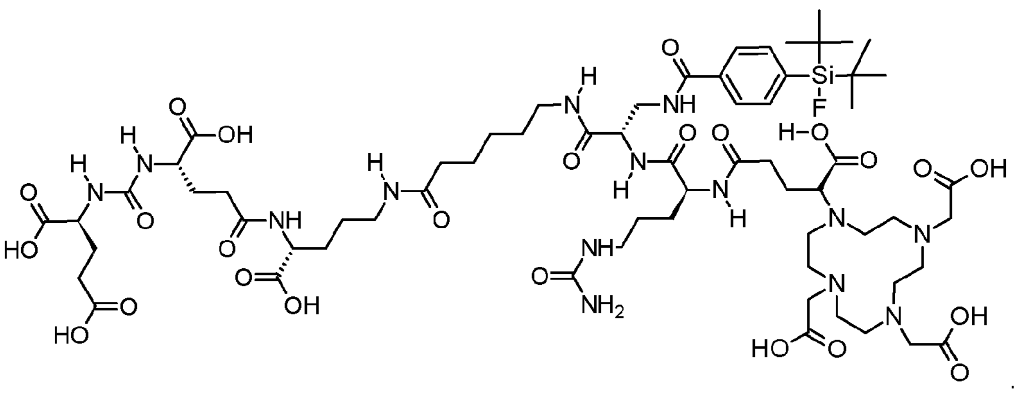

.

oder ein pharmazeutisch unbedenkliches Salz davon, wobei die Verbindung gegebenenfalls ein chelatisiertes nichtradioaktives oder radioaktives Kation enthält und das Fluoratom gegebenenfalls $^{18}F$ ist.

**18.** Verbindung nach einem der Ansprüche 1 bis 17, wobei die Chelatbildnergruppierung ein chelatisiertes Kation enthält, das aus den Kationen von $^{43}Sc$, $^{44}Sc$, $^{47}Sc$, $^{64}Cu$, $^{67}Cu$, $^{67}Ga$, $^{68}Ga$, $^{90}Y$, $^{111}In$, $^{149}Tb$, $^{152}Tb$, $^{155}Tb$, $^{161}Tb$, $^{166}Ho$, $^{177}Lu$, $^{186}Re$, $^{188}Re$, $^{212}Pb$, $^{212}Bi$, $^{213}Bi$, $^{225}Ac$ und $^{227}Th$ oder einem kationischen Molekül, das $^{18}F$ umfasst, ausgewählt ist.

**19.** Verbindung nach Anspruch 1, bei der es sich um:

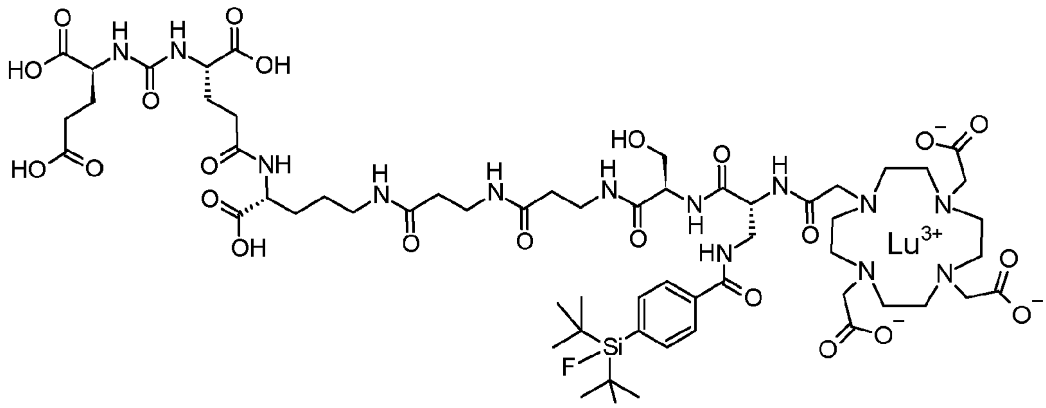

;

handelt, oder ein pharmazeutisch unbedenkliches Salz davon, wobei das Fluoratom gegebenenfalls $^{18}F$ ist.

**20.** Pharmazeutisch unbedenkliches Salz einer Verbindung nach einem der Ansprüche 1 bis 19.

**21.** Pharmazeutische oder diagnostische Zusammensetzung, die eine oder mehrere Verbindungen nach einem der Ansprüche 1 bis 20 umfasst oder daraus besteht.

**22.** Verbindung nach einem der Ansprüche 1 bis 20 oder Zusammensetzung nach Anspruch 21 zur Verwendung als Krebstherapeutikum, Krebsdiagnostikum oder - bildgebungsmittel.

**23.** Verbindung nach einem der Ansprüche 1 bis 20 oder Zusammensetzung nach Anspruch 21 zur Verwendung als Krebsdiagnostikum oder -bildgebungsmittel oder zur Verwendung bei der Behandlung von Krebs, wobei es sich bei dem Krebs um Prostata-, Brust-, Lungen-, Kolorektal- oder Nierenzellkarzinom handelt.

## Revendications

**1.** Composé de formule (1c) :

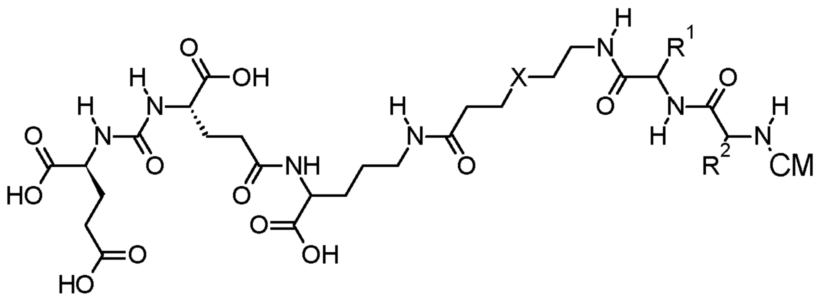

(1c)

ou sel ou tout isomère individuel pharmaceutiquement acceptable correspondant, soit
$R^1$ est $-(CH_2)_nR^3$, où n est 1, 2 ou 3 et $R^3$ est choisi parmi OH, $NH_2$ ou $NHC(O)NH_2$ et $R^2$ est

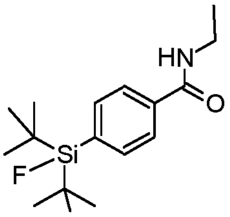

;

soit
$R^2$ est $-(CH_2)_nR^3$, où n est 1, 2 ou 3 et $R^3$ est choisi parmi OH, $NH_2$ ou $NHC(O)NH_2$ et $R^1$ est

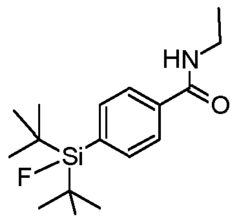

;

X est $CH_2$ ou NHCO et

CM représente un groupement chélateur, éventuellement contenant un cation chélaté non radioactif ou radioactif et l'atome de fluor est éventuellement $^{18}F$.

**2.** Composé selon la revendication 1 de formule (1a) :

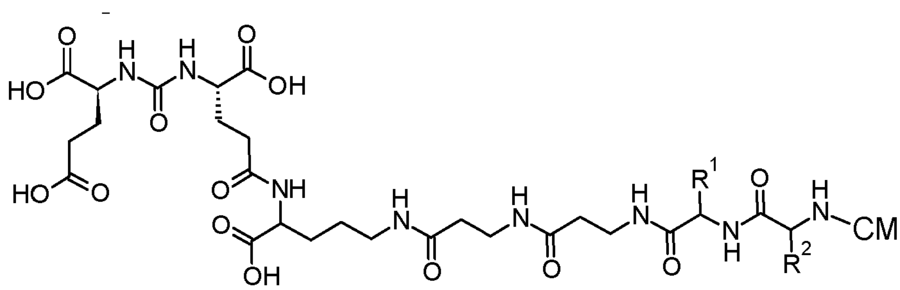

(1a),

ou sel ou tout isomère individuel pharmaceutiquement acceptable correspondant, soit

$R^1$ est $-(CH_2)_nR^3$, où n est 1, 2 ou 3 et $R^3$ est choisi parmi OH, $NH_2$ ou $NHC(O)NH_2$ et $R^2$ est

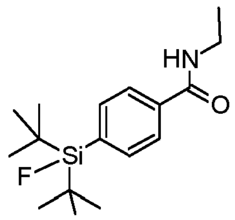

;

soit

$R^2$ est $-(CH_2)_nR^3$, où n est 1, 2 ou 3 et $R^3$ est choisi parmi OH, $NH_2$ ou $NHC(O)NH_2$ et $R^1$ est

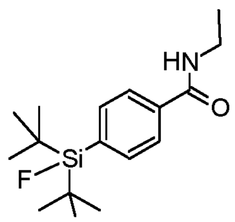

;

et

CM représente un groupement chélateur, éventuellement contenant un cation chélaté non radioactif ou radioactif et l'atome de fluor est éventuellement $^{18}F$.

**3.** Composé selon la revendication 1 de formule (1b) :

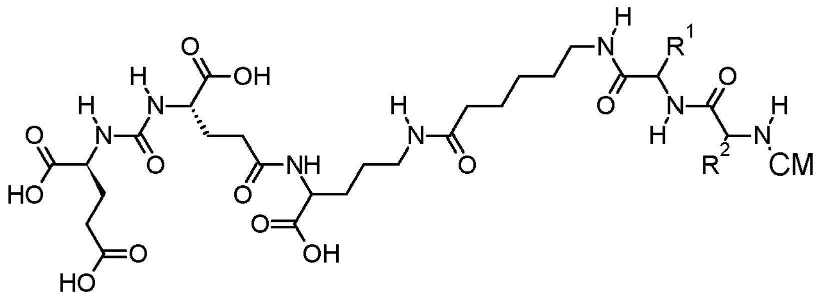

(1b)

ou sel ou tout isomère individuel pharmaceutiquement acceptable correspondant, soit
$R^1$ est $-(CH_2)_nR^3$, où n est 1, 2 ou 3 et $R^3$ est choisi parmi OH, $NH_2$ ou $NHC(O)NH_2$ et $R^2$ est

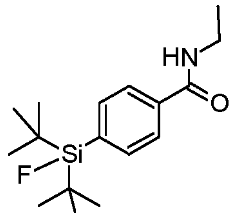

;

soit
$R^2$ est $-(CH_2)_nR^3$, où n est 1, 2 ou 3 et $R^3$ est choisi parmi OH, $NH_2$ ou $NHC(O)NH_2$ et $R^1$ est

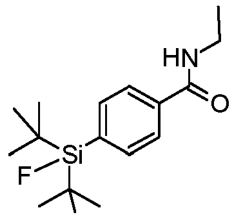

;

et
CM représente un groupement chélateur, éventuellement contenant un cation chélaté non radioactif ou radioactif et l'atome de fluor est éventuellement $^{18}F$.

**4.** Composé selon la revendication 1 de formule (1) :

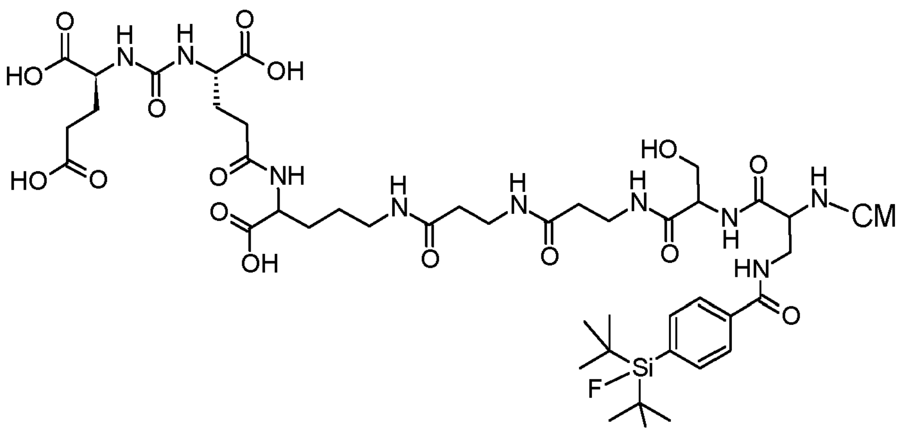

(1);

ou sel ou tout isomère individuel pharmaceutiquement acceptable correspondant, dans laquelle
CM représente un groupement chélateur, éventuellement contenant un cation chélaté non radioactif ou radioactif et l'atome de fluor est éventuellement $^{18}F$.

**5.** Composé selon la revendication 4 qui est un composé de formule (2) :

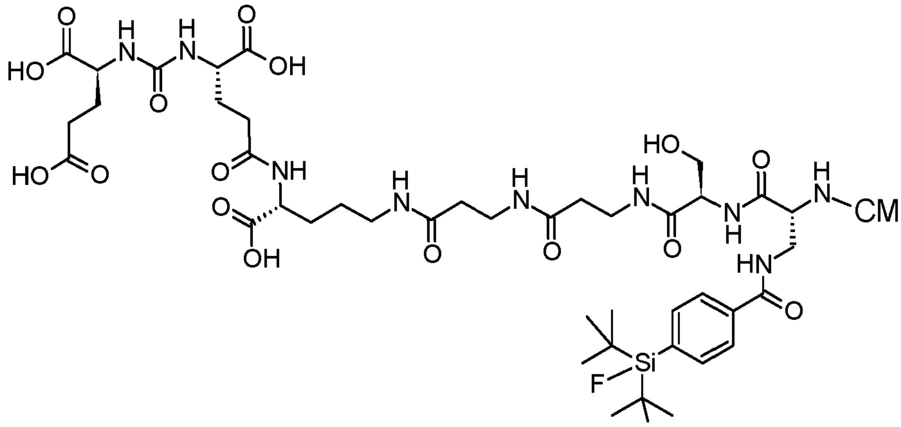

(2);

ou sel ou tout isomère individuel pharmaceutiquement acceptable correspondant, dans laquelle CM représente un groupement chélateur, éventuellement contenant un cation chélaté non radioactif ou radioactif et l'atome de fluor est éventuellement $^{18}F$.

**6.** Composé selon la revendication 5 qui est un composé de formule (3) :

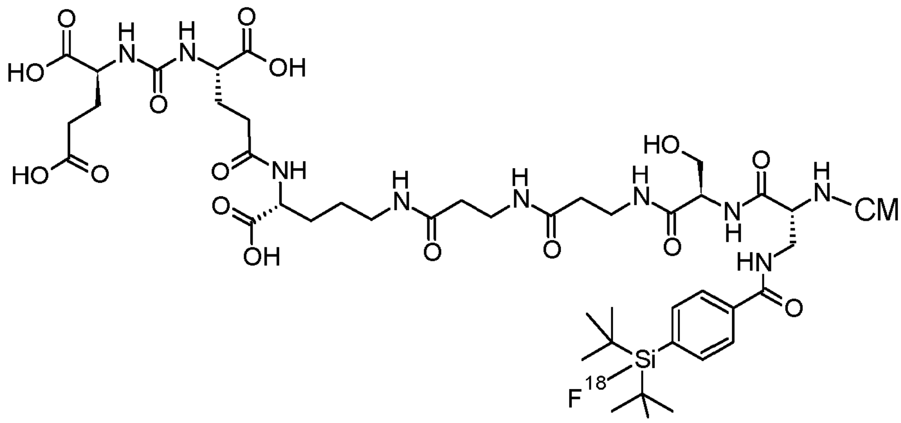

(3);

ou sel ou tout isomère individuel pharmaceutiquement acceptable correspondant, dans laquelle CM représente un groupement chélateur, éventuellement contenant un cation chélaté non radioactif ou radioactif.

**7.** Composé selon la revendication 2 qui est un composé de formule (1') :

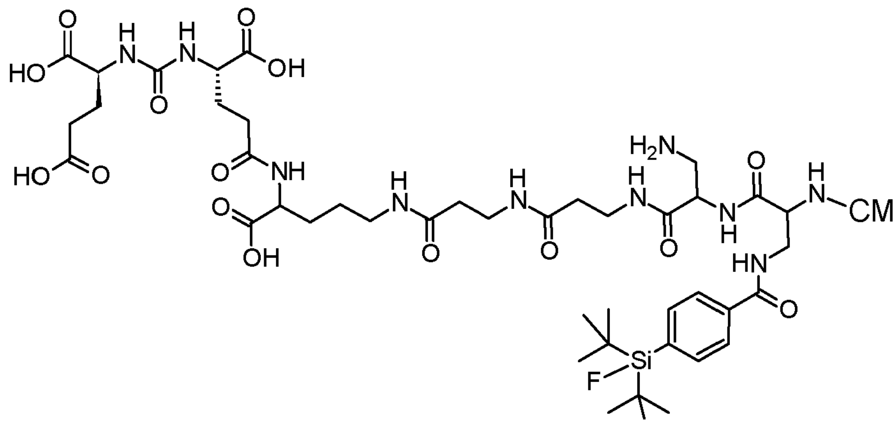

(1');

ou sel ou tout isomère individuel pharmaceutiquement acceptable correspondant, dans laquelle
CM représente un groupement chélateur, éventuellement contenant un cation chélaté non radioactif ou radioactif et l'atome de fluor est éventuellement $^{18}F$.

**8.** Composé selon la revendication 7 qui est un composé de formule (2a) :

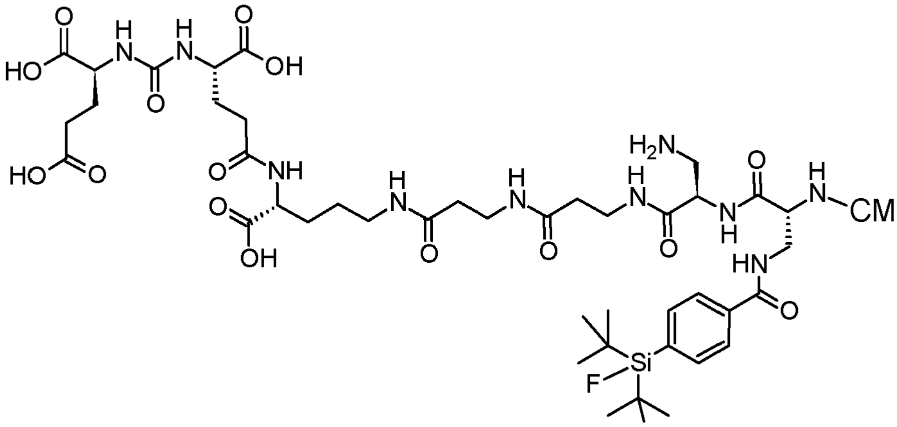

(2a);

ou sel ou tout isomère individuel pharmaceutiquement acceptable correspondant, dans laquelle
CM représente un groupement chélateur, éventuellement contenant un cation chélaté non radioactif ou radioactif et l'atome de fluor est éventuellement $^{18}F$.

**9.** Composé selon la revendication 8 qui est un composé de formule (3a) :

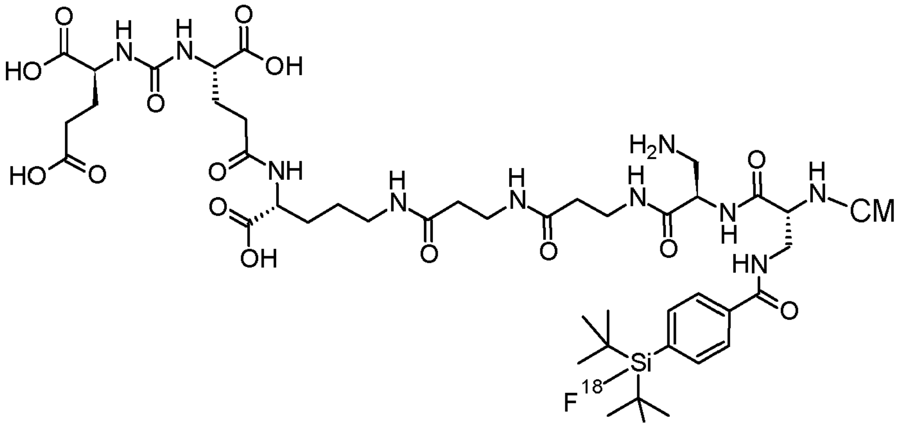

(3a);

ou sel ou tout isomère individuel pharmaceutiquement acceptable correspondant, dans laquelle
CM représente un groupement chélateur, éventuellement contenant un cation chélaté non radioactif ou radioactif.

**10.** Composé selon la revendication 3 qui est un composé de formule (1") :

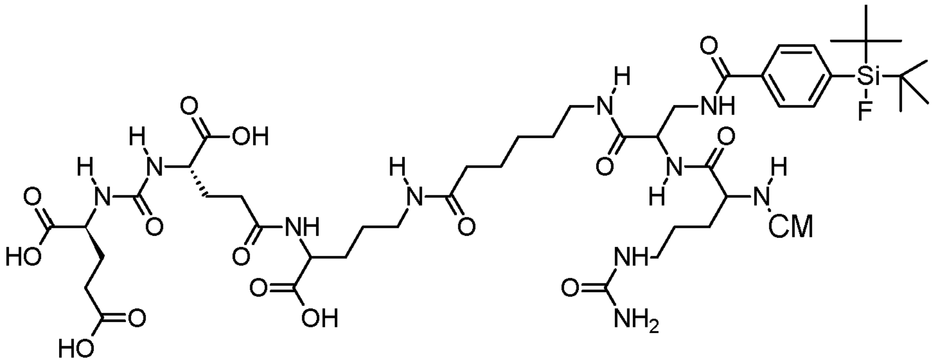

(1");

11. Composé selon la revendication 10 qui est un composé de formule (2b) :

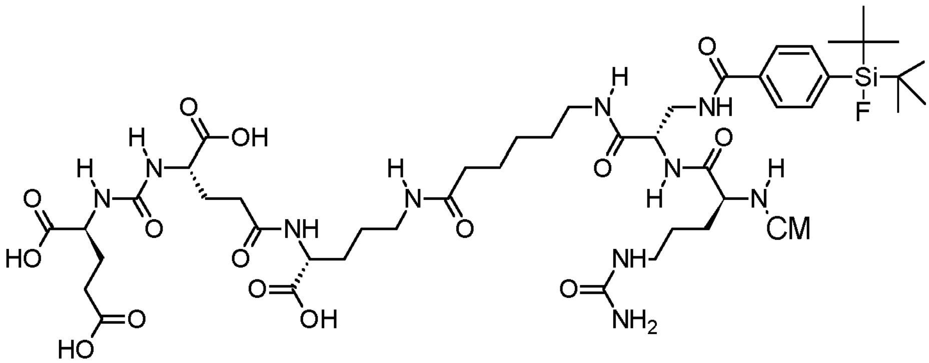

(2b);

ou sel ou tout isomère individuel pharmaceutiquement acceptable correspondant, dans laquelle
CM représente un groupement chélateur, éventuellement contenant un cation chélaté non radioactif ou radioactif et l'atome de fluor est éventuellement $^{18}F$.

12. Composé selon la revendication 11 qui est un composé de formule (3b) :

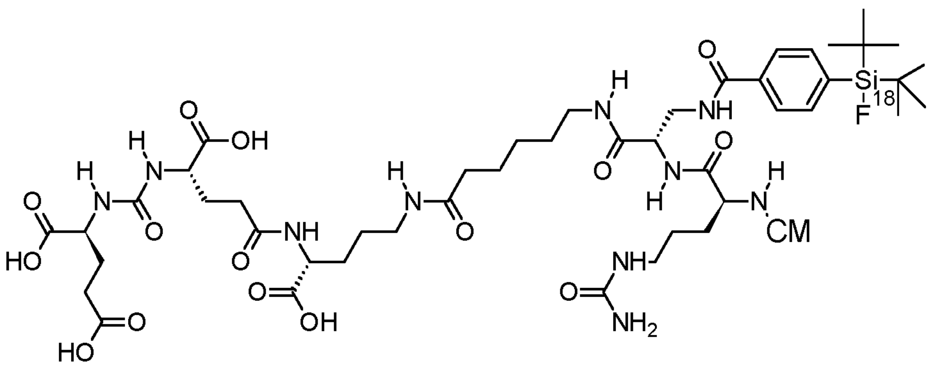

(3b);

ou sel ou tout isomère individuel pharmaceutiquement acceptable correspondant, dans laquelle
CM représente un groupement chélateur, éventuellement contenant un cation chélaté non radioactif ou radioactif.

13. Composé selon l'une quelconque des revendications 1 à 12, dans lequel le groupement chélateur comprend au moins l'un parmi :

(i) une structure cyclique macrocyclique comportant 8 à 20 atomes de cycle parmi lesquels 2 ou plus sont des hétéroatomes choisis parmi des atomes d'oxygène et des atomes d'azote ;
(ii) une structure chélatante acyclique, à chaîne ouverte comportant 8 à 20 atomes de chaîne principale parmi

lesquels 2 ou plus sont des hétéroatomes choisis parmi des atomes d'oxygène et des atomes d'azote ; ou
(iii) une structure chélatante ramifiée contenant un atome de carbone quaternaire.

**14.** Composé selon l'une quelconque des revendications 1 à 12, dans lequel le groupement chélateur est choisi parmi bis(carboxyméthyl)-1,4,8,11-tétraazabicyclo[6.6.2]hexadécane (CBTE2a), acide cyclohexyl-1,2-diaminetétraacétique (CDTA), acide 4-(1,4,8,11-tétraazacyclotétradéc-1-yl)-méthylbenzoïque (CPTA), N'-[5-[acétyl(hydroxy)amino]pentyl]-N-[5-[[4-[5-aminopentyl-(hydroxy)amino]-4-oxobutanoyl]amino]pentyl]-N-hydroxybutandiamide (DFO), 4,11-bis(carboxyméthyl)-1,4,8,11-tétraazabicyclo[6.6.2]hexadécane (DO2A), acide 1,4,7,10-tétracyclododécan-N,N',N",N'''-tétraacétique (DOTA), acide α-(2-carboxyéthyl)-1,4,7,10-tétraazacyclododécane-1,4,7,10-tétraacétique (DOTAGA), acide 1,4,7,10 tétraazacyclododécane N,N',N",N'''-1,4,7,10-tétra(méthylène)phosphonique (DOTMP), N,N'-dipyridoxyléthylènediamine-N,N'-diacétate-5,5'-bis(phosphate) (DPDP), acide diéthylène triamine N,N',N" penta(méthylène) phosphonique (DTMP), acide diéthylènetriaminepentaacétique (DTPA), acide éthylènediamine-N,N'-tétraacétique (EDTA), acide éthylèneglycol-0,0-bis(2-aminoéthyl)-N,N,N',N'-tétraacétique (EGTA), acide N,N-bis(hydroxybenzyl)-éthylènediamine-N,N'-diacétique (HBED), acide hydroxyéthyldiaminetriacétique (HEDTA), 1-(p-nitrobenzyl)-1,4,7,10-tétraazacyclodécan-4,7,10-triacétate (HP-DOA3), 6-hydrazinyl-N-méthylpyridine-3-carboxamide (HYNIC), chélateurs de tétra 3-hydroxy-N-méthyl-2-pyridinone (acide 4-((4-(3-(bis(2-(3-hydroxy-1-méthyl-2-oxo-1,2-dihydropyridine-4-carboxamido)éthyl)amino)-2-((bis(2-(3-hydroxy-1-méthyl-2-oxo-1,2-dihydropyridine-4-carboxamido)éthyl)amino)méthyl)propyl)phényl)amino)-4-oxobutanoïque), abrégé en Me-3,2-HOPO, acide 1,4,7-triazacyclononan-1-succinique-4,7-diacétique (NODASA), 1-(1-carboxy-3-carboxypropyl)-4,7-(carbooxy)-1,4,7-triazacyclononane (NODAGA), acide 1,4,7-triazacyclononanetriacétique (NOTA), 4,11-bis(carboxyméthyl)-1,4,8,11-tétraazabicyclo[6.6.2]hexadécane (TE2A), acide 1,4,8,11-tétraazacyclododécane-1,4,8,11-tétraacétique (TETA), tris(hydroxypyridinone) (THP), acide terpyridin-bis(méthylèneaminotétraacétique (TMT), acide 1,4,7-triazacyclononane-1,4,7-tris[méthylène(2-carboxyéthyl)phosphinique] (TRAP), acide 1,4,7,10-tétraazacyclotridécan-N,N',N",N'''-tétraacétique (TRITA), acide 3-[[4,7-bis[[2-carboxyéthyl(hydroxy)phosphoryl]méthyl]-1,4,7-triazonan-1-yl]méthyl-hydroxy-phosphoryl]propanoïque et acide triéthylènetétraaminehexaacétique (TTHA).

**15.** Composé selon la revendication 14, dans lequel le groupement chélateur est l'acide 1,4,7,10-tétracyclododécan-N,N',N",N'''-tétraacétique (DOTA) ou l'acide α-(2-carboxyéthyl)-1,4,7,10-tétraazacyclododécane-1,4,7,10-tétraacétique (DOTAGA).

**16.** Composé selon la revendication 1, qui est choisi parmi :

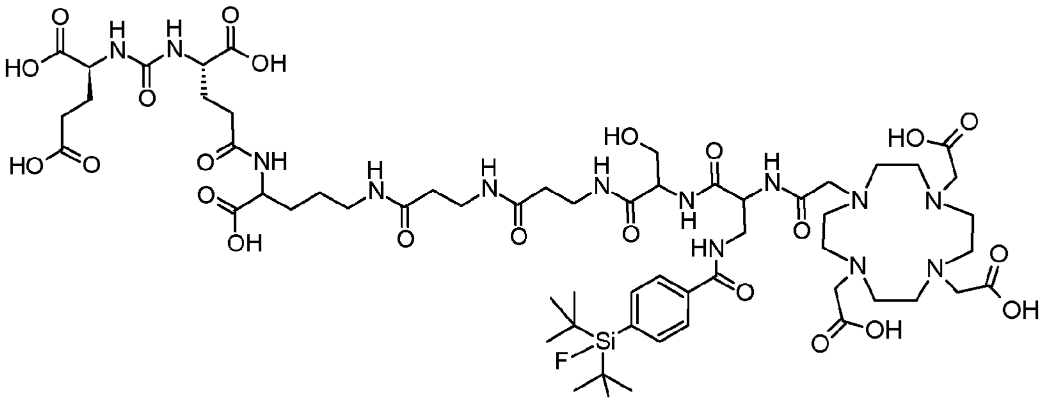

;

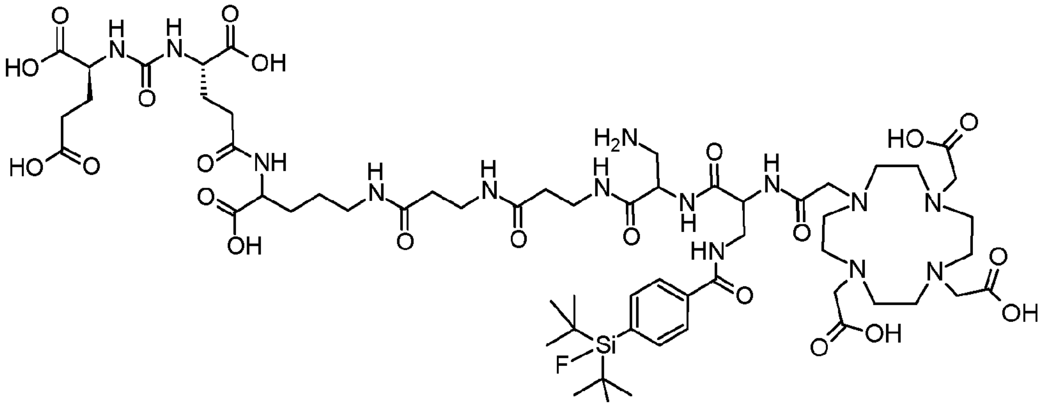

;

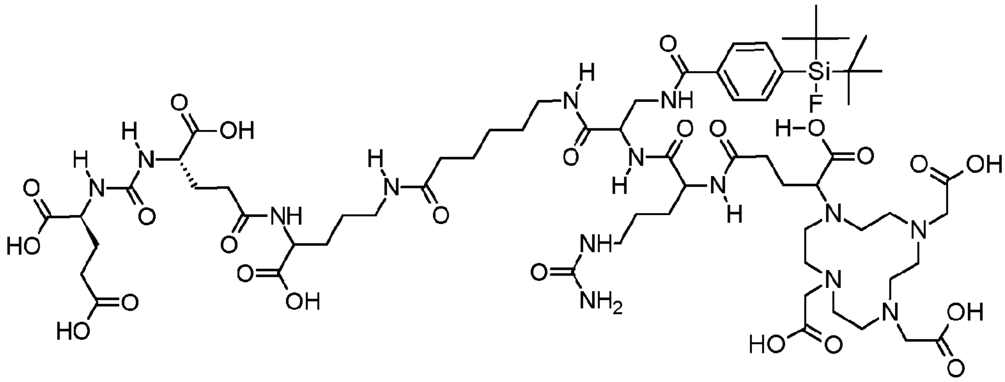

ou sel ou tout isomère individuel pharmaceutiquement acceptable correspondant, dans lequel le composé contenant éventuellement un cation chélaté non radioactif ou radioactif et l'atome de fluor est éventuellement $^{18}$F.

**17.** Composé selon la revendication 1 qui est choisi parmi :

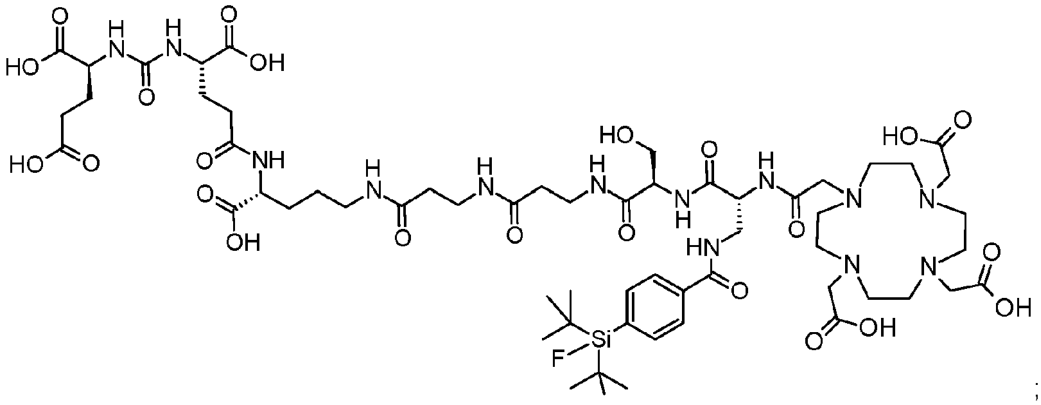

;

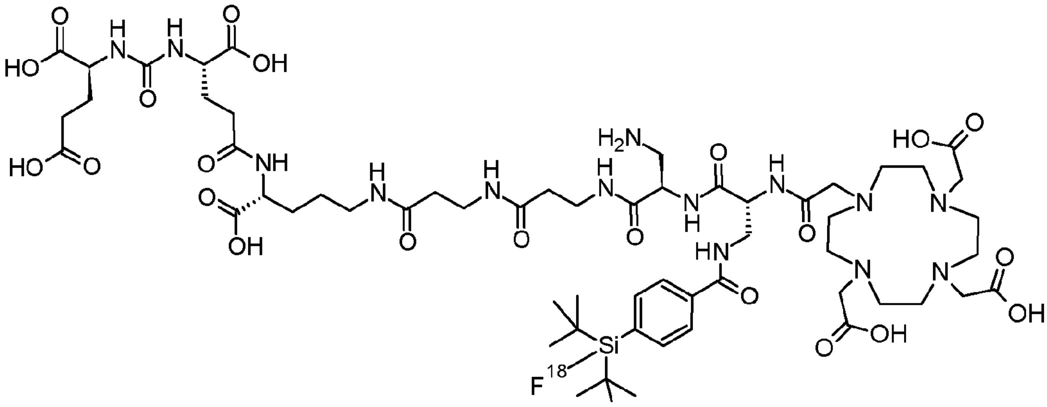

;

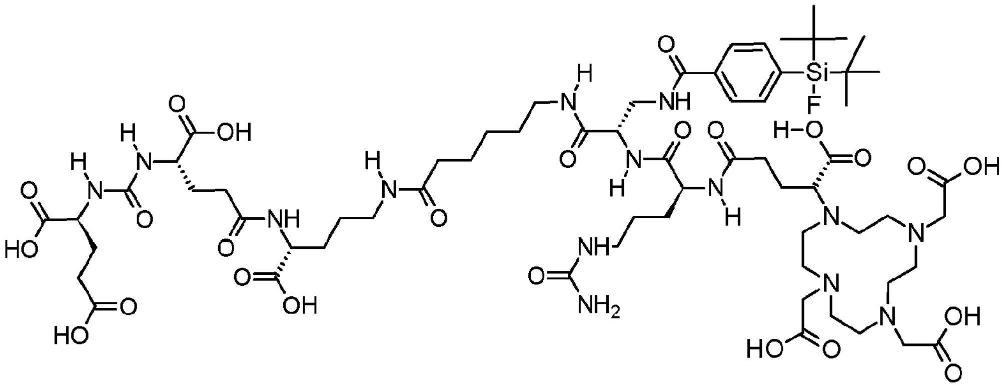

;

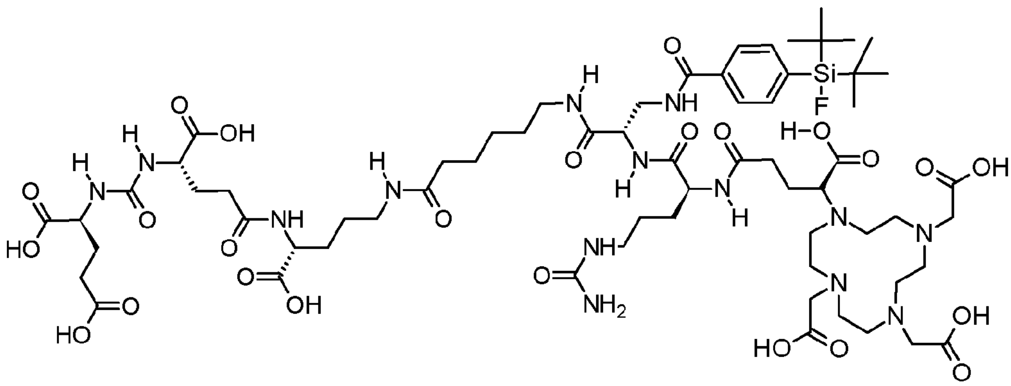

.

ou sel pharmaceutiquement acceptable correspondant, dans lequel le composé contenant éventuellement un cation chélaté non radioactif ou radioactif et l'atome de fluor est éventuellement $^{18}F$.

**18.** Composé selon l'une quelconque des revendications 1 à 17, dans lequel le groupement chélateur contient un cation chélaté choisi parmi les cations de $^{43}Sc$, $^{44}Sc$, $^{47}Sc$, $^{64}Cu$, $^{67}Cu$, $^{67}Ga$, $^{68}Ga$, $^{90}Y$, $^{111}In$, $^{149}Tb$, $^{152}Tb$, $^{155}Tb$, $^{161}Tb$, $^{166}Ho$, $^{177}Lu$, $^{186}Re$, $^{188}Re$, $^{212}Pb$, $^{212}Bi$, $^{213}Bi$, $^{225}Ac$, et $^{227}Th$ ou une molécule cationique comprenant $^{18}F$.

**19.** Composé selon la revendication 1 qui est :

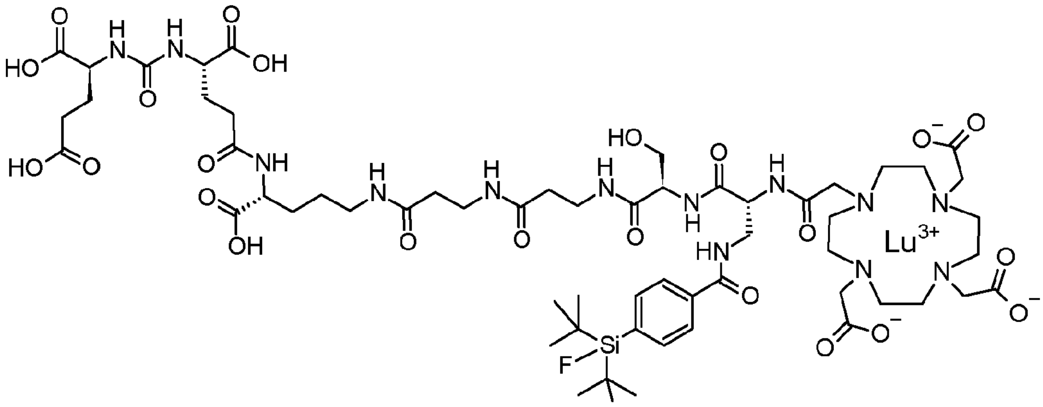

;

ou sel pharmaceutiquement acceptable correspondant, dans lequel l'atome de fluor est éventuellement $^{18}F$.

**20.** Sel pharmaceutiquement acceptable d'un composé selon l'une quelconque des revendications 1 à 19.

**21.** Composition pharmaceutique ou de diagnostic comprenant un ou plusieurs composés selon l'une quelconque des revendications 1 à 20 ou constituée d'un ou plusieurs de ceux-ci.

**22.** Composé selon l'une quelconque des revendications 1 à 20 ou composition selon la revendication 21, pour une utilisation en tant qu'agent thérapeutique du cancer, agent diagnostique du cancer ou agent d'imagerie du cancer.

**23.** Composé selon l'une quelconque des revendications 1 à 20 ou composition selon la revendication 21, pour une utilisation en tant qu'agent de diagnostic ou d'imagerie du cancer ou pour une utilisation dans le traitement d'un cancer, dans lequel/laquelle le cancer est un cancer de la prostate, du sein, du poumon, colorectal ou un carcinome de cellules rénales.

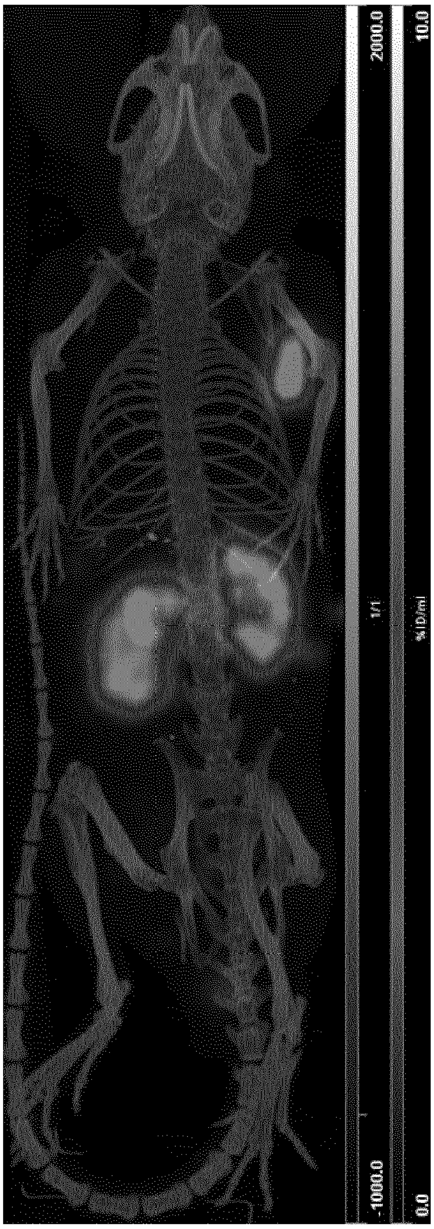


[$^{177}$Lu]Lu-rhPSMA-**7.3**

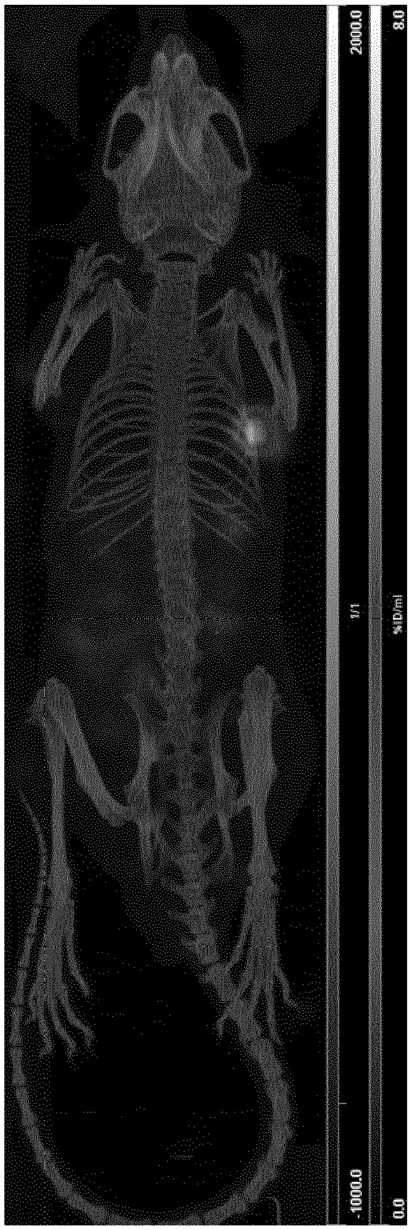


[$^{177}$Lu]Lu-**2C013**

**Fig. 1**

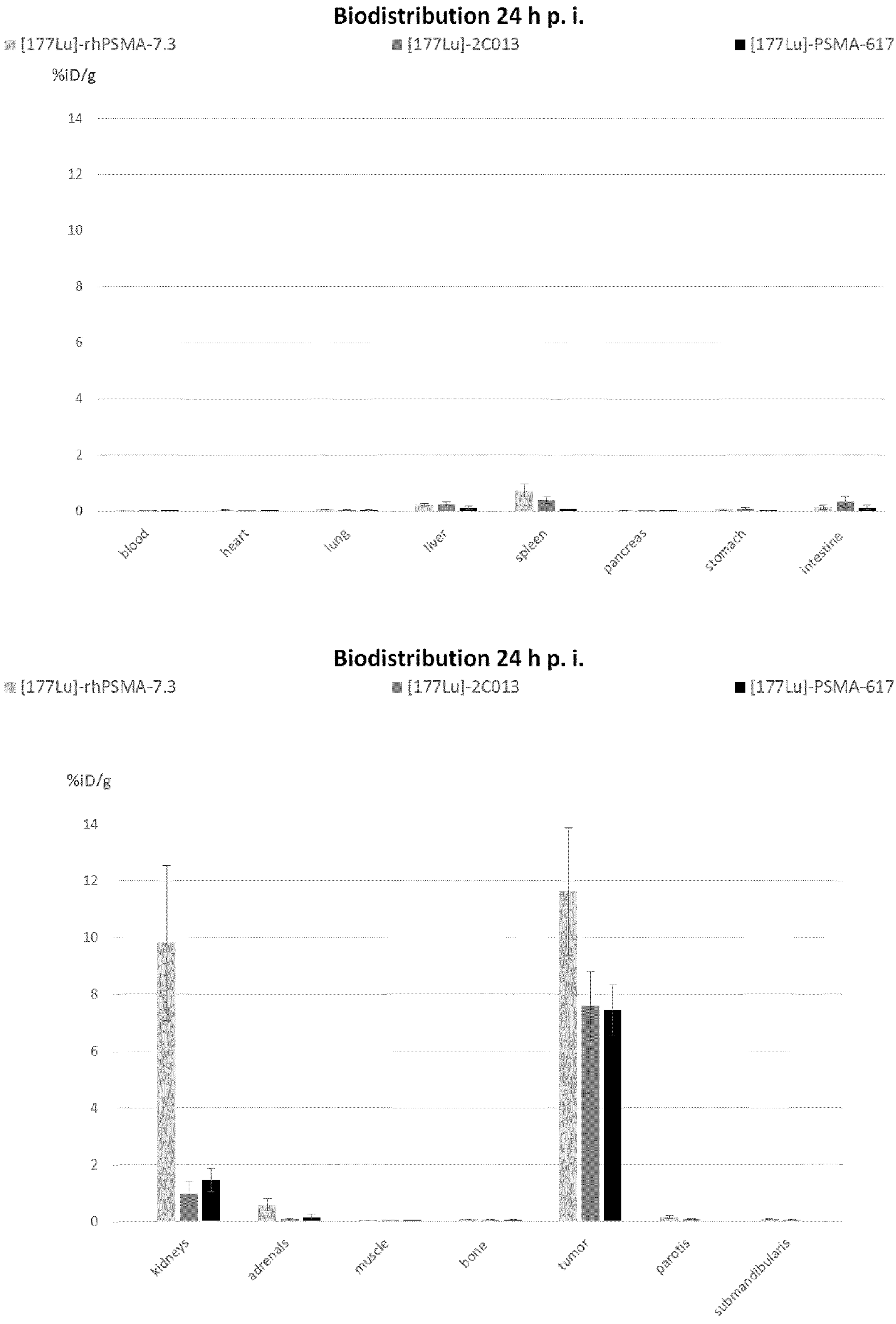
Biodistribution 24 h p. i.
[177Lu]-rhPSMA-7.3
[177Lu]-2C013
[177Lu]-PSMA-617
%iD/g
14
12
10
8
6
4
2
0
blood
heart
lung
liver
spleen
pancreas
stomach
intestine
Biodistribution 24 h p. i.
[177Lu]-rhPSMA-7.3
[177Lu]-2C013
[177Lu]-PSMA-617
%iD/g
14
12
10
8
6
4
2
0
kidneys
adrenals
muscle
bone
tumor
parotis
submandibularis

**Fig. 2**

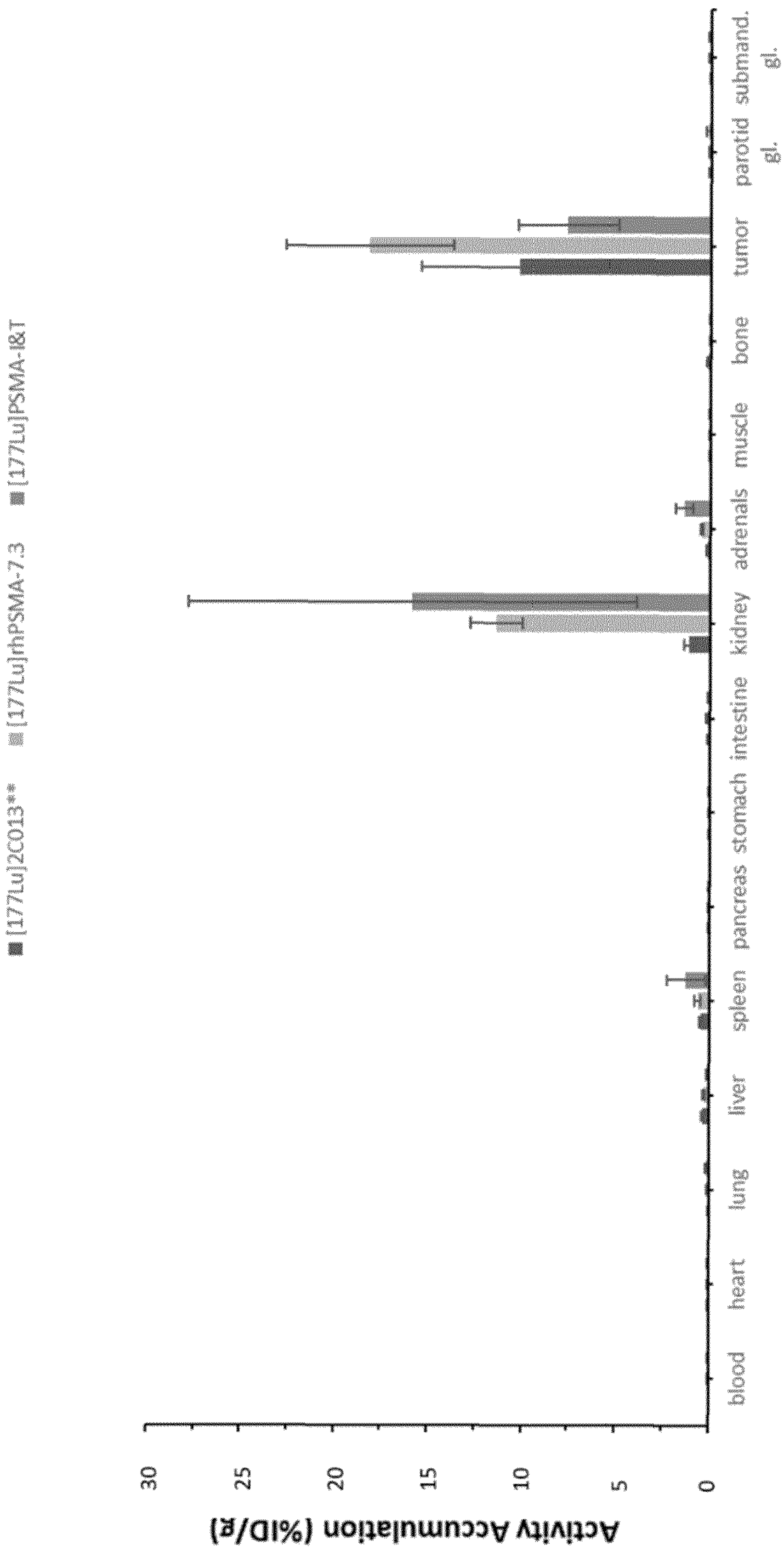
[177Lu]2C013**
[177Lu]rhPSMA-7.3
[177Lu]PSMA-I&T
Activity Accumulation (%ID/g)
0
5
10
15
20
25
30
blood
heart
lung
liver
spleen
pancreas
stomach
intestine
kidney
adrenals
muscle
bone
tumor
parotid gl.
submand. gl.

Fig. 3

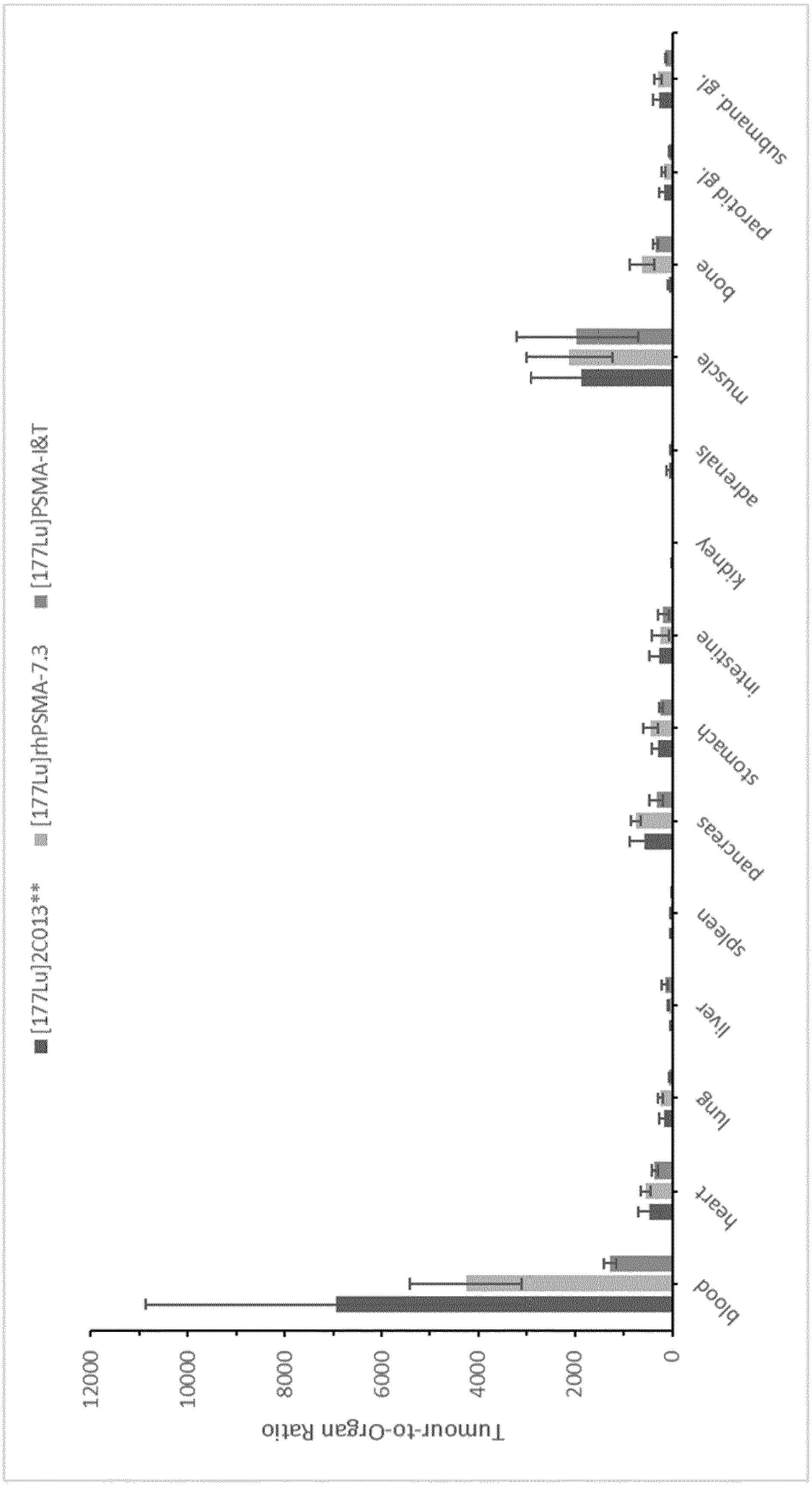
[177Lu]2C013**
[177Lu]rhPSMA-7.3
[177Lu]PSMA-I&T
Tumour-to-Organ Ratio
0
2000
4000
6000
8000
10000
12000
blood
heart
lung
liver
spleen
pancreas
stomach
intestine
kidney
adrenals
muscle
bone
parotid gl.
submand. gl.

Fig. 4a

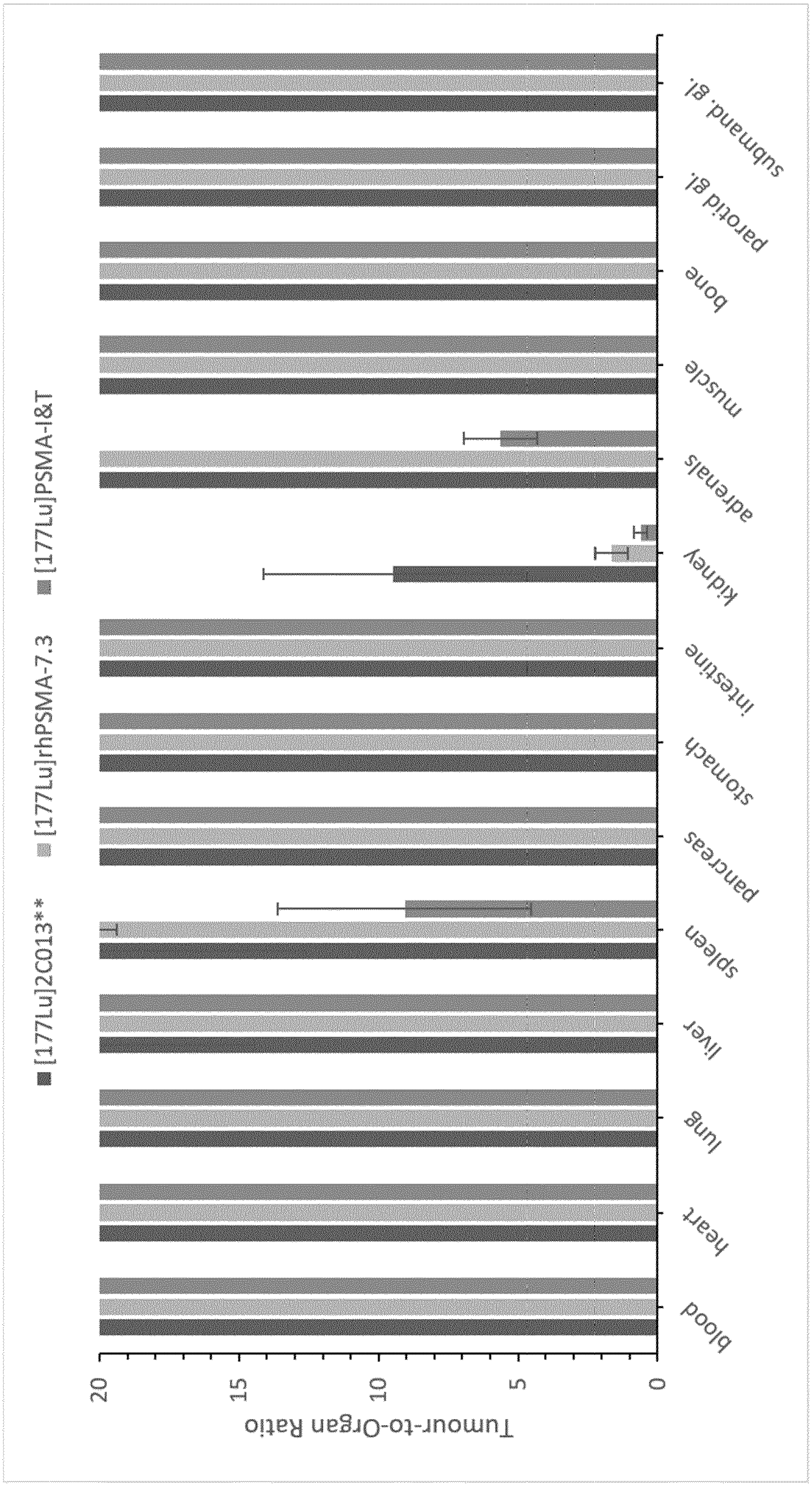
[177Lu]2C013**
[177Lu]rhPSMA-7.3
[177Lu]PSMA-I&T
Tumour-to-Organ Ratio
20
15
10
5
0
blood
heart
lung
liver
spleen
pancreas
stomach
intestine
kidney
adrenals
muscle
bone
parotid gl.
submand. gl.

Fig. 4b

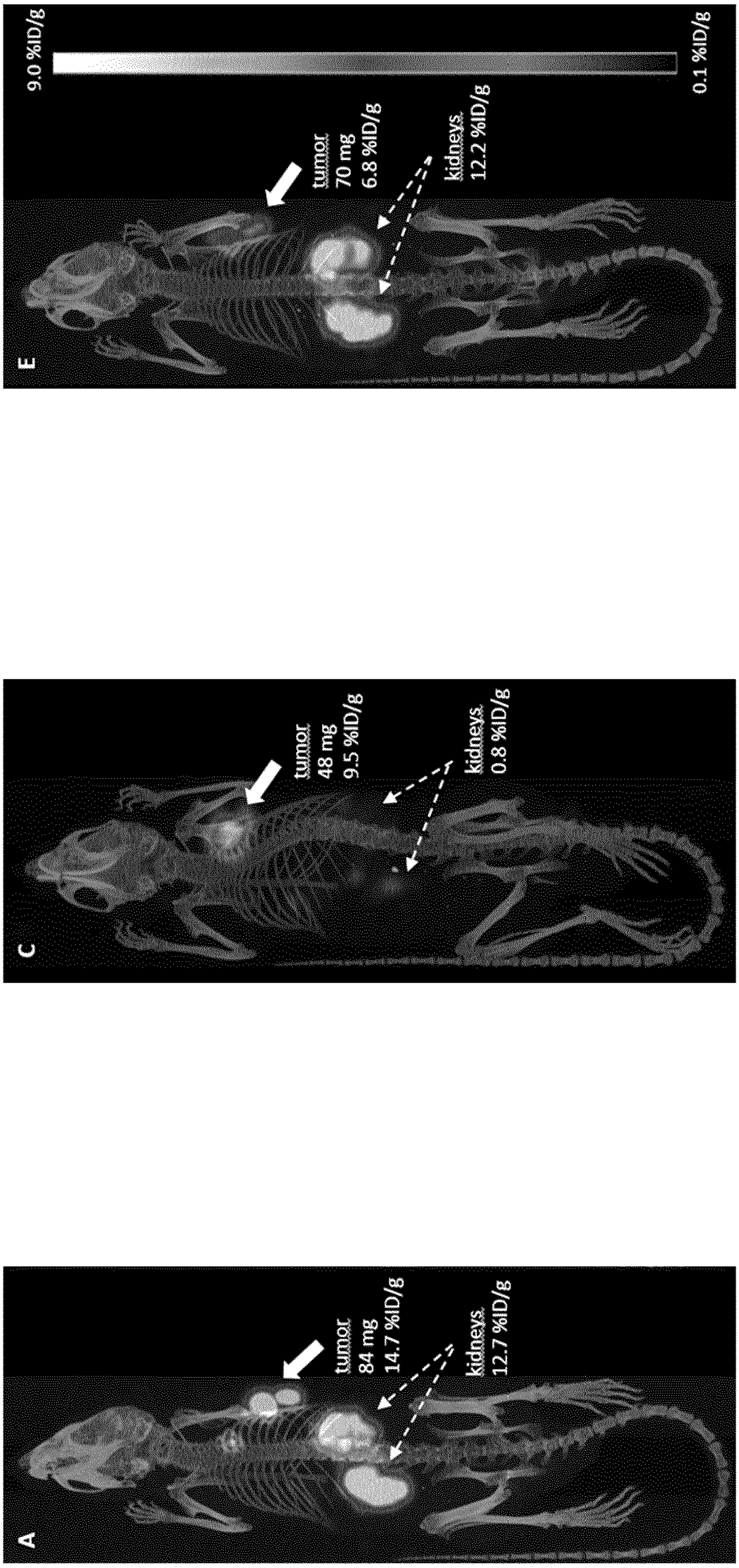
E
tumor
70 mg
6.8 %ID/g
kidneys
12.2 %ID/g
9.0 %ID/g
0.1 %ID/g
C
tumor
48 mg
9.5 %ID/g
kidneys
0.8 %ID/g
A
tumor
84 mg
14.7 %ID/g
kidneys
12.7 %ID/g

**Fig. 5**

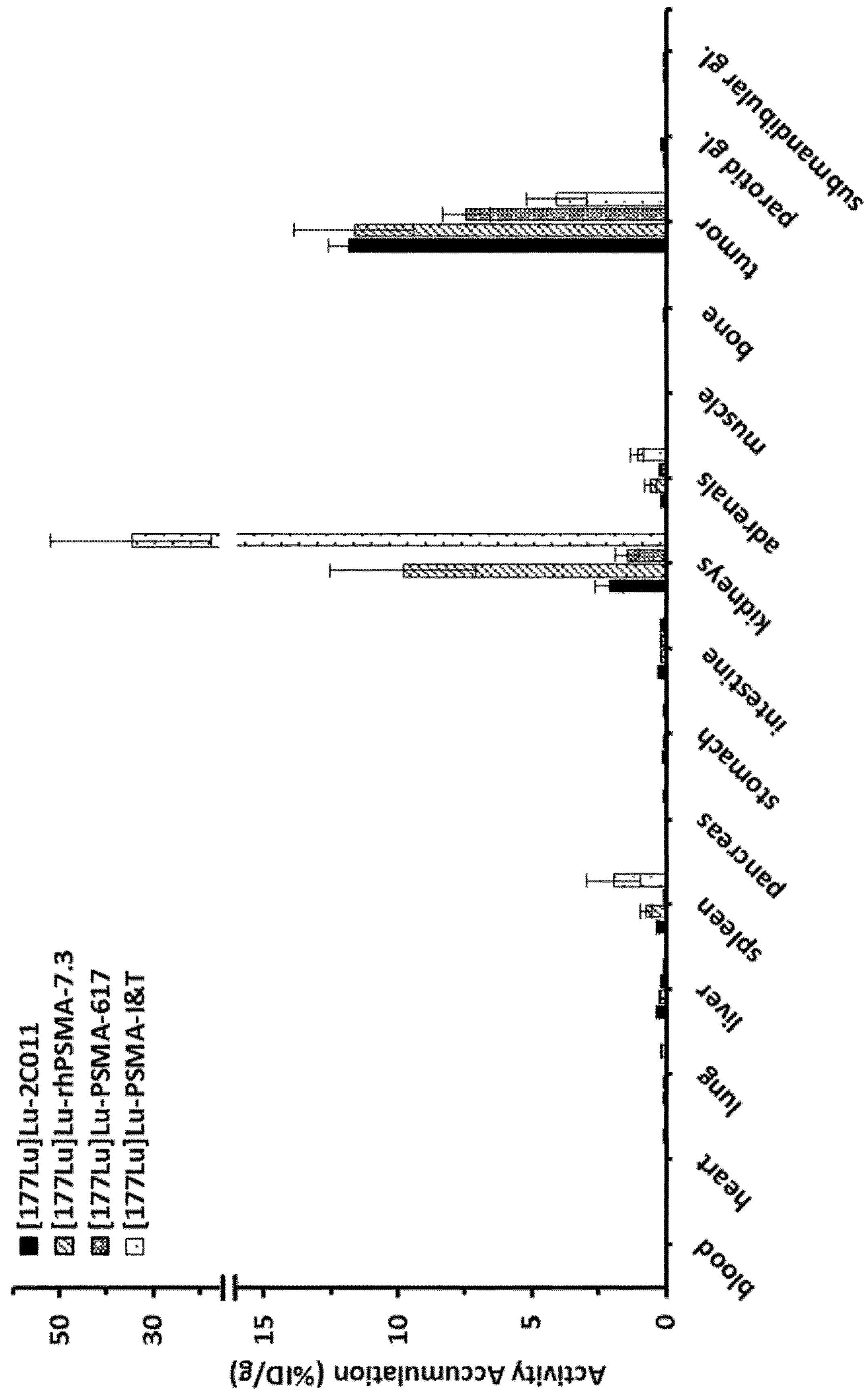
[177Lu]Lu-2C011
[177Lu]Lu-rhPSMA-7.3
[177Lu]Lu-PSMA-617
[177Lu]Lu-PSMA-I&T
Activity Accumulation (%ID/g)
0
5
10
15
30
50
blood
heart
lung
liver
spleen
pancreas
stomach
intestine
kidneys
adrenals
muscle
bone
tumor
parotid gl.
submandibular gl.

Fig. 6

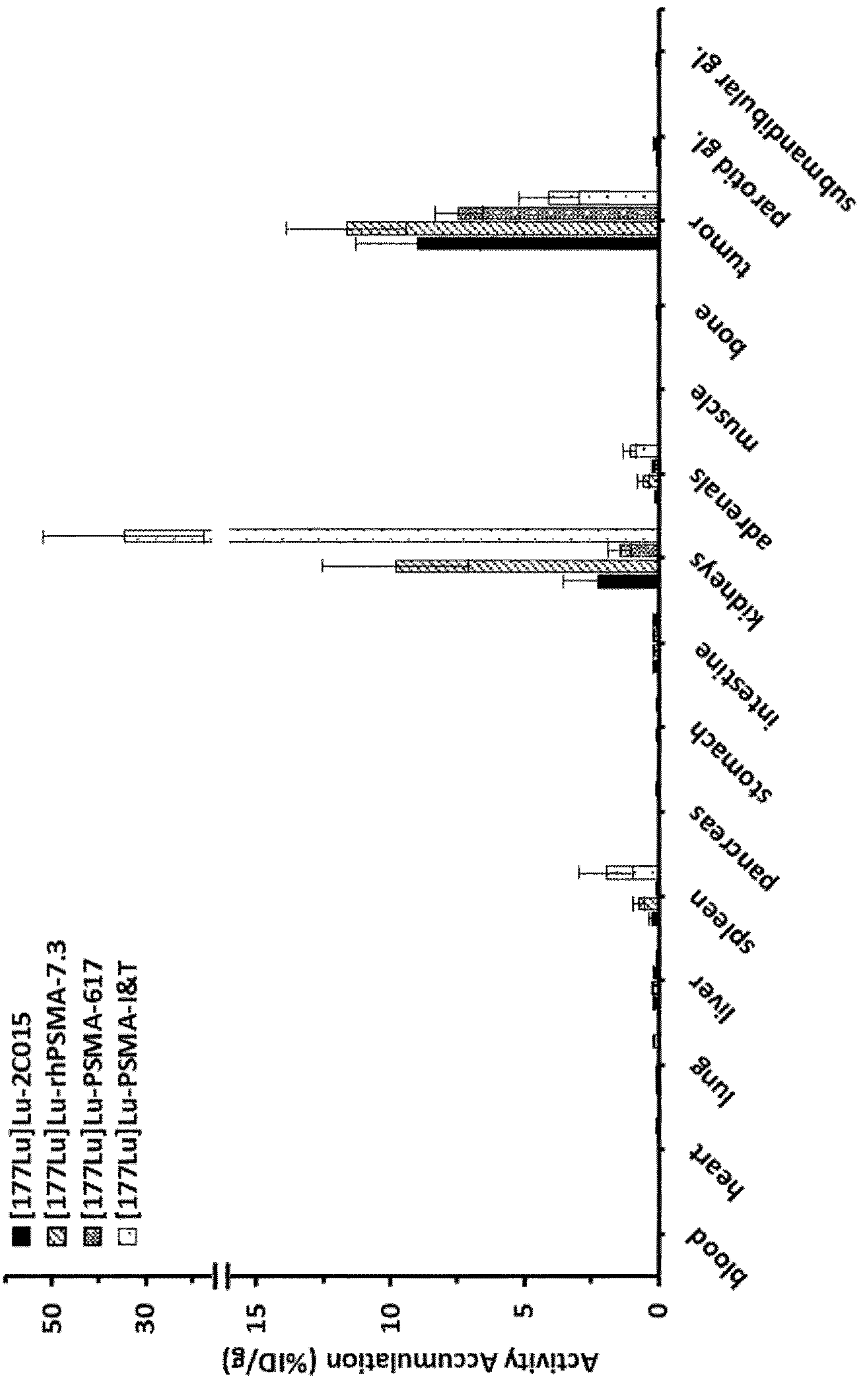
[177Lu]Lu-2C015
[177Lu]Lu-rhPSMA-7.3
[177Lu]Lu-PSMA-617
[177Lu]Lu-PSMA-I&T
Activity Accumulation (%ID/g)
0
5
10
15
30
50
blood
heart
lung
liver
spleen
pancreas
stomach
intestine
kidneys
adrenals
muscle
bone
tumor
parotid gl.
submandibular gl.

Fig. 7

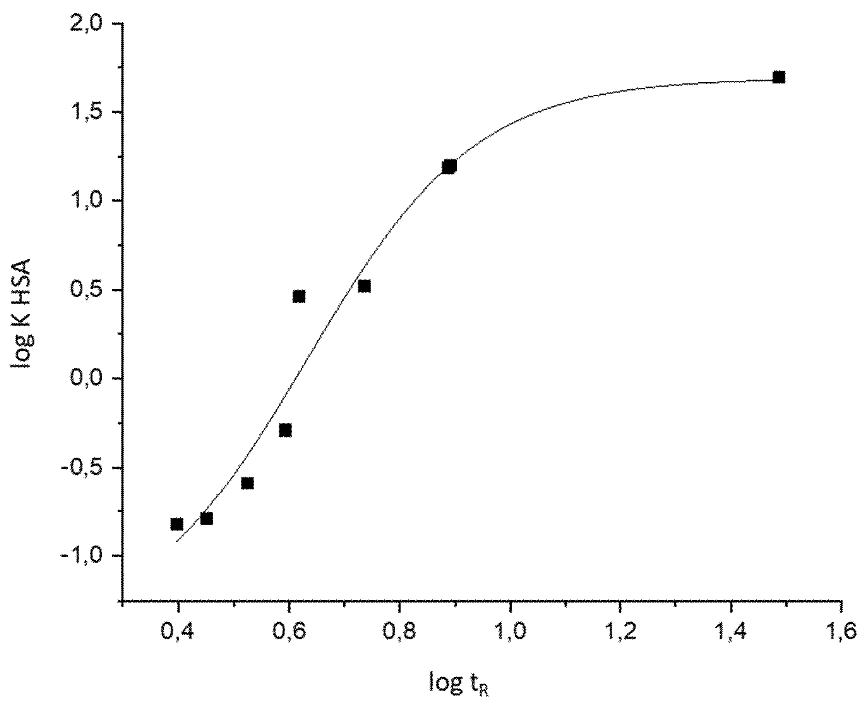


| reference | $t_R$ | Log $t_R$ | lit. HSA [%] | Log K HSA |
|---|---|---|---|---|
| p-benzyl alcohol | 2.50 | 0.40 | 13.15 | -0.82 |
| aniline | 2.83 | 0.45 | 14.06 | -0.79 |
| phenol | 3.35 | 0.52 | 20.69 | -0.59 |
| benzoic acid | 3.92 | 0.59 | 34.27 | -0.29 |
| Carbamazepin | 4.15 | 0.62 | 75.00 | 0.46 |
| p-nitrophenol | 5.45 | 0.74 | 77.65 | 0.52 |
| estradiol | 7.71 | 0.89 | 94.81 | 1.19 |
| probenecid | 7.78 | 0.89 | 95.00 | 1.20 |
| glibenclamide | 30.76 | 1.49 | 99.00 | 1.69 |

Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019020831 A
- WO 2020157184 A
- WO 2020157177 A


### Non-patent literature cited in the description


- **SILVER et al.** *Clinical Cancer Research*, 1997, vol. 3, 81-85
- **AFSHAR-OROMIEH et al.** *European journal of nuclear medicine and molecular imaging*, 2015, vol. 42, 197-209
- **ROBU et al.** *Journal of Nuclear Medicine, jnumed*, 2016, vol. 116, 178939
- **WEINEISEN et al.** *Journal of Nuclear Medicine*, 2014, vol. 55, 1083-1083
- **ROWE et al.** *Prostate cancer and prostatic diseases*, 2016
- **MAURER et al.** *Nature Reviews Urology*, 2016
- **ZHOU et al.** *Nature Reviews Drug Discovery*, 2005, vol. 4, 1015-1026
- **MACHULKIN et al.** *Journal of drug targeting*, 2016, 1-15
- **BARINKA et al.** *Journal of medicinal chemistry*, 2008, vol. 51, 7737-7743
- **ZHANG et al.** *Journal of the American Chemical Society*, 2010, vol. 132, 12711-12716
- **LIU et al.** *Bioorganic & medicinal chemistry letters*, 2011, vol. 21, 7013-7016
- **KIESS et al.** *The quarterly journal of nuclear medicine and molecular imaging*, 2015, vol. 59, 241
- **EDER et al.** *Bioconjugate chemistry*, 2012, vol. 23, 688-697
- **ROWE et al.** *Molecular Imaging and Biology*, 2016, 1-9
- **DIETLEIN et al.** *Molecular Imaging and Biology*, 2015, vol. 17, 575-584
- **CARDINALE et al.** *Journal of nuclear medicine: official publication, Society of Nuclear Medicine*, 2017, vol. 58, 425-431
- **GIESEL et al.** *European journal of nuclear medicine and molecular imaging*, 2016, vol. 43, 1929-1930
- **GIESEL et al.** *European journal of nuclear medicine and molecular imaging*, 2017, vol. 44, 678-688
- **LINDNER et al.** *Bioconjugate Chemistry*, 2014, vol. 25, 738-749
- **SCHIRRMACHER E. et al.** *Bioconjugate Chem.*, 2007, vol. 18, 2085-2089
- **WÄNGLER C. et al.** *Bioconjugate Chem.*, 2009, vol. 20 (2), 317-321
- **WÄNGLER C. et al.** *Bioconjug Chem.*, 15 December 2010, vol. 21 (12), 2289-96
- **BERNARD-GAUTHIER et al.** *Biomed Res Int.*, 2014, vol. 2014, 454503
- **LINDNER S. et al.** *Bioconjug Chem.*, 16 April 2014, vol. 25 (4), 738-49
- **NIEDERMOSER S. et al.** *J Nucl Med.*, July 2015, vol. 56 (7), 1100-5
- **WURZER et al.** *J. Nucl. Med.*, 2020, vol. 61 (5), 735-742
- **WURZER et al.** *EJNMMI Research*, 2020, vol. 10 (1), 1-10
- **SANGSTER**. Octanol-water Partition Coefficients: fundamentals and physical chemistry. John Wiley & Sons, 1997
- **BRYN et al.** Solid-State Chemistry of Drugs. SSCI, Inc, 1999
- **VALKO, K.** ; **NUNHUCK, S.** ; **BEVAN, C.** ; **ABRAHAM, M. H.** ; **REYNOLDS, D. P.** *Journal of pharmaceutical sciences*, 2003, vol. 92, 2236-2248
- **YAMAZAKI, K.** ; **KANAOKA, M.** *Journal of Pharmaceutical sciences*, 2004, vol. 93, 1480-1494